(19) **Europäisches Patentamt / European Patent Office / Office européen des brevets**

(11) **EP 1 850 797 B1**

(12) **EUROPEAN PATENT SPECIFICATION**

(45) Date of publication and mention
of the grant of the patent:
**30.04.2014 Bulletin 2014/18**

(21) Application number: **06711221.9**

(22) Date of filing: **22.02.2006**

(51) Int Cl.:
*A61L 27/26* (2006.01)      *A61L 24/04* (2006.01)
*A61B 17/16* (2006.01)      *A61F 2/46* (2006.01)
*A61B 17/88* (2006.01)      *B01F 7/30* (2006.01)
*B01F 15/00* (2006.01)      *B01F 15/02* (2006.01)

(86) International application number:
**PCT/IL2006/000239**

(87) International publication number:
**WO 2006/090379 (31.08.2006 Gazette 2006/35)**

(54) **Bone cement**

Knochenzement

Ciment osseux

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.02.2005 US 654495 P**
**31.07.2005 PCT/IL2005/000812**
**01.08.2005 US 194411**
**28.09.2005 US 721094 P**
**28.09.2005 US 720725 P**
**25.10.2005 US 729505 P**
**22.11.2005 US 738556 P**
**26.01.2006 US 762789 P**
**26.01.2006 US 763003 P**
**02.02.2006 US 765484 P**

(43) Date of publication of application:
**07.11.2007 Bulletin 2007/45**

(60) Divisional application:
**10192300.1 / 2 319 441**
**10192301.9 / 2 319 439**
**10192302.7 / 2 319 440**

(73) Proprietor: **Depuy Spine, Inc.**
**Raynham, MA 02767 (US)**

(72) Inventors:
• **BEYAR, Mordechay**
  **38900 Caesarea (IL)**
• **GLOBERMAN, Oren**
  **46910 Kfar-shmaryahu (IL)**
• **SHAVIT, Ronen**
  **69413 Tel-aviv (IL)**
• **WACHSLER-AVRAHAMI, Hila**
  **69400 Tel-aviv (IL)**

(74) Representative: **Orr, Robert et al**
**Urquhart-Dykes & Lord LLP**
**Tower North Central**
**Merrion Way**
**Leeds LS2 8PA (GB)**

(56) References cited:
**EP-A2- 0 475 077      WO-A-01/60270**
**WO-A-02/072156      US-A- 4 093 576**
**US-A- 4 115 346      US-A- 4 268 639**
**US-A- 4 341 691**

• **LEWIS GLADIUS: "Properties of acrylic bone cement: State of the art review" JOURNAL OF BIOMEDICAL MATERIALS RESEARCH, WILEY, NEW YORK, NY, US, vol. 38, no. 2, 1 January 1997 (1997-01-01), pages 155-182, XP002432739 ISSN: 0021-9304**
• **FARRAR D F ET AL: "Rheological properties of PMMA bone cements during curing", BIOMATERIALS, ELSEVIER SCIENCE PUBLISHERS BV., BARKING, GB, vol. 22, no. 22, 15 November 2001 (2001-11-15), pages 3005-3013, XP004301379, ISSN: 0142-9612, DOI: 10.1016/S0142-9612(01)00047-3**

**Description**

The present application relates to a bone cement.

A common occurrence in older persons is compression fractures of the vertebrae, causing both pain and a shortening (or other distortion) of stature. One common treatment is vertebroplasty, in which cement is injected into a fractured vertebra. While this treatment fixes the fracture and reduces pain, it does not restore the vertebra and person to their original height. Another problem is that the cement is injected in a liquid phase so that it may be unintentionally injected outside of the vertebra and/or may migrate out through cracks in the vertebra. This may cause considerable bodily harm.

Another common treatment is kyphoplasty, in which the fracture is reduced, for example by first inflating a balloon inside the vertebra and then injecting a fixing material and/or an implant. The problem of cement migration is reduced, but not avoided, as a lower pressure can be used to inject the cement.

In general, polymeric cements become more viscous as the polymer chain grows by reacting directly with the double bond of a monomer. Polymerization begins by the "addition mechanism" in which a monomer becomes unstable by reacting with an initiator, a volatile molecule that is most commonly a radical (molecules that contain a single unpaired electron). Radicals bond with monomers, forming monomer radicals that can attack the double bond of the next monomer to propagate the polymer chain. Because radicals are so transient, initiators are often added in the form of an unreactive peroxide form that which is stable in solution. Radicals are formed when heat or light cleaves the peroxide molecule. For applications in which high temperatures are not practical (such as the use of bone cement in vivo), peroxide is cleaved by adding a chemical activator such as N,N-dimethyl-p-toluidine. (Nussbaum DA et al: "The Chemistry of Acrylic Bone Cement and Implication for Clinical Use in Image-guided Therapy", J Vase Interv Radiol (2004); 15:121-126).

Viscous cement is advantageous not only in reducing the risk of its leakage, but also, because of its ability to infiltrate into the intravertebral cancellous bone (interdigitation) [see G Baroud et al, Injection biomechanics of bone cements used in vertebroplasty, Bio-Medical Materials and Engineering 00 (2004) 1-18]. Baroud also suggests that about 95% of the applied injection pressure is required to overcome friction in the cannula. In addition, viscous material may reduce the fracture.

Examples of commercially available viscous bone cements include, but are not limited to, CMW$^R$ Nos. 1, 2 and 3 (DePuy Orthopaedics Inc.; Warsaw, IN, USA) and Simplex™ -P and -RO (Stryker Orthopaedics; Mahwah, NJ, USA). These cements are characterized by a liquid phase after mixing and prior to achieving a viscosity of 500 Pascal second. In a typical use scenario, these previously available cements are poured, while in a liquid phase, into a delivery device.

There have also been attempts to reduce cement migration by injecting more viscous cement, for example, during the doughing time and the beginning of polymerization. However, the injection methods suggested require higher pressures for the more viscous material. Also, some types of viscous materials, such as hardening PMMA, have a small workability window at high viscosities, as they harden very quickly once they reach a high viscosity. This has generally prevented very viscous materials and the associated very high pressures from being used. One possible reason is that as pressures increase, the physician is prevented from receiving feedback on the resistance of the body to the injection of the cement. Thus, over-injection can easily occur.

Some fixing materials, such as polymethylmethacrylate (PMMA), emit heat and possibly toxic materials while setting. These may further weaken the bone and possibly cause the cement to loosen and/or the bone to fracture.

It has recently been suggested that some fixing materials, being harder than bone, induce fractures in nearby bones.

It is also known to use bone-like repair materials, such as a slurry of bone chips, which apparently do not induce such fractures. However, injecting such materials is difficult due to their viscosity.

US patents and applications 4,969,888, 5,108,404, 6,383,188, 2003/0109883, 2002/0068974, 6,348,055, 6,383,190, 4,494,535, 4,653,489 and 4,653,487 describe various tools and methods for treating bone.

An additional manner to deliver bone cement into the vertebra is using a tamping instrument (US Patents Nos. 6,241,734 and 6,613,054), comprising a cannula and a rod, which urges the material within the cannula into the bone.

US patent application 20040260303 teaches an apparatus for delivering bone cement into a vertebra. Cannulae with working sleeves are described, for example, in US 6,241734 and 6,613,054.

US-A-4341691 discloses an acrylic cement-like substance with a polymer powder component comprising PMMA in the form of beads and a liquid monomer component comprising MMA.

The invention relates to formulations of bone cement which provide a window of time during which the cement is suitably viscous for injection.

Thus, the present invention provides a bone cement comprising: a first powder component which includes poly(methyl methacrylate) (PMMA) with at least 80% of the PMMA having a bead size of between 10 and 200 $\mu$m, and a second liquid component which includes methyl methacrylate (MMA), in which contacting the first component and the second component produces a mixture which is suitable for *in vivo* use, wherein a portion of the PMMA has a molecular weight between about 600,000 Dalton and about 1,200,000 Dalton and the mixture attains a viscosity greater than 500 Pascal.seconds within an initial period, and the viscosity of the mixture remaining between 500 and 2000 Pascal.seconds for a working time of at least 5 minutes after the initial period, the viscosity of the mixture being measured using a capillary extrusion rheometer for measuring an applied force acting on a piston to displace the mixture in a capillary tube at a constant displacement rate, the measurements being performed at an average temperature of 22.3°C and a constant displacement rate of 3mm/min.

In an exemplary embodiment of the invention, the cement achieves a high viscosity rapidly when components are mixed and then sets slowly. In an exemplary embodiment of the invention, the cement remains viscous when held above a threshold temperature and sets when cooled below the threshold temperature. In an exemplary embodiment of the invention, the cement is a non-setting cement. Optionally, there is no liquid phase when the cement components are mixed.

Embodiments of the invention relate to a viscous bone cement which has an enhanced high-viscosity window before it sets and where viscosity, while high, does not vary to a degree which influences injection parameters. Optionally, the viscosity in the window is 500, optionally 1,000, optionally 1,500, optionally 2,000 Pascal-sec or intermediate values. Optionally, the cement is sufficiently viscous to move fractured bone, such as vertebral plates of a collapsed vertebra, as it is injected. Injection of viscous cement may contribute to fracture reduction and/or restoration of vertebral height.

In an exemplary embodiment of the invention, the mixture produces a high viscosity material within 1 second, within 5 seconds, within 10 seconds, within 15 seconds, within 30 seconds, within 60 seconds, within 120 seconds or intermediate times. Optionally, once a high viscosity is achieved, the viscosity remains stable for 5 minutes or more. This interval of stable viscosity provides a window of opportunity for performance of a medical procedure.

The working window is optionally at least 8 minutes, optionally at least 10 minutes, optionally at least 15 minutes, optionally at least 20 minutes. In an exemplary embodiment of the invention, increased viscosity is provided a short time after mixing of the cement, for example, zero time (for a pre-provided viscous material), less than 1 minute or less than 2 or less than 3 minutes after mixing is complete.

The cement may include styrene. Optionally, an average molecular weight of the PMMA is in excess of 60,000, optionally 70,000, optionally 80,000, optionally 90, 000, optionally 100,000 Dalton. In an exemplary embodiment of the invention, the average molecular weight of the PMMA in the beads is in the range of about 100,000 to 120,000, optionally about 110,000 Dalton. A portion of the PMMA in the beads has a high molecular weight. Optionally, the portion includes 0.25%, 0.5%, 1%, 2%, 3% or lesser or intermediate or greater percentages of the total PMMA in the beads. Optionally, high molecular weight PMMA in the beads is 600,000, optionally 900,000, optionally 1,100,000 Dalton or intermediate or lesser or greater values. In an exemplary embodiment of the invention, approximately 1% of the PMMA in the beads is characterized by a molecular weight of 700,000 to 1,000,000 Dalton and the average molecular weight of the PMMA is approximately 110,000 Dalton. In an exemplary embodiment of the invention, bead diameter is in the range 10-200 microns, optionally 20 to 150 microns, optionally with an average of about 60 microns or lesser or intermediate or greater sizes.

In some embodiments of the invention the bone cement has a glass transition temperature higher than 37 degrees Celsius. In an exemplary embodiment of the invention, heating the bone cement above the glass transition temperature transforms the bone cement to a dough-like or putty-like state. Optionally, dough-like indicates a viscosity of at least 500, optionally at least 900, optionally at least 1000 Pascal seconds or lesser or intermediate or greater values. In an exemplary embodiment of the invention, the dough-like state is characterized in that a pressure less than 200 atmospheres is sufficient to cause it to flow through a tube with an ID of 3mm and a length of 100mm. Optionally, the dough-like material is suitable for delivery using a high-pressure fluid delivery system, for example as disclosed herein. In an exemplary embodiment of the invention, the temperature is lower than a maximum temperature allowed inside the human body, for example, being below 60 degrees Celsius, below 50 degrees, below 45 degrees and/or below 40 degrees or intermediate or lesser temperatures. A penetrating tube may be subject to temperature control, for example by means of insulation. Optionally, a double-walled tube with vacuum pressure between its walls provides the isolation.

In an exemplary embodiment of the invention, the bone cement includes processed bone (from human or animals origin) and/or synthetic bone. Optionally, the cement has osteoconductive and/or osteoinductive behaviour.

The bone cement may be injected into a bone void as a preventive therapy or as a treatment for an existing condition.

A temperature control unit may be provided to facilitate heating and/or cooling of bone cement. Optionally, temperature control is applied to increase the handling and/or working time (e.g., heat may be applied to offset temperature changes resulting from high pressures). The temperature control unit may operate on the cement in an external reservoir and/or cement in a delivery system reservoir.

Hydraulic systems may be used for delivering the bone cement into a bone. Optionally, delivery is via a small diameter tube such as a bone access cannula. For example delivery is via a tube with an inner diameter of 1.5 mm, 2 mm, 3 mm, 4 mm or lesser or greater or intermediate values. For example the system is operable by foot and/or is battery powered. For example the system provides sufficient pressure to deliver at least 5ml, optionally at least 10 ml, of a viscous bone cement as a single continuous aliquot.

The system design may assure the physician's hands are located outside an X-ray radiation zone. For example a hand operable actuator for a pressure source is located 20 cm, 40 cm, 60 cm, 100 cm or intermediate or greater distances from a cement reservoir.

The actuator for the pressure source may employ a threaded steel rod which passes through a plastic nut. Optionally, this combination of materials reduces the friction coefficient. Optionally, reducing a diameter of the bolt reduces a radius-of-friction. Optionally, reducing a radius-of-friction reduces an applied moment. A short actuation handle may permit an operator to grasp the handle on both sides of the axis (bolt) so that moment is applied without generating undesired radial force.

The pressure source may include a safety valve. Solidification of the cement may contribute to increased pressure. Optionally, solidification of the cement increases pressure to a threshold which operates the safety valve. For example, the threshold is significantly below a maximum internal pressure which the system can withstand.

The cement reservoir may include a floating piston. Optionally, the floating piston separates between cement and a hydraulic fluid. For example, the cement reservoir includes a piston mounted on a rod.

The hydraulic actuator may be operable by various means. Optionally, the pressure source includes a foot operable actuator. The foot operable actuator includes a clutch plate with a hole with a varying aspect ratio. The pressure source may provide a pressure of 50, optionally 100, optionally 150, optionally 200, optionally 300 atmospheres or lesser or greater or intermediate values. Optionally, the hydraulic actuator provides pressure amplification. For example pressure amplification is provided by mechanical advantage (levers) and/or bolt thread step and/or a hydraulic amplification.

Mated threaded materials may be chosen to reduce the friction coefficient ($\mu$). Optionally, the friction coefficient is 0.2 or less. Optionally, the friction coefficient increases maneuverability. Optionally, a cover serves as a nut and is made of a polymer/plastic. Optionally, a drive shaft serves as a bolt and is made of steel. Optionally, a Radius of Friction (R; bolt radius in this example) is reduced by reducing the thread diameter of the bolt and nut. For example R is 2, optionally 3, optionally 4, optionally 5 mm or intermediate values.

The cannula may be equipped with one or more apertures for cement delivery at a distal end and/or near the distal end. For example lateral openings on the cannula permit sideways injection to a desired target in a bone.

An apparatus for mixing the bone cement uses a revolving paddle which does not rotate. Optionally, the paddle provides high shearing forces. Optionally, the bone cement is easily removable from a flat paddle. Optionally, the mixer includes a delivery mechanism adapted to facilitate delivery of the bone cement from the mixer to an external reservoir. Optionally, the paddle presses materials against a wall of a container.

A high-pressure delivery system may be used to fill a small diameter cannula with the bone cement. Pressure to fill the cannula may be provided by a hydraulic delivery system. Optionally, once the cannula is filled, the cement may be injected into the body in various means, for example, using a tamping instrument including a rod adapted to comply with a lumen of the cannula. Pressure to fill the cannula may be provided by a piston with a diameter greater than the cannula lumen. A plurality of cannulae may be pre-filled and used as needed. Optionally, the cannulae are provided into the body through an outer sheath which remains in place when the cannulae are changed. Alternatively or additionally, the cannula is refilled while inside the body.

A bone cement cannula may be used with a permanently closed distal tip. Optionally, delivery of cement is through one or more apertures on a lateral surface of the cannula near the distal tip. An orientation marking on a proximal portion of the cannula may facilitate correct orientation of the lateral aperture(s) after the distal tip of the cannula is inserted. Optionally, the cannula includes a depth marking or coining.

The closed tip may be used as a trocar tip for penetrating tissue and/or bone. Alternatively or additionally, the closed tip is used to aim cement delivery.

Optionally, the working time is at least 8 minutes long.

Optionally, the initial setting period is less than 3 minutes.

Optionally, the initial setting period does not exceed 1 minute.

Optionally, the mixture solidifies after the working time.

Optionally, the initial setting period is less than 3 minutes and the mixture solidifies after the working time.

Optionally, the first component includes PMMA and Barium Sulfate.

Optionally, the second component includes MMA and DMPT.

Optionally, the first component includes PMMA, Barium Sulfate and Benzoyl Peroxide and the second component includes MMA, DMPT and Hydroquinone. Optionally, the viscosity of greater than 500 Pascal-second results at least partly from a polymerization reaction.

Optionally, the first component contains about 69.4% w/w PMMA, about 30.1% Barium Sulfate and about 0.5% Benzoyl Peroxide and the second component contains about 98.5% v/v MMA, about 1.5% DMPT and about 20 ppm Hydroquinone.

Optionally, the average molecular weight of the PMMA is between about 80,000 Dalton and about 180,000 Dalton. Optionally, the average molecular weight of the PMMA is about 110,000 Dalton.

Optionally, the cement further comprises processed bone and/or synthetic bone that is mixed together with the first component and the second component.

In a vertebral implant, a quantity of the bone cement may be injectable into a vertebral body during the at least 5 minutes and capable of subsequent hardening therein.

Optionally, the at least 5 minutes is at least 8 minutes. Optionally, the quantity of cement is at least 1 ml.

An apparatus for injecting the bone cement may comprise: a reservoir configured to hold at least 5 ml of unhardened bone cement, the reservoir having an outlet and a hydraulically driven plunger configured to press the cement in the reservoir against the outlet with an internal pressure of at least 40 atm so that at least a portion of the cement will be forced out of the reservoir through the outlet; wherein the reservoir and the plunger are configured to withstand the internal pressure.

Optionally, the apparatus comprises a cannula configured to route the cement that is forced out of the outlet into a bone in a living subject.

Optionally, the pressure to operate the hydraulically driven plunger is generated by a hydraulic pressure source that is located at least 25 cm away from the plunger, wherein the hydraulic pressure source has an actuator.

Optionally, each actuation of the actuator by a user causes the plunger to force a predetermined amount of cement out of the reservoir.

Optionally, the predetermined amount is between 0.15 and 0.5 ml.

Optionally, pressure to operate the hydraulically driven plunger is generated by a hydraulic pressure source including a foot-operable actuator. Optionally, each actuation of the foot-operable actuator by a user causes the plunger to force a predetermined amount of cement out of the reservoir.

Optionally, the cannula has an inner diameter not exceeding 2.5 mm and a length of at least 100 mm. Optionally, at least part of the reservoir is made of amorphous nylon.

Optionally, at least part of the reservoir is transparent.

Optionally, the reservoir is configured to hold at least 10 ml of cement.

Optionally, the internal pressure is at least 100 atm.

Optionally, the internal pressure is at least 200 atm.

By way of example, an apparatus for injecting the bone cement comprises: a reservoir configured to hold at least 5 ml of a bone cement having a viscosity of at least 900 Pascal seconds, the reservoir having an outlet; and a hydraulically actuated plunger configured to press the cement in the reservoir against the outlet with enough pressure to force at least some of the cement out of the reservoir in response to an actuation input.

Optionally, the apparatus comprises a cannula operatively configured to route cement from the outlet into a bone in a living subject.

Optionally, the pressure for operating the hydraulically actuated plunger is generated by a hydraulic pressure source located at least 25 cm away from the plunger.

Optionally, each actuation of the actuator by a user causes the plunger to force a predetermined amount of cement out of the reservoir. Optionally, the predetermined amount is between 0.15 and 0.5 ml.

Optionally, the pressure for operating the hydraulically actuated plunger is generated by a hydraulic pressure source actuatable by a foot-operable actuator.

Optionally, each actuation of the foot-operable actuator by a user causes the plunger to force a predetermined amount of cement out of the reservoir. Optionally, at least part of the reservoir is made of amorphous nylon.

Optionally, at least part of the reservoir is transparent.

Optionally, the reservoir is configured to hold at least 10 ml of cement.

In a bone cement delivery cannula, the cannula may comprise:

(a) an inner lumen adapted to provide a channel of fluid communication between a bone cement reservoir and at least one lateral cement ejection aperture; and
(b) a permanently axially closed distal tip.

In a system for injection of the bone cement into a bone, the system comprises:

(a) a reservoir containing bone cement to be injected into a bone, said reservoir deployed external to a body of a subject; (b) a pressure source adapted to apply sufficient pressure to the material in the reservoir to expel at least a portion of the bone cement from the reservoir through a cannula; (c) an actuator operable to activate the pressure source; and
(d) a cannula adapted for insertion into the bone, said cannula connectable to the external reservoir; wherein an operator of the system may perform an injection touching only the actuator.

Optionally, the system is used to inject the bone cement in proximity to an implant to strengthen the implant.

Optionally, a system described herein is employed to perform a vertebroplasty procedure.

Optionally, the bone cement described herein is employed in a vertebroplasty procedure.

Some embodiments of the invention relate to a bone cement which sets to a hardened condition, which maintains a high viscosity value during a substantial window of time. In an exemplary embodiment of the invention, the viscosity is between 600 Pascal- second and 1,800 Pascal-second during a period of at least 5 or at least 8 minutes or greater or intermediate values. In an exemplary embodiment of the invention, the bone cement is composed of a mixture of PMMA and styrene beads and MMA monomers, with the increase in viscosity being provided by the size of the beads and/or by changing the ratio between beads and liquid MMA monomer, and/or by changing molecular weight of polymer in the beads. Optionally, as setting progresses, viscosity due to the beads is replaced/increased by viscosity due to the polymerization process. Alternatively or additionally, addition of Butyl Methacrylate to PMMA provides an increase in viscosity for any given ratio between monomer and polymer and/or for any given set of bead parameters.

In an exemplary embodiment of the invention, the mixture attains, within a period of up to 2 minutes, a putty-like consistency wherein the viscosity of the resulting mixture remains approximately constant during a period of time of at least 5 minutes.

Exemplary non-limiting embodiments of the invention will be described with reference to the following description of embodiments in conjunction with the figures. Identical structures, elements or parts which appear in more than one figure are generally labeled with a same or similar number in all the figures in which they appear, in which:

Fig. 1A is a general flowchart of a process of treating a compression fracture;

Fig. 1B is a more detailed flowchart of a process of treating a compression fracture;

Fig. 2 shows a composite tool for accessing a vertebra;

Figs. 3A-3F show stages of a method of treatment according to Figs 1A and 1B;

Figs. 4A and 4B illustrate basic material delivery systems;

Figs. 5A and 5B show details of material extruder tips;

Figs. 6A-6C illustrate narrowing lumen sections of a delivery system;

Fig. 7A illustrates a hydraulic delivery system;

Figs. 7B and 7C show alternative methods of providing hydraulic power to the system of Fig. 7 A;

Figs. 7D and 7E illustrate an exemplary hydraulic system including a disposable unit;

Figs. 7F-7G illustrate an exemplary hydraulic delivery system;

Fig. 7H illustrates an exemplary hydraulic delivery system;

Fig. 7I is a cross sectional view of a hydraulic actuator for a delivery system (the inset shows a portion of the actuator in greater detail);

Figs. 7J and 7K are exploded and cross sectional views respectively of a portion of a hydraulic actuator for a delivery system;

Fig. 7L is an exploded view of a pressure release valve for a delivery system;

Figs. 7M, 7N and 7O are cross sectional views and a side view of a pressure release valve for a delivery system in operation;

Figs. 7P and 7Q are cross sectional views of a cement reservoir illustrating a floating piston;

Figs. 7R and 7S are cross sectional views of a cement reservoir illustrating assembly of a distal injection port;

Fig. 7S1 is a cross sectional view of a cement reservoir;

Fig. 7T is a perspective view of a foot operated actuator suitable for use in a delivery system;

Fig. 7U is a cut away view of a foot operated actuator suitable for use in a delivery system;

Figs. 7V5 7W, 7X and 7Y are additional views of a foot operated actuator suitable for use in a delivery system;

Fig. 8A shows a cassette based delivery system;

Fig. 8B is a detail showing the delivery of unit element;

Figs. 9A and 9B show a material pusher with reduced material twisting;

Fig. 10A- 10F show sleeve based material pushers;

Figs. 11A and 11B show squeeze based delivery systems;

Fig. 12A and 12B illustrate a one step access and delivery system;

Fig. 12C shows an over-the-wire delivery system;

Fig. 13 is a graph showing compressibility of a material;

Figs 14A and 14B are exploded and perspective views respectively of an exemplary apparatus for mixing viscous material;

Figs. 14C1, 14C2, 14C3 and 14C4 are a series of top views illustrating an exemplary travel path of a mixing element within a mixing well of the exemplary apparatus of Figs. 14A and 14B;

Fig. 15 is a graph of viscosity (Pascal Sec) as a function of time (minutes) for an exemplary cement according to the invention and an exemplary prior art cement;

Figs. 16 and 17 are graphs indicating viscosity as Newtons of applied force per unit displacement (mm) under defined conditions for exemplary cements according to the invention and illustrate the time window for injection which is both early and long;

Figs. 18, 19, 20 and 21 are perspective views of an exemplary transfer apparatus for loading a viscous material into a container; and

Figs. 22, 23, 24, 25 and 26 illustrate an additional exemplary transfer apparatus.

**Overview of exemplary process**

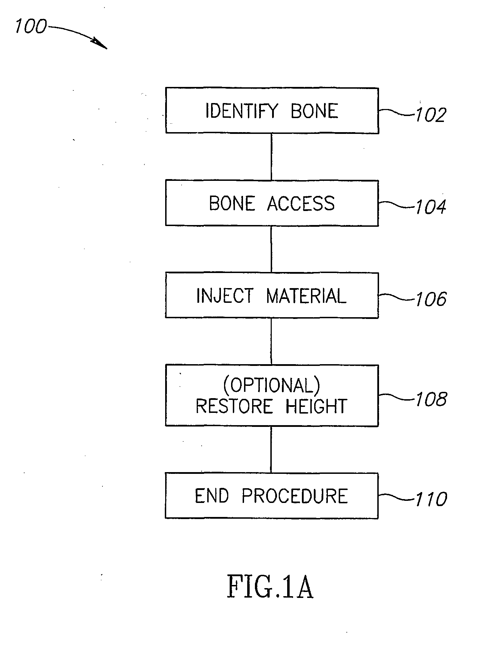

Fig. 1A is a general flowchart 100 of a process of treating a compression fracture.

At 102, a bone to be treated is identified. In the case of a vertebra, this usually involves X-ray or CT images to identify a vertebra or other bone that is fractured, for example by a compression fracture. The following description focuses on vertebral compression fractures.

The access is minimally invasive, for example, only a single channel is formed into the body. Optionally, the procedure is carried out via a cannula having a diameter of, for example 5 mm, 4 mm or less in diameter inserted into the body. In some cases, multiple openings into the body are formed. The procedure can also be carried out using a surgical or key-hole incision; however, this may require a longer recuperation period by the patient. Optionally, the cannula (and corresponding length of a delivery tube described below) is at least 50 mm, 70 mm, 100 mm or more or intermediate or smaller values.

At 104, the vertebra is accessed.

At 106, a material having a high viscosity in some embodiments of the invention is injected into the vertebra. Optionally, the vertebra is fractured due to weakening caused by osteoporosis or other pathological conditions.

At 108, material is optionally provided in a manner and/or amount which restores at least part of the height of the vertebra, for example, 20%, 40%, 50% or intermediate or a higher percentage of a pre-compression height. A particular feature is that the provided material is of sufficient viscosity or sufficiently solid that leakage from the vertebra is reduced or prevented, as compared to liquid PMMA cement. A pressure used to advance the material may be higher than what is known in the art to match the increased viscosity.

At 110, the procedure is completed and the tube is removed.

**Exemplary bone access set**

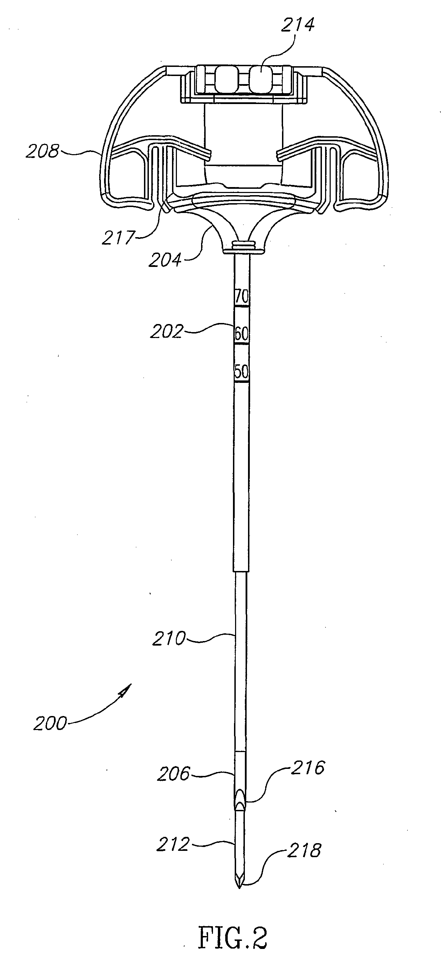

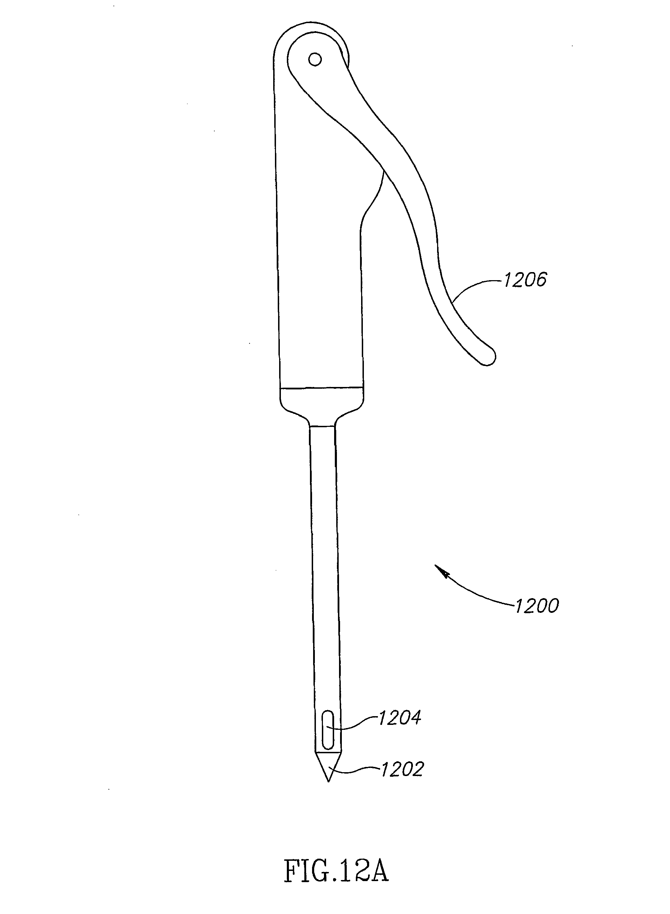

Before going into the details of the procedure, the tools used are first described. Fig. 2 shows a composite tool 200 optionally used for accessing the bone. The access tools comprise a set of component tools that interlock to act, selectively, as a single tool or as separate tools. This composite set/tool may serve as a one step access system in which only a single insertion of objects into the body is required. Optionally, as described below, the delivery system is also inserted at the same time. Optionally, a cannula portion of the tool is omitted, for example as described in Figs. 12A-12C.

The components of tool 200 may be coaxially matched components which fit one within the lumen of the next.

An optional cannula 202 comprises a handle 204 and a body including a lumen.

An optional drill tool 206 includes an elongate body adapted for drilling and a handle 208. Optionally, handle 208 selectively rotationally locks to handle 204, for manipulation using a single hand, optionally using a snap-lock 217. The body of tool 206 fits in the lumen of cannula 202. Optionally, a section 210 of tool 206 is marked to be visible on an x-ray image, even in contrast to cannula 202. Optionally, this allows the difference in diameters between cannula 202 and drill tool 206 to be minimal. Absent such a marker, in some cases, the difference in diameters may not be visible on an x-ray image and the two tools cannot be distinguished. An optional guidewire 212 is provided inside a lumen of drill tool 206. Optionally, a knob or other control 214 is provided for selective advancing and/or retracting of guidewire 212 relative to drill 216. The knob may be marked with relative or absolute positions.

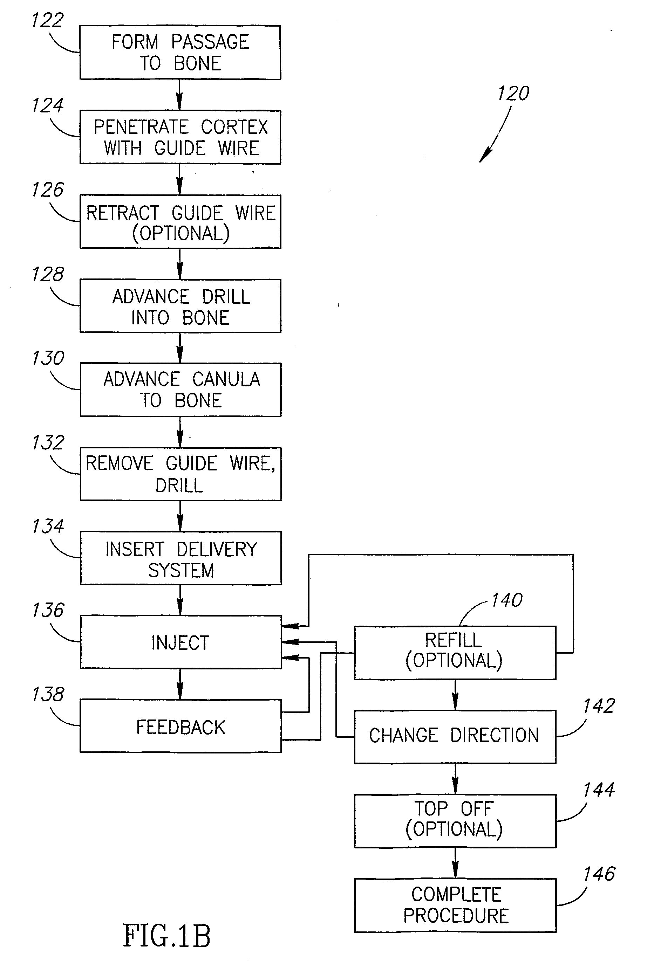

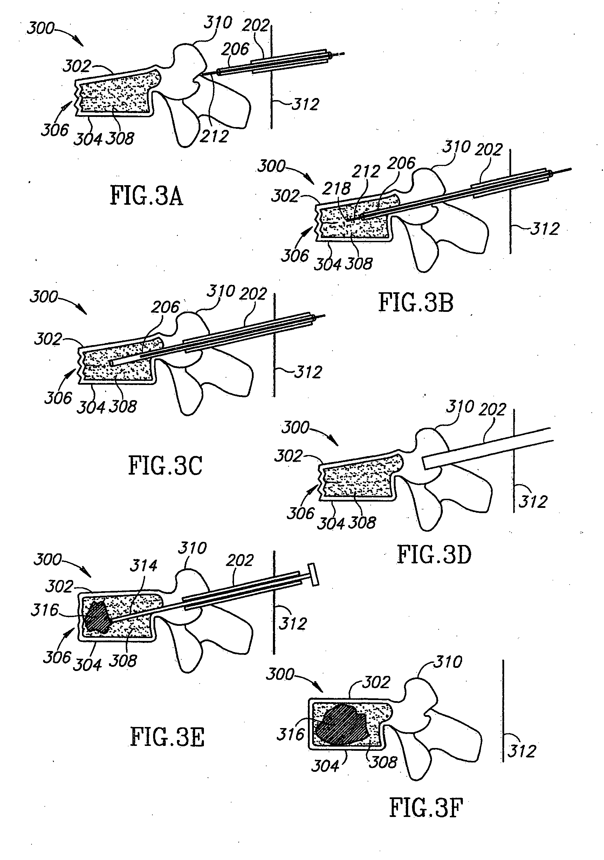

Optional depth marking are provided on cannula 202. An exemplary use of these tools will be described below, in which Figs. 3A-3F schematically show the progress as a vertebra 300 having a compression fracture 306 is being treated, paralleling a detailed flowchart 120 shown in Fig. 1B.

**Penetrate to bone**

At 122 (Fig. 1B), a passage is formed to the bone through a skin layer 312 and intervening tissue, such as muscle and fat. Optionally, the passage is formed by advancing composite tool/set 200 until a tip 218 of guidewire 212 contacts the bone. Tip 218 may be designed to drill in soft tissue (e.g., includes a cutting edge). Alternatively or additionally, tip 218 includes a puncturing point adapted to form a puncture in soft tissue.

This is shown in Fig. 3A. Also shown are cortical plates 302 and 304 of the vertebra and a cancellous bone interior 308.

A single pedicle 310 is shown, due to the view being cross-sectional. Optionally, the access to the vertebra is via a pedicle. Optionally, the access is via both pedicles. Optionally, an extrapedicular approach is used. Optionally, the access point or points are selected to assist in an even lifting of the vertebra.

**Penetrate bone**

At 124, tip 218 penetrates through the cortex of the bone being treated (Fig. 3B). Tip 218 may be separately manipulated from the rest of composite tool 200. Optionally, tip 218 is advanced until it contacts a far side of the vertebra.

Tip 218 of guidewire 212 may be formed to drill in bone and is advanced through the vertebral cortex by rotation or vibration. Optionally, it is advanced by tapping thereon or applying pressure thereto.

Optionally, a relative position of the guidewire and the cannula is noted, to assist in determining the inner extent of the vertebra.

At 126, the guidewire is optionally retracted. Optionally, the guidewire is axially locked to drill tool 206. Optionally, guidewire 212 and drill tool 206 align so that tip 218 and a tip 216 of the drill tool form a single drilling tip.

At 128, drill tool 206 is advanced into the bone (Fig. 3C). Optionally, tip 216 of drill tool 206 designed for drilling and/or is advanced, for example, by tapping, rotation and/or vibration. Optionally, the drill tool is advanced to the far side of the vertebra. Optionally, the previous depth marking of the guidewire is used to limit this advance. Optionally, the guidewire is not retracted at 126. Instead, drill tool 206 is advanced over the guidewire until it reaches the end of the guidewire. At 130, cannula 202 is optionally advanced to the bone over the drill. Optionally, the leading edge of the cannula is threaded or otherwise adapted to engage the bone at or about the bore formed by the drill tool. Optionally, the cannula is inserted into the bone.

At 132, the guidewire and/or drill tool are optionally removed (Fig. 3D). In some cases, the cannula is not advanced all the way to the bone. In others, the cannula may be advanced into the bone, for example, to prevent contact between the treatment and cortical bone and/or weak or fractured bone. Optionally, the cannula is advanced past the pedicle and to the vertebral interior 308.

Optionally, a creamer (not shown) is inserted into the cannula and used to remove tissue from interior 308.

### Inject material

At 134, a material delivery system 314 is provided into cannula 202 (shown in Fig. 3E). Optionally, the delivery system delivers material to a side thereof (described below).

At 136, system 134 is activated to inject material 316 into interior 308. Fig. 3E shows that when enough material is injected, vertebral height may be partially or completely restored. The injected material may partially or completely compress interior 308.

### Feedback

At 138, feedback is optionally provided to an operator, to decide if injection is completed. Optionally, feedback is provided by fluoroscopic imaging of the site. However, other imaging methods may be used.

Optionally, non-imaging feedback is provided, for example a pressure inside the vertebra, using a pressure sensor (not shown), or using an indicator (visual or audio) for the amount of material injected.

Optionally, the feedback is used to decide if the procedure is progressing as desired, e.g., desired amount of height restoration (if any), verify a lack of material leakage, determine symmetry or asymmetry and/or the presence of new fractures in bone.

### Repeat and/or change

Optionally, the material is provided in a magazine having a fixed amount (described below). If that magazine is finished and additional material is required, a refill may be provided (140), for example by replacing the magazine with a new one.

Optionally, a property of the delivery of material is changed, for example one or more of a delivery pressure, a delivery rate, an amount of delivery when delivery is in discrete units, a viscosity, composition and/or type of the delivered material, a pre-heating or pre-cooling of the material, a location of provision inside the vertebra, a spatial pattern of provision and/or a direction of provision in the vertebra.

Optionally, the direction of provision of the material is changed (142), for example, to assist in maintaining symmetry of lifting or to point in the injection of material away from a fracture or towards an empty space. Optionally, the direction of provision is changed by rotating delivery system 314. Alternatively or additionally, injection is continued through a new access hole in the vertebra. Optionally, the cannula is moved axially.

Optionally, a different material is used to top off the procedure, for example, a cement which sets to a hardened condition (e.g., PMMA) is used to seal the entry hole and/or stiffen the non-hardening material (144).

### Complete procedure

At 146, the tools are removed. Fig. 3F shows vertebra 300 after the procedure is completed. Optionally, the entry incision is sealed, for example, using tissue glue or a suture.

### Exemplary basic delivery system

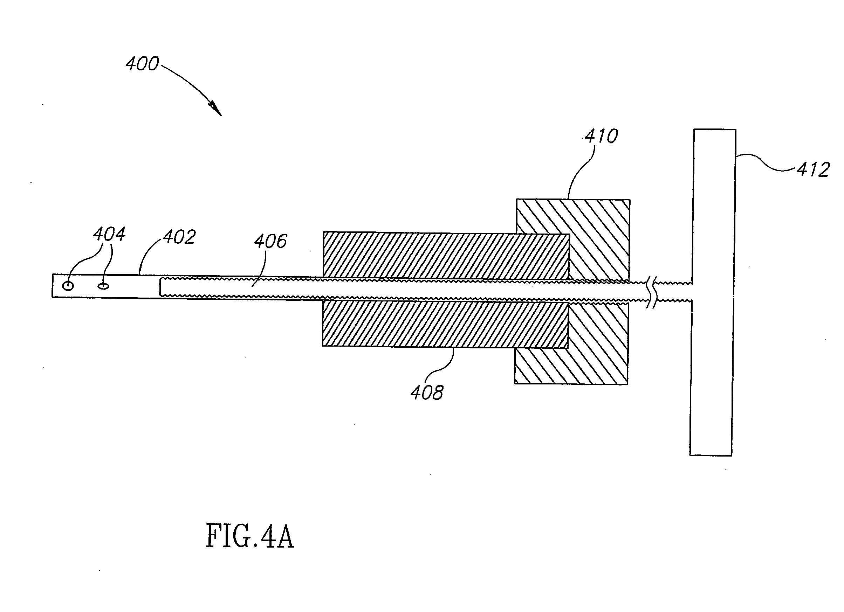

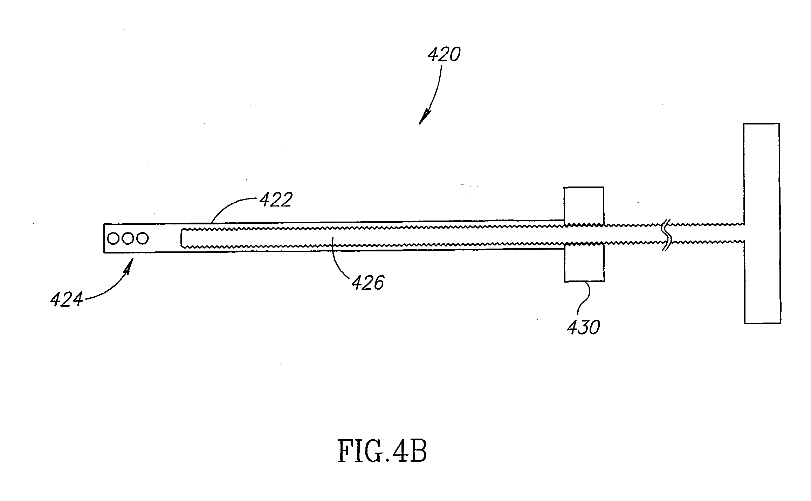

Figs. 4A and 4B illustrate basic delivery systems.

Fig. 4A is a cross-sectional view of a delivery system 400, comprising generally a delivery tube 402 having one or more extrusion apertures 404. Optionally, the distal end of tube 402 is sealed. Alternatively it may be at least partially open, so forward injection of material is provided. It is noted that when the end is sealed, there may be less force acting to retract the delivery system from the vertebra. Material inside tube 402 is advanced by a threaded pusher 406.

In the design shown, tube 402 is attached to a barrel 408 with a permanent or temporary attachment method. Threading (not shown) may be provided inside of barrel 408, to match the threading on pusher 406. Alternatively (not shown), the inner diameter of barrel 408 is greater than that of tube 402. Optionally, barrel 408 and/or tube 402 serve as a reservoir of material.

A body 410 which acts as a nut and includes an inner threading engages pusher 406. When a handle 412 of pusher 402 is rotated (while holding on to body/nut 410), pusher 406 may be advanced, injecting material out of apertures 404 into the body. Optionally, barrel 408 is detachable from body 410, for example, for replacing barrel 408 with a material-filled barrel, when one barrel is emptied. The coupling can be, for example, a threading or a quick connect, for example, a rotate-snap fit. Optionally, tube 402 is detachable from barrel 408, for example using the same type of coupling. When the distal tip of pusher 406 goes past apertures 404 (where it is that long), the passage may cut the material in front of the pusher away from the material exiting the aperture, releasing the exiting material from the delivery system.

Fig. 4B shows an alternative delivery system, 420, in which a different design of apertures 424 is used. A delivery tube 422 serves as a barrel and storage for the material and is optionally detachable from a threaded nut body 430. Optionally, tube 422 is long enough to include an amount of material sufficient for injection, for example, 8-10 cc. Optionally body 430 includes a pistol or other grip (not shown) and, as above, may be threaded to engage a pusher 426.

The delivery system may be made of metal, for example, stainless steel. Alternatively or additionally, at least some of the components are made of a polymer material, for example, PEEK, PTFE, Nylon and/or polypropylene.

Optionally, one or more components are formed of coated metal, for example, a coating with Teflon to reduce friction.

The threading of the pusher may be made ofNitronic 60 (Aramco) or Gall-Tough (Carpenter) stainless steels.

Instead of a standard threading, a ball screw is used. Optionally, the use of a ball screw increases energy efficiency and makes operation easier for manual systems as shown in Fig. 4A and 4B. Optionally, a gasket is provided to separate the balls from the material.

The delivered material may be provided as an elongate sausage with a diameter similar to that of the delivery tube and/or aperture(s). Optionally, a long delivery tube is provided. Alternatively, a plurality of such strings/sausages are implanted. Optionally, the material is provided in a diameter smaller than that of the delivery tube, for example, 0.1-0.01 mm or smaller so that there is reduced friction.

**Exemplary extrusion details**

Referring back to Fig. 4A, it is noted that the more proximal extrusion aperture 404 is optionally smaller than the more distal one. Optionally, the relative sizes are selected so that the extrusion rate and/or forces at the two holes is the same. Alternatively, the holes are designed so that the rates and/or forces are different. Referring to Fig. 4B, three axially spaced apertures may be provided and the profile of extrusion can be that a greatest extrusion and/or force is applied at the middle hole.

The sizes of apertures may be selected so that the total amount of material ejected is as desired, taking into account the possible sealing of some of the apertures by the advance of the pusher.

The apertures may be designed so that the extruded material is ejected perpendicular to the delivery system. Optionally, the delivery system is shaped so that the ejection is at an angle, for example, an angle in the plane of the axis and/or an angle in a plane perpendicular to the axis. Optionally, the angle is selected to offset forces which tend to push the delivery system out of the vertebra. Alternatively or additionally, the angle is selected to match a desired lifting direction of the vertebra or, for example, to prevent direct lifting by the extruded material. Optionally, the delivery system is inserted at a desired angle into the vertebra. Optionally, the angles of different apertures, for example, apertures on opposite sides of the delivery tube, are different, for example, defining a 180 degree angle between the apertures on opposite sides or a more acute (towards the proximal side) or oblique angle. The extrusion angle may be 30 degrees, 45 degrees, 60 degrees, 80 degrees or smaller, intermediate or larger angles to the tube axis. Optionally, the material is extruded with a bend radius of 1 mm, 2 mm, 3 mm, 4 mm, 5 mm, 10 mm or intermediate, smaller or larger radii.

The radial arrangement of the extrusion apertures can be of various designs. In one example, for example to ensure even filling of space 308, three, four or more axial rows of apertures are provided. Each row can have, for example, one, two, three or more apertures. In another example, apertures are provided only on opposing sides, so that, for example, a user can select if to extrude towards cortical plates 302 and/or 304, or not.

Rather than rows, a staggered arrangement may be used. One possible advantage for a staggered arrangement is that the delivery tube may be overly weakened by aligned rows of apertures.

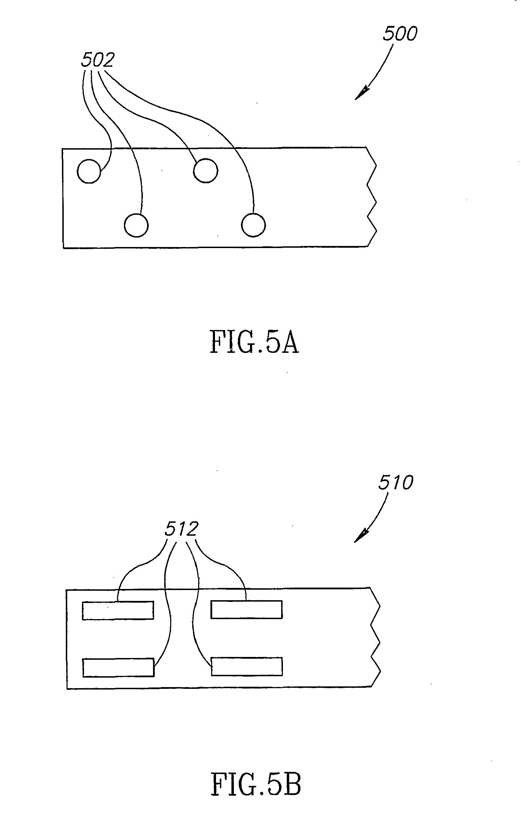

Fig. 5A shows a design of a delivery tip 500 in which round apertures 502 in a staggered row design are used. Fig. 5B shows a design of a delivery tip 510 in which elongated rectangular apertures 512 are arranged in a non-staggered manner.

As shown, the shape of the apertures can be various, for example, round, ellipsoid, rectangular, axially symmetric or asymmetric, parallel to the tube axis or not and/or elongate.

Optionally, the edges of the apertures are jagged. Optionally, the shape of the apertures is selected for one or more of the following reasons: shape of extrusion, preventing failure of the aperture and/or preventing failure of the delivery tip. Optionally, the apertures have a lip (optionally pointing inwards), which may assist in shaping the extrusion. For example, the lip may be between 0.1 and 1 mm in width, for example, 0.3 mm or 0.5 mm.

The delivery tube may be rigid. Optionally, the delivery tube is flexible or is mechanically shaped (e.g., using a vise) before insertion. The cannula may be flexible and may allow the insertion of a delivery tube which is curved at its end.

The type of delivery tip may selected by a user. Optionally, the delivery tip is replaceable, for example attached by a threading to the delivery system.

Optionally, an overtube or ring is selectively provided over part of the delivery system to selectively block one or more of the apertures.

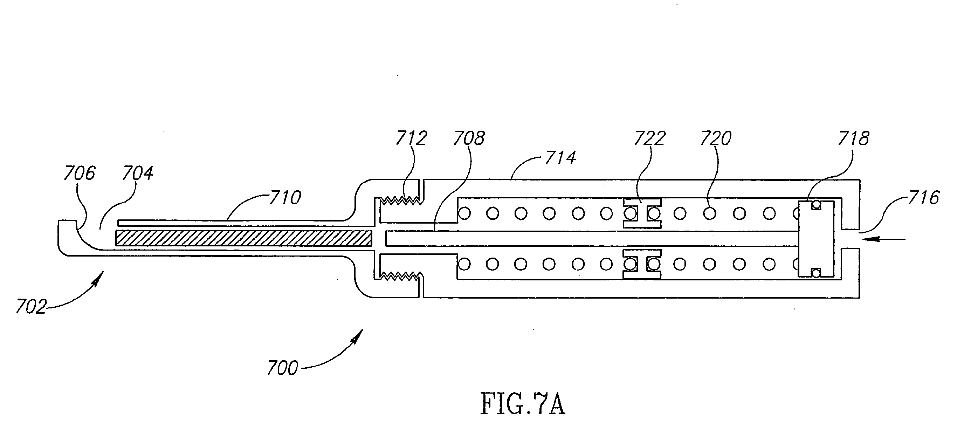

Referring briefly to Fig. 7A, a closed distal tip 702 of cannula 710 is shown, in which a guiding incline 706 is provided to guide the ejected material out of a lateral aperture 704. Optionally, the use of such an incline reduces turbulence in the flow/distortion of the material and/or may assist in reducing friction and/or improving control over the shape of the extrusion. Also to be noted is that material extrusion is provided on only one side of the delivery system. This may allow better control over the force vectors inside the vertebra, caused by the extrusion. The angles defined by the guiding incline (90 degrees and in the plane of the tube axis) may help to determine the extrusion direction.

Also shown in Fig. 7A is a non-twisting pusher 708, which may reduce turbulence, friction and/or other difficulties in extruding the material, such as voids.

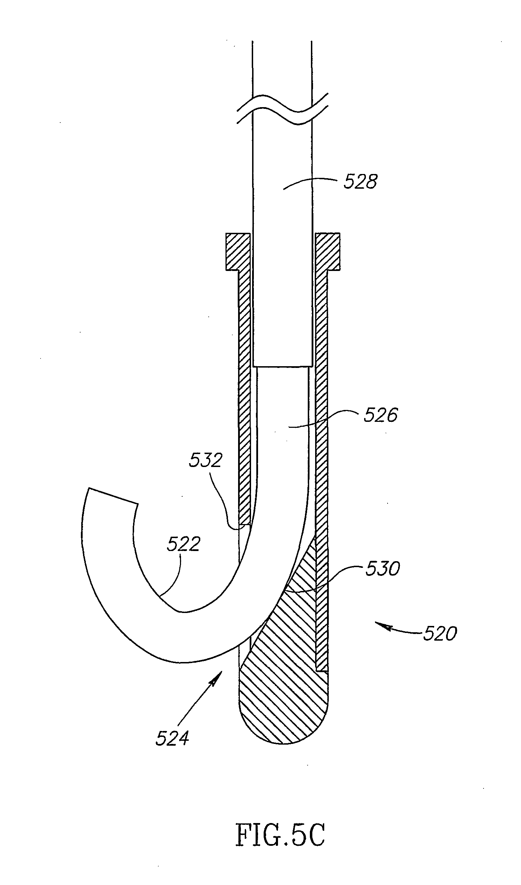

Fig. 5C shows a cannula delivery tip 520, from which a material 526 is extruded by a pusher 528 in a curved extrusion shape 522. The curvature may be controlled by controlling the relative friction on a proximal side 532 and on a distal sidle 530 of a lateral aperture 524. Alternatively or additionally, the degree of curvature depends on the size of the aperture and the shape of the incline. Optionally, the material is plastically deformed by the extrusion and may maintain a shape conferred thereby barring contact with a deforming surface (e.g., a bone plate). A distal tip of the cannula may be closed, optionally permanently closed, so that cement 522 is forced laterally outwards via aperture 524.

Alternatively or additionally, extrusion 522 can be curved or bent due to axial or rotational motion of tip 520. Optionally, the rotation is used to more uniformly fill space 308.

The delivery tube may move and/or rotate during delivery. Optionally, a gear mechanism couples movement of the pusher with rotation and/or axial motion of the tube. Optionally, a manual motion is provided by an operator. Optionally, a vibrator is coupled to the delivery system.

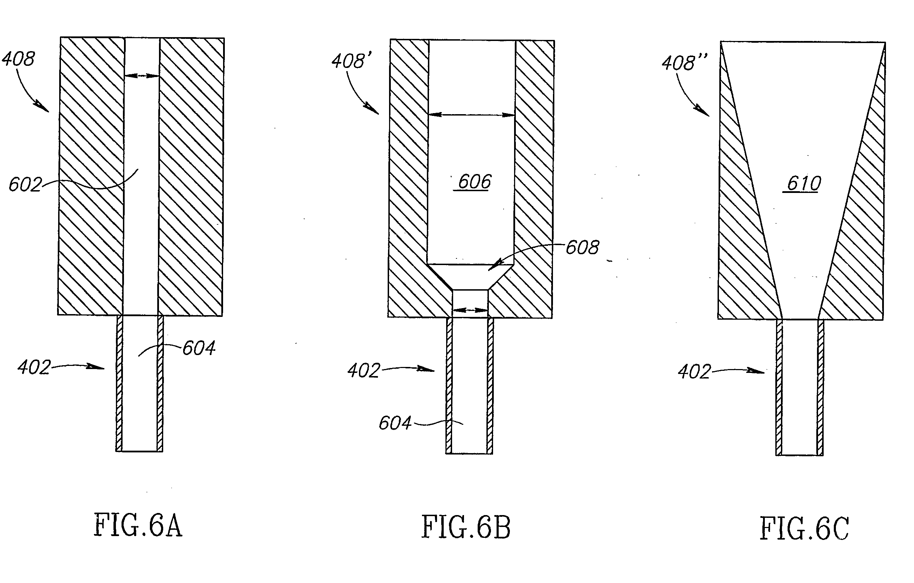

One consideration mentioned above, is that the amount of material in barrel 408 may not be sufficient for a complete procedure. A matching design is illustrated in Fig. 6A, in which the diameter of an inner lumen 602 of barrel 408 is the same as the diameter of an inner lumen 604 of delivery tube 402. A longer delivery tube/barrel maybe required to reduce the number of barrel changes.

Fig. 6B shows an alternative design, in which a barrel 408' has a lumen 606 with a greater inner diameter and thus a greater storage volume. Optionally, the greater diameter provides an additional hydraulic amplification factor as the diameter changes. Optionally, a sudden change in diameter may cause turbulence, resistance and/or void creation. In some materials, diameter change requires compression of the material. Optionally, as shown, a gradual change in diameter is provided, with an intermediate sloped section 608 with an inner diameter varying between the diameters of lumen 606 and 604. Optionally, the pusher has a diameter matching lumen 606 and does not fit into lumen 604. Optionally, an extension is provided to the pusher, which extension does fit in lumen 604.

Referring to Fig. 6C, a gradually changing lumen 610 is provided in a barrel 408". Optionally, the distal end of the pusher is made of a flexible material, which can conform to the change in diameter. Optionally, the flexible material is harder than the injected material. Alternatively or additionally, the distal end of the pusher is shaped to match the geometry of lumen 610.

The lumen of the barrel may be larger than the diameter of the pusher, at least in a proximal section of the barrel. After the pusher advances an amount of material into the bone, the pusher is retracted and the material remaining in the barrel is rearranged so that the next advance of the pusher will advance it. Optionally, the rearranging is by advancing a second plunger having a diameter similar to that of the barrel. Optionally, this plunger is coaxial with the pusher.

The delivery tube may have various cross-sectional shapes, for example, circular, rectangular, arcuate and/or square. Optionally, the cross-section is matched to the shape of extrusion apertures. Optionally, the inside of the apertures is made sharp to cut the extruded material as it is advanced, instead of or in addition to plastically deforming or shearing it.

**Exemplary Viscosity/Plasticity and Pressure**

The viscosity of the bone cement may be 500, optionally 1,000, optionally 1,500, optionally 2,000 Pascal-sec or lesser or greater or intermediate values at the time of loading to an injection system, optionally 3, or 2 or 1 minutes or lesser or intermediate values after mixing. Optionally, a cement with viscosity in this range is useful in vertebral repair, for example in vertebroplasty and/or kyphoplasty procedures. Use of a bone cement which is viscous at the time of injection reduces the risk of material leakage. Reduced leakage optionally contributes to increased likelihood of a positive clinical outcome.

In an exemplary embodiment of the invention, the bone cement is sufficiently viscous to move the bone as it is injected. Optionally, moving of the bone contributes to fracture reduction and/or restoration of vertebral height.

In an exemplary embodiment of the invention, the provided material has a viscosity of above 600 Pascal-second. Optionally, the material is advanced into the body using a pressure of at least 40 atmospheres or higher, for example, 100 or 200 atmospheres or more. If the material is plastic, it may have a hardness, for example, of between 10A shore and 100A shore and/or a Young modulus higher than 200 MPa.

Pressure requirements may be relaxed at a beginning of a procedure, for example, if a void is created by bone access or by rotation of the delivery system.

The outer diameter of the delivery system may be, for example, 2 mm, 3 mm, 4 mm, 5 mm or intermediate or smaller or larger diameters. Optionally, the wall thickness of the delivery system is 0.2 or 0.3 mm. Optionally, the wall thickness increases towards the distal tip

It should be noted that the pressure used for delivery may depend on one or more of: the friction between the material and the delivery system, the length of material being pushed, the pressure applied to the material, the pressure desired to be applied by the material to the vertebra, the manner in which the extrusion applies pressure against the vertebra, the viscosity of the material, an area of contact between the material and the cylinder and/or other causes of resistance to motion of the material.

Lower pressures may be used, for example, if it is deemed that the vertebra may be damaged or material leakage possible. The volume injected may be, for example, 2-4 cc for a typical vertebra and as high as 8-12 cc or higher. Other volumes may be appropriate, depending for example, on the volume of space 308 and the desired effect of the injection.

The rate of injection may be 0.25 cc/sec. Higher or lower rates may be provided, for example, between 25 cc/sec and 0.1 cc/sec or less, and between 25 cc/sec and 1 cc/sec or more. Optionally, the rate is controlled using electronic or mechanical circuitry. Optionally, the rate is decided by an operator responsive to expected or imaged bone deformation in response to the pressure. Optionally, the rate is changed over the length of the procedure, for example, being higher at a beginning and lower at an end. Optionally, the rate of injection is controlled by the operator (or automatically) responsive to a feedback mechanism, such as fluoroscopy.

**Fast setting cement**

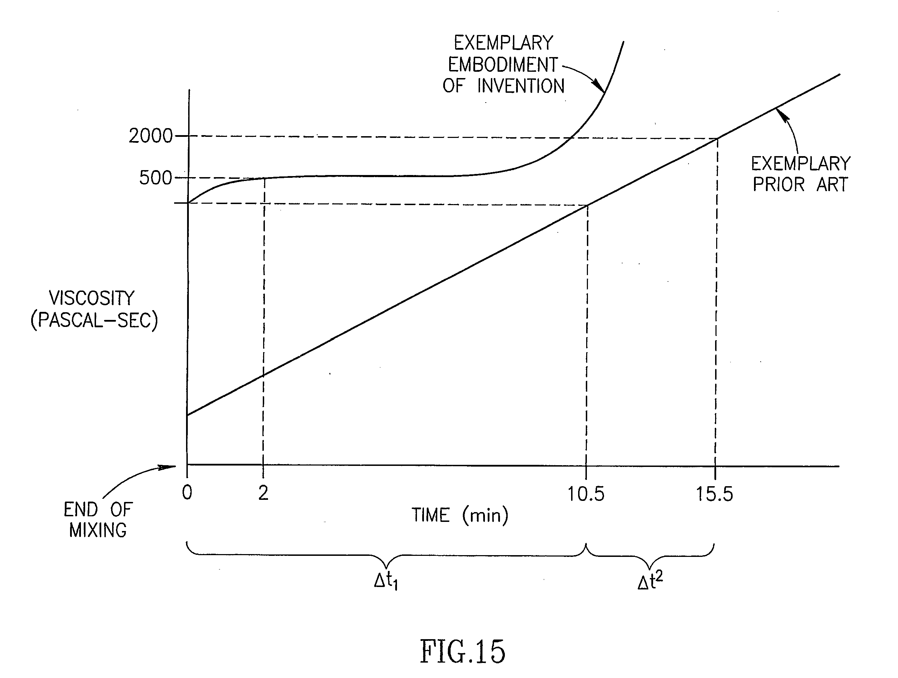

Fig. 15 is a plot of viscosity as a function of time for an exemplary bone cement according to the present invention. The figure is not drawn to scale and is provided to illustrate the principles of the invention. The end of a mixing process is denoted as time 0. In an exemplary embodiment, bone cement according to the invention enters a plastic phase upon mixing so that it has substantially no liquid phase. Optionally, mixture of the polymer and monomer components produces a material with viscosity of about 500 Pascal- second or more within 15, optionally 30, optionally 60, optionally 90, optionally 120 seconds or lesser or greater or intermediate times. In an exemplary embodiment of the invention, the material reaches a viscosity higher than 500 Pa-s within about 30 seconds after its components mixing. Once a high viscosity is achieved, the viscosity remains relatively stable for 5, optionally 8 minutes or more. This interval of stable viscosity provides a window of opportunity for performance of a medical procedure. A stable viscosity may mean that the viscosity of the cement changes by less than 200 Pascal-second during a window of at least 2 minutes optionally at least 4 minutes after mixing is complete. Optionally, the window begins 1, 2, 3, 6, or 8 minutes after mixing is complete or lesser or intermediate times. In an exemplary embodiment of the invention, the viscosity of the cement remains below 1500 Pascal-second for at least 4, optionally at least 6, optionally at least 8 optionally at least 10 minutes or intermediate or greater times.

An applied injection pressure in the cement reservoir may be reduced as the cement flows through the cannula. Optionally, friction between the cement and the cannula walls reduces pressure. Injection of the bone cement may be continuous. The term continuous as used here indicates that the greatest interruption in a flow of cement exiting a distal tip of the cannula is less than 5, optionally less than 2, optionally less than 1, optionally less than 0.5, optionally less than 0.1 seconds or lesser or intermediate times. In Fig. 15 the end of a mixing process is denoted as time 0. Mixing is deemed to end when all acrylic polymer beads have been wetted with monomer.

For purposes of comparison, the graph illustrates that an exemplary prior art cement reaches a viscosity suitable for injection at a time of approximately 10.5 minutes post mixing and is completely set by about 15.5 minutes.

A window of opportunity for injection with an exemplary cement according to the invention ($\Lambda t_1$) is both longer and earlier than a comparable window for an exemplary prior art cement ($\Lambda t_2$). Optionally, ($\Lambda t_1$) begins substantially as soon as mixing is complete. In an exemplary embodiment of the invention, the cement has substantially no time in a liquid phase before entering a plastic phase.

**Viscosity measurements over time for exemplary fast setting cements**

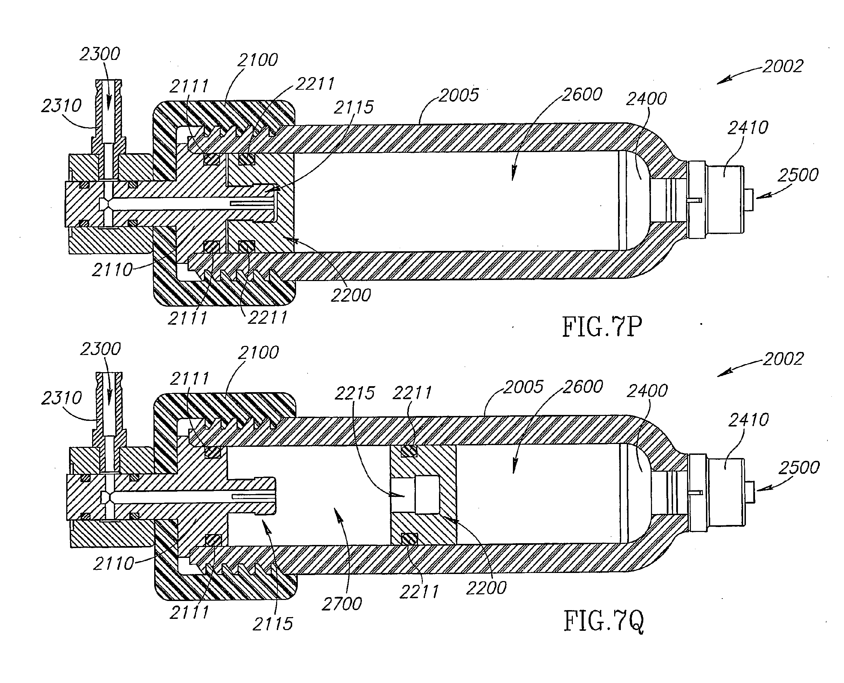

In order to evaluate the viscosity profile of different exemplary batches of cement according to some embodiments of the invention, a bulk of pre-mixed bone cement is placed inside a Stainless Steel injector body (e.g. 2002 of Fig. 7P and 7Q). Krause et al. described a method for calculating viscosity in terms of applied force. ("The viscosity of acrylic bone cements", Journal of Biomedical Materials Research, (1982): 16:219-243).

In the experimental apparatus inner diameter of injection chamber 2600 is approximately 18 mm. The distal cylindrical outlet 2500 has inner diameter of approximately 3 mm and a length of more than 4 mm. This configuration simulates a connection to standard bone cement delivery cannula / Jamshidi needle. A piston 2200 applies force (F), thus causing the bone cement to flow through outlet 2500. Piston 2200 is set to move with constant velocity of approximately 3mm/min. As a result, piston deflection is indicative of elapsed time.

The experimental procedure serves as a kind of capillary extrusion rheometer. The rheometer measures the pressure difference from end to end of the capillary tube. The device is made of a 18mm cylindrical reservoir and a piston. The distal end of the reservoir consists of a 4mm long 3 mm hole. Assuming steady flow, isothermal conditions and incompressibility of the tested material, the viscose force resisting the motion of the fluid in the capillary is equal to the applied force acting on the piston measured by a load cell. Results are presented as force vs displacement. As displacement rate was constant and set to 3mm/min, the shear rate was constant as well. In order to measure the time elapses from test beginning, the displacement rate is divided by 3 (jog speed).

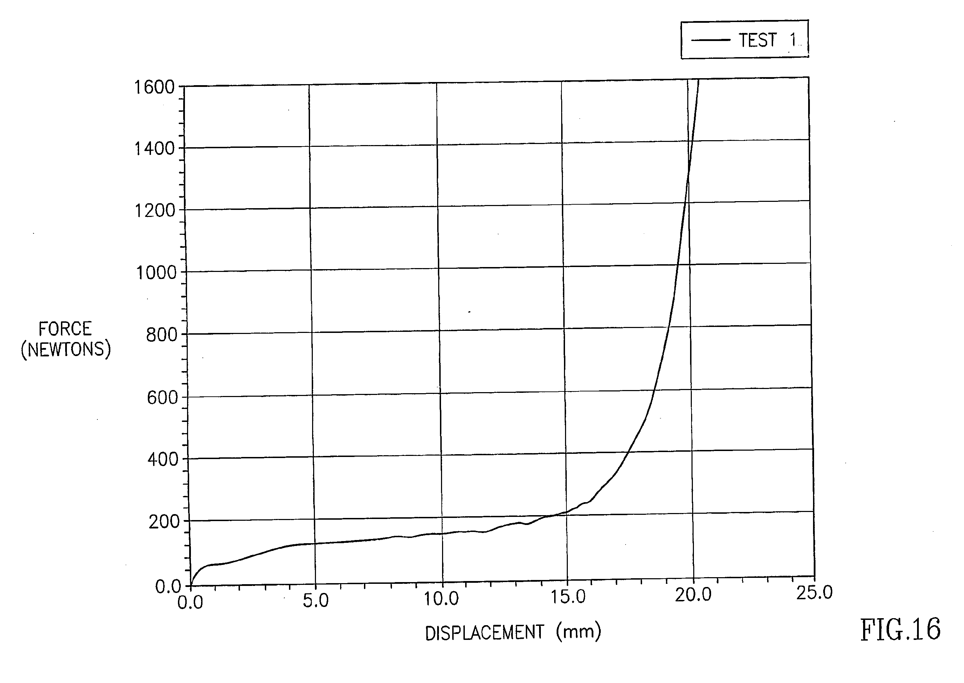

Fig.16 indicates a viscosity profile of a first exemplary batch of cement according to the invention as force (Newtons) vs displacement (mm). The cement used in this experiment included a liquid component containing approximately 98.5% v/v MMA, approximately 1.5% DMPT and approximately 20 ppm of Hydroquinone and a powder component containing approximately 69.39% w/w PMMA, approximately 30.07% Barium Sulphate and approximately 0.54% of Benzoyl Peroxide. The average molecular weight of the PMMA was approximately 110,000 Dalton. Approximately 1% of the PMMA had a molecular weight of 700,000 to 1,000,000 Dalton. The bead size was in the range 10-200 microns.

In this test (Average temperature: 22.3°C; Relative Humidity: app. 48%) the cement was mixed for 30-60 seconds, then manipulated by hand and placed inside injector 2002. Force was applied via piston 2200 approximately 150 seconds after end of mixing, and measurements of force and piston deflection were taken.

At a time of 2.5 minutes after mixing (0 mm deflection) the force applied was higher than 30 N.

At a time of 6.5 minutes after mixing (12 mm deflection) the force applied was about 150 N.

At a time of 7.5 minutes after mixing (15 mm deflection) the force applied was higher than 200 N.

At a time of 8.5 minutes after mixing (18 mm deflection) the force applied was higher than 500 N.

At a time of 9.17 minutes after mixing (20 mm deflection) the force applied was higher than 1300 N.

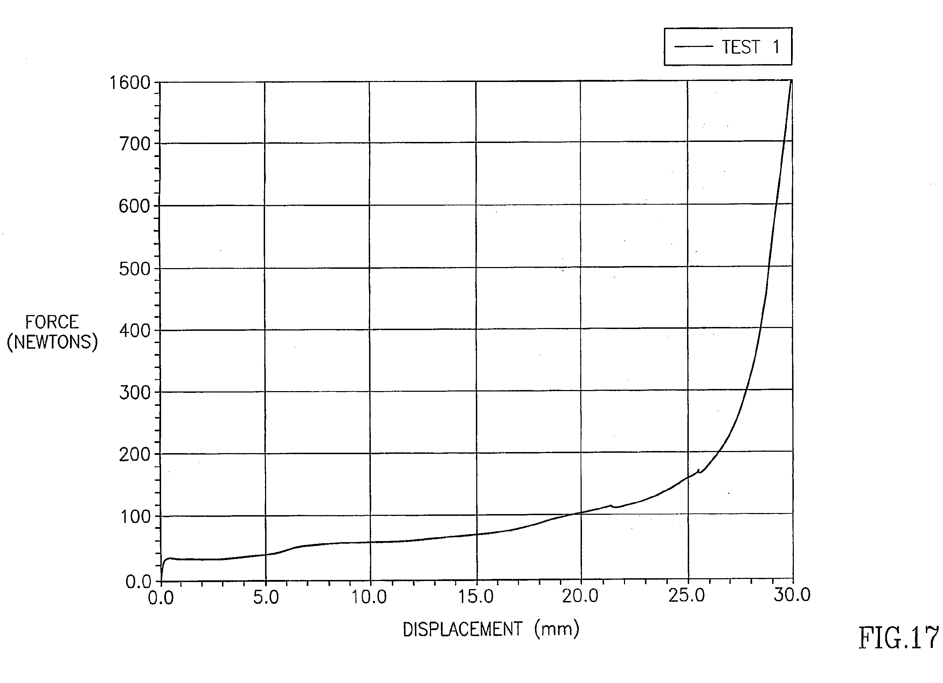

Fig. 17 indicates a viscosity profile of an additional exemplary batch of cement according to the invention as force (Newtons) vs displacement (mm). The cement in this test was prepared according to the same formula described for the experiment of Fig. 16. In this test (Average 21.1°C; Relative Humidity: app. 43%) the cement was mixed for approximately 45 seconds, then manipulated by hand and placed inside injector 2002. Force was applied via piston 2200 approximately 150 seconds after end of mixing, and measurements of force and piston deflection were taken.

At a time of 2.25 minutes after mixing (0 mm deflection) the force applied was higher than 30 N.

At a time of 8.25 minutes after mixing (18 mm deflection) the force applied was about 90 N.

At a time of 10.3 minutes after mixing (25 mm deflection) the force applied was higher than 150 N.

At a time of 11.4 minutes after mixing (28.5 mm deflection) the force applied was higher than 500 N.

At a time of 12.25 minutes after mixing (30 mm deflection) the force applied was higher than 800 N.

Results shown in Figs. 16 and 17 and summarized hereinabove illustrate that exemplary bone cements according to some embodiments the invention are ready for injection in as little as 2.25 minutes after mixing is completed. Alternatively or additionally, these cements are characterized by short mixing time (i.e. transition to plastic phase in 30 to 60 seconds). The exemplary cements provide a "working window" for injection of 4.5 to 6.3 minutes, optionally longer if more pressure is applied. These times correspond to delivery volumes of 14.9 and 20.8 ml respectively. These volumes are sufficient for most vertebral repair procedures. These results comply with the desired characteristics described in Fig. 15. Differences between the two experiments reflect the influence of temperature and humidity on reaction kinetics.

**Hydraulic material provision system**

Fig. 7A shows a delivery system 700 which is powered hydraulically. A cannula 710 is filled with material to be ejected into the body. Cannula 710 is optionally detachable via a connection 712 to a body 714. Optionally, the connection is by threading. Alternatively, a fast connection method, such as a snap connection, is used. Body 714 converts hydraulic pressure provided via an input port 716 into an advance of a pusher rod 708. Optionally, body 714 is integral with tube 710, but this prevents replacing tube 710 when the material to be ejected is exhausted.

Incoming hydraulic (or pneumatic) fluid may push against a piston 718, which advances pusher 708 directly. Optionally, a hydraulic advantage is provided by the ratios of the piston and the pusher. Optionally, a spring 720 is provided for retracting pusher 708 when the fluid pressure is released.

Optionally, one or more spacers 722 are provided surrounding pusher 708, to prevent buckling thereof. Optionally, the spacers are mounted on spring 720. Optionally, spacers are provided at several axial locations. Alternatively to spacers, fins may extend from pusher 708 to body 714.

Optionally, in use, when material is used up, pressure is reduced, pusher 708 retracts and delivery tube 710 is replaced. Optionally, a barrel filled with material for injection, separate from tube 710 is provided, so that tip 702 does not need to be removed from the body.

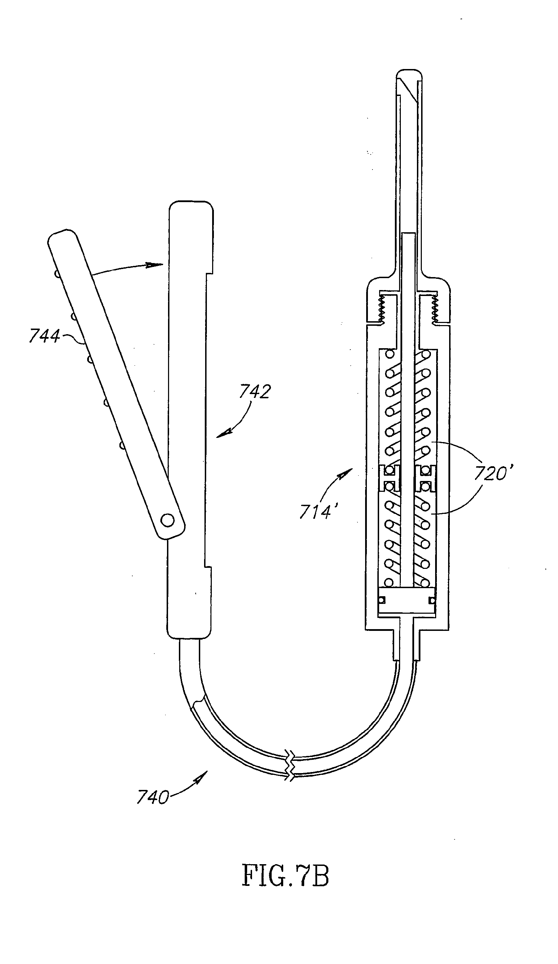

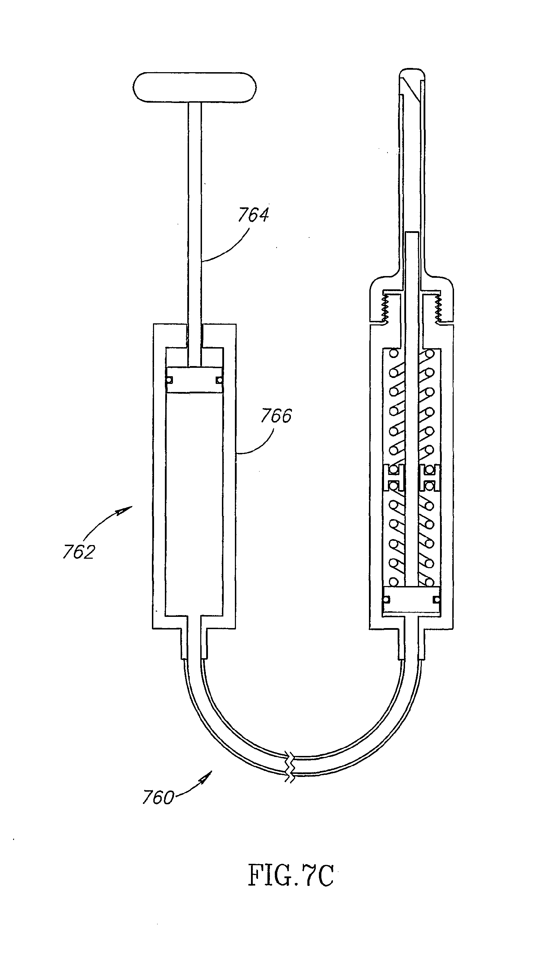

Figs. 7B and 7C show two alternative methods of providing hydraulic power. In Fig. 7B, a foot pedal pump 740 is used, in which a user places his foot on a pedal 744 and depresses it against a plate 742. Various foot pumps are known in the art. Optionally, a long press releases the pressure. Optionally, the hydraulic subsystem is a sealed system which is provided ready to use (e.g., including fluid) to the user and/or distributor. Exemplary lengths of the flexible tubing are between 0.2 and 3 meters, for example, between 1 and 2 meters. However, greater lengths can be used as well.

A foot operable actuator may employ a 1.5 m, optionally 2 m, optionally 2.5 m tube or a tube of lesser or intermediate or greater length (see Fig. 7B).

A hand operable actuator may employ a 0.25m, optionally 0.5m, optionally 1.0 m tube or a tube of lesser or intermediate or greater length.

Also shown in Fig. 7B is a variant of body 714, indicated as 714'. Instead of a single spring 720, two springs 720' are shown, with the spacer(s) between the springs. Optionally, the use of multiple springs helps maintain the spacers near a middle (or other relative length unit) of the pusher in danger of buckling.

Fig. 7C shows an alternative arrangement in which a hand pump 760 is used, which pump can be of any type known in the art, for example, a mechanism 762 comprising a piston 764 and a cylinder 766. Optionally, the pumping is by rotating piston 764 relative to cylinder 766, which components include matching threading. Alternatively, linear motion is used. Optionally, a hydraulic gain is achieved between the pump and the delivery mechanism, for example a gain of 1:3, 1:5, 1:10 or any smaller, intermediate or greater gain.

The hydraulic system may be provided as a disposable unit, with a non-disposable (or a disposable) foot pump.

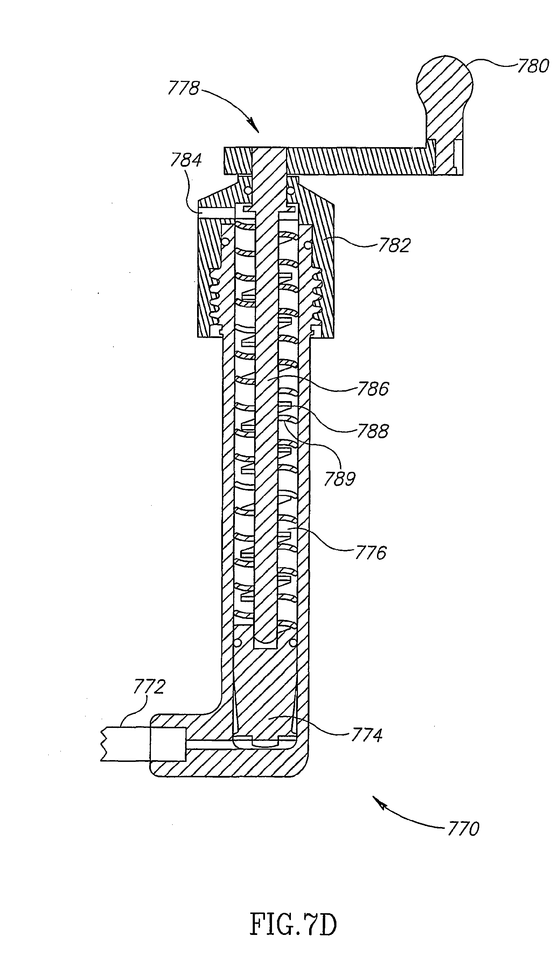

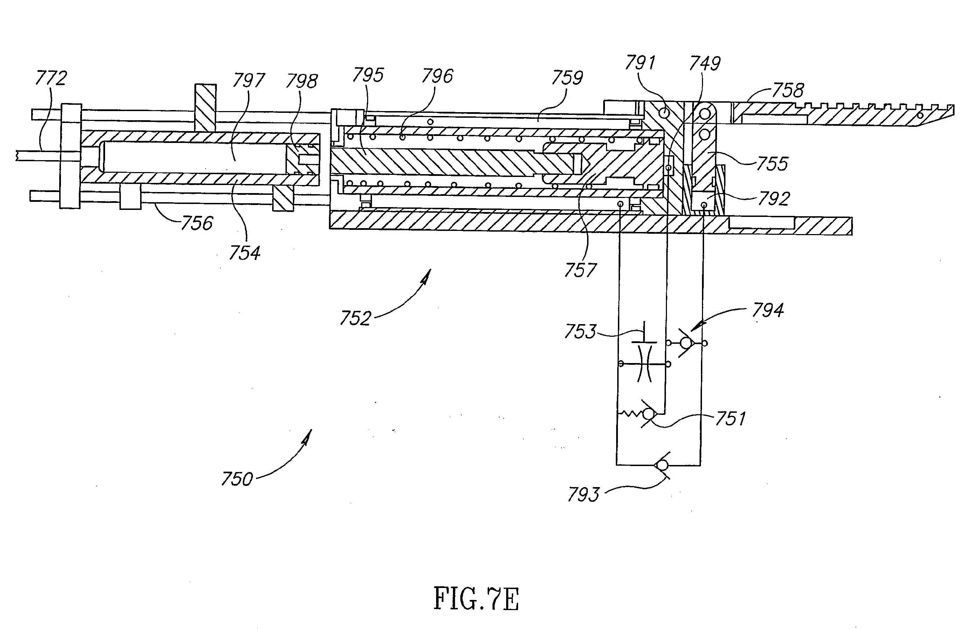

Fig. 7D shows a disposable mixing and storage chamber 770 and Fig. 7E shows a reusable pump 750 with a disposable hydraulic capsule 754.

Referring to Fig. 7D, a same capsule 770 is optionally used both for mixing and for storage/delivery of a material. Optionally, the material is a setting cement such as PMMA. In a hydraulic delivery stream, a flexible tube 772 is optionally permanently connected to a pump (Fig. 7E). When fluid is provided through tube 772, a piston 774 moves through a cylinder volume 776 and pushes the material out (e.g., and into a delivery system). In the figure, the capsule is shown loaded with a mixer 778. Optionally, materials are provided into volume 776 using a detachable funnel (not shown) and then the funnel is removed and mixer 778 inserted instead. In the exemplary mixer shown, a cap 782 covers cylinder 776. When mixing is completed, this cap may be replaced by a fitting adapted to couple to the delivery tube.

In use, a handle 780 is rotated, rotating a shaft 786 having a rotor 788 defined thereof, for example, as a helix. An optional stator 789 is provided. An optional vent 784 may be connected to a vacuum source, to suck out toxic and/or bad smelling fumes caused by the setting of the material. Optionally, a viscosity of the materials is estimated by the difficulty in turning the handle. Optionally, the handle includes a clutch (not shown) that skips when a desired viscosity is reached. Optionally, the clutch is settable. Optionally, a viscosity meter is used or viscosity is estimated based on temperature, formulation and time from mixing. Cap 782 optionally includes a squeegee or other wiper, to wipe material off of mixer 778 when it is removed from capsule 770.

Referring to Fig. 7E, tube 772 connects to a capsule 754 which includes a piston 798 and a volume 797, pre-filled with fluid. A frame 756 may be provided attached to pump 750 for selectively receiving capsule 754.

Pump 750 is, for example, a hydraulic oil based pump-mechanism 752 that extends a pushing rod 795 which advances piston 798.

In the arrangement shown, a foot pedal 758, attached to an axis 791, forces a piston 755 into a cylinder 792. A one way valve 794 allows the fluid in cylinder 792 to flow into a volume 749 where it pushes against a piston 757. When pedal 758 is released, a spring (not shown) pulls it back to an upward position and allows a hydraulic fluid to flow from a storage chamber 759 (e.g., which surrounds the pump) through a one way valve 793 into cylinder 792.

A pressure relief valve 751 is optionally provided to prevent over pressurizing of cylinder 749. A spring 796 may be provided to push back piston 757 and pusher 795 with it, when pressure is released. Optionally, pressure is released using a bypass valve 753, which is manually operated. Once pusher rod 795 is retracted, capsule 740 is optionally removed.

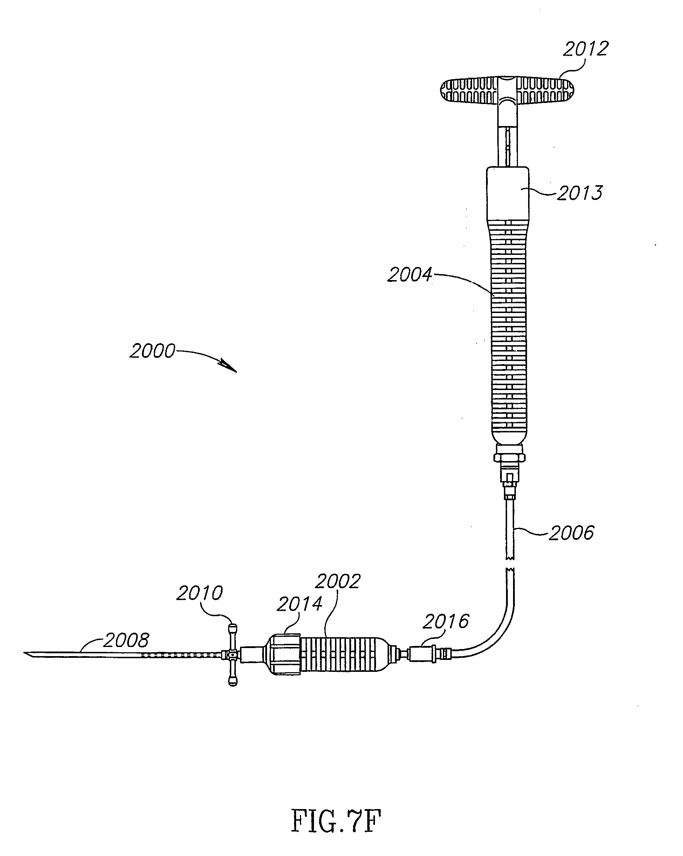

Figs. 7F (side view) and 7G (cross section) illustrate a hydraulic delivery system 2000, comprising a reservoir 2002 for material (e.g. bone cement) and a pressure source 2004. Optionally a flexible tube 2006 which connects reservoir 2002 and pressure source 2004 is included in system 2000. Reservoir 2002 is connectable to a cannula 2008. Cannula 2008 can optionally be introduced into a bone prior to injection of material from reservoir 2002. Reservoir 2002 and cannula 2008 may be connected using, for example, a luer lock connector 2010 and/or threaded connector 2014. Alternately, reservoir 2002 and cannula 2008 may be fashioned as a single piece. Optionally, cannula 2008 is compatible with a stylet (not shown).

Cannula 2008 may be a plastically deformable cannula, optionally a slitted cannula. Plastically deformable cannulae in general, and slitted cannulae in particular are described in detail in co-pending US application 60/721,094 entitled "Tools and Methods for Material Delivery into the Body", and in co-pending application entitled "Cannula" identified as attorney docket number 110/05085 and filed concurrently with the instant application. Optionally, use of a plastically deformable cannula 2008 facilitates positioning of reservoir 2002 outside of an X-ray imaging field.

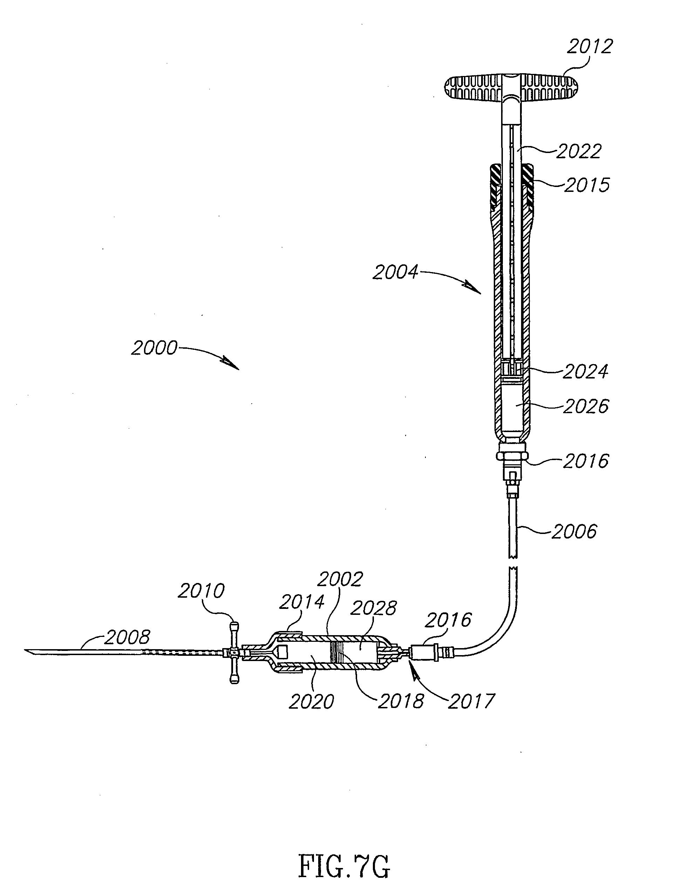

Pressure source 2004 may be a hydraulic pressure source. Pressure source 2004 may be filled with any liquid. Pressure source 2004 may be filled is filled with a sterile liquid 2026 (Fig. 7G), such as saline or water. Optionally, filling is via flexible tube 2006. Optionally, an outlet of tube 2006 is immersed in the liquid, and hydraulic actuator 2012 is operated to fill pressure source 2004 with liquid. In Figs. 7F and 7G, the actuator is depicted as a ball screw connection 2013 equipped with a rotating handle 2012. Handle 2012 is rotated to fill pressure source 2004 with liquid.

Pressure source 2004 may be provided as a prefilled unit. Optionally the prefilled unit includes body 2004, tube 2006 and/or cap 2013 and/or handle 2012 with drive shaft 2022. Optionally the prefilled unit includes body 2004, optionally with tube 2006 and is provided with temporary seals at each end. The seals may be removed or broken when additional system components are connected.

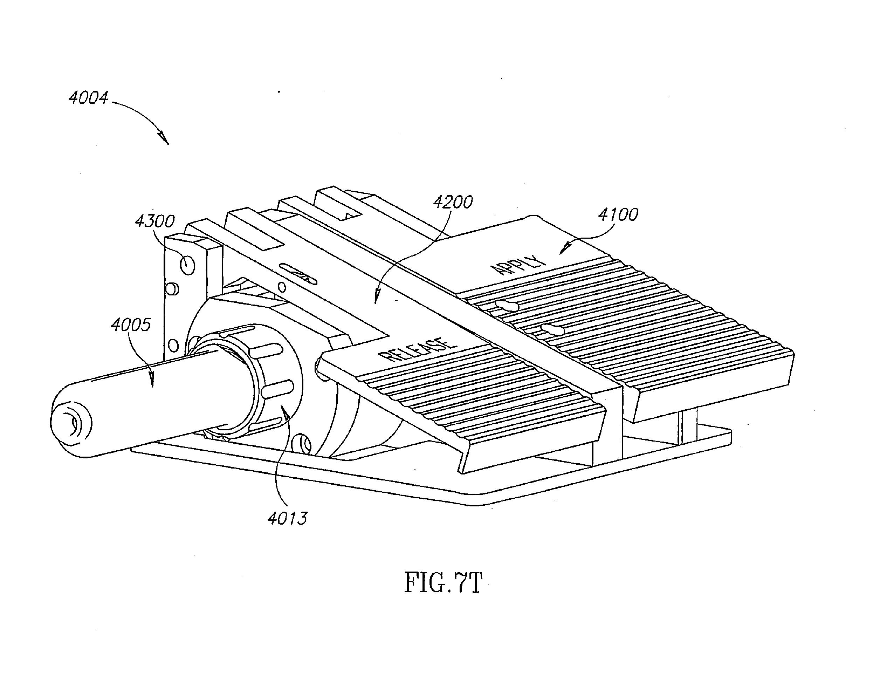

Although an exemplary manually operable actuator is pictured, hydraulic actuator 2012 may be operable by, for example, a foot pedal (see Fig. 7T described hereinbelow) or an electric actuator, such as a linear actuator or a motor. Electric operation may be achieved, for example, by employing a battery (as described hereinbelow) and/or mains supply. The hydraulic actuator may cause pressures source 2004 to generate a pressure of 50, optionally 100, optionally 150, optionally 200, optionally 300 atmospheres or lesser or greater or intermediate values.

Optionally, a safety mechanism (described in greater detail hereinbelow) limits pressure. The safety mechanism may include a valve with a defined pressure threshold. The threshold may be set to prevent injection of cement after it has solidified and/or to protect the system from being damaged by attempts to inject solidified cement.

For example if a particular cement formulation is flowable at a pressure of 150 atmospheres during the "working window", a valve threshold may be set at 170 atmospheres. In such a case, the system would be designed to withstand a greater internal pressure, such as 200 atmospheres. This arrangement reduces the chance that the system will be damaged when cement solidifies.

Optionally, the pressure threshold is 150 or 210 atmospheres or lesser or greater or intermediate values. The safety mechanism may be used to release trapped gases (e.g. air) and/or cement. Each activation of the hydraulic actuator (e.g., handle rotation, foot pedal pressing) may result in injection of defined amount of cement. Optionally, the hydraulic actuator provides pressure amplification.

Optionally, air is removed from pressure source 2004 and/or tube 2006 prior to connection of pressure source 2004 to reservoir 2002. One or more connectors 2016 can be optionally employed to connect of reservoir 2002 to pressure source 2004, optionally via tube 2006. Connectors 2016 may be, for example, luer lock connectors, quick release connectors or threaded connectors.

Delivery system 2000 may be employed to deliver a viscous material, optionally a viscous bone cement. Cement components (e.g. powder and liquid) may be mixed. The mixture is loaded into reservoir 2002, optionally via a reservoir cap 2014. Optionally cap 2014 is unscrewed and reservoir 2002 is filled with bone cement 2020 (Fig. 7G). Cement 2020 may be inserted into reservoir 2002 manually or by use of a tool or filling device. Cement 2020 may be sufficiently viscous that it may be preformed to a size and shape which permit easy insertion into reservoir 2002. After cement 2020 is inserted in reservoir 2002, cap 2014 is replaced. Pressure source 2004 is connected to reservoir 2002, optionally via flexible tube 2006, via connector(s) 2016. Pump 2004, tube 2006, connector 2016 and fluid 2026 may be provided as a pre-assembled sterile unit. Optionally, air is removed from the system via a pressure release valve 2017, optionally on connector 2016. Optionally rotation of handle 2012 increases pressure in the system and drives air out. Optionally, air is released through valve 2017 or is expelled via cannula 2008 prior to insertion of the cannula in the body. Optionally, mixing of cement components is performed under vacuum to prevent air bubbles from being entrapped in the cement.

Reservoir 2002 is connected to cannula 2008 via connector 2010. Connector 2010 may serve as an orientation marker which indicates an ejection direction of cement injected through the cannula. Operation of handle 2012 delivers cement 2020 from reservoir 2002 to cannula 2008. Rotation of handle 2012 may rotate pump thread 2022 and advance piston 2024.

Advancing piston 2024 applies pressure to liquid 2026 within pressure source 2004 and causes liquid 2026 to advance into reservoir 2002, optionally via tube 2006. Liquid 2028 in reservoir 2002 applies pressure to a piston 2018 within reservoir 2002. As piston 2018 advances through reservoir 2002, it causes cement 2020 to advance and exit reservoir 2002 via the cannula 2008.

Cannula 2008 may be equipped with one or more apertures for cement delivery. Optionally, these apertures are located at a distal end and/or near the distal end of cannula 2008. Optionally, the apertures face axially and/or radially with respect to the cannula. Optionally, the cannula distal end is closed. One or more lateral openings on the cannula may permit sideways injection to a desired target in a bone from a cannula with a permanently closed distal tip.

The injection procedure may be monitored by a medical imaging system (e.g. fluoroscopy). When a desired amount of cement 2020 has been delivered through cannula 2008, injection is stopped. Optionally, reservoir 2002 is disconnected from cannula 2008 and/or tube 2006 and/or pressure source 2004. Cannula 2008 is removed from the bone, and the operation site is closed.

Cannula 2008 and/or cement 2020 may be composed of biocompatible materials. Components of system 2000 which contact cement 2020 are not adversely affected by the cement. For example if MMA is employed as a component of the cement, reservoir 2002 may be constructed of an MMA monomer resistant polymer/Plastic while cannula 2008 may be constructed of Stainless Steel. Reservoir 2002 may be made of, for example, nylon, pressure source 2004 may be made of, for example, metal and/or plastic (e.g. polycarbonate), and the flexible tube 2006 is made of, for instance, nylon or Teflon[R].

Reservoir 2002 and/or pressure source 2004 may be constructed of Amorphous Nylon (e.g. Nylon Nos. 6, 6/6 or 12, e.g. Grilamid 90 or Durethan) and/or of a Cyclic Olefin Copolymer (COC) (e.g., Topas[R]; Ticona GmbH, Kelsterbach, Germany). These materials are resistant to cement components, including the monomer component.

Reservoir 2002 may be designed to withstand pressures in the range of 100 to 300 atmospheres. Optionally, a reservoir with an internal diameter of 18 mm was constructed with a wall thickness of 5mm so that the outer diameter is 28mm. Optionally, the walls are ribbed to increase strength and/or reduce weight.

The pressure source 2004 will come in contact only with the hydraulic fluid, such as water or a saline solution. Optionally, pressure source 2004 is constructed of Polycarbonate and/or polysulphone and/or PEEK or other materials which are not corroded by the hydraulic fluid.

Optionally, system 2000 employs a pressure of at least 100, optionally 150, optionally 200, optionally 300 atmospheres or lesser or intermediate or greater values to inject cement 2020. System 2000 may be constructed to withstand these operational pressures. The actual pressure which accumulates in the system may vary, for example as the viscosity of cement 2020 varies. Various types of connectors and/or pressure sources and/or reservoirs and/or cannulae may be employed depending upon an anticipated pressure. One of ordinary skill in the art will be able to select commercially available components such as connectors, tubing and O-rings which are suitable for use in construction of a system 2000 with a given anticipated operating pressure. Pressure may be provided by a tamping instrument including a rod adapted to comply with a lumen of the cannula (see Fig. 7A). Pressure may be provided by a piston (e.g. 2018 in Fig. 7G) with a diameter wider than the cannula lumen.

It is stressed that the combination of high viscosity cements employed in the context of the invention and the small diameter of the cannula to be filled renders standard syringes or other non-amplifying pressure sources ill-suited for cannula filling. Optionally, the viscous cement is manually manipulated to facilitate loading of the delivery device reservoir as described hereinbelow under "Transfer of viscous materials".

In those arrangements in which a tamping rod is employed, it may optionally be introduced into the vertebra via a working sleeve characterized by a slightly larger diameter than the cannula diameter.

Optionally, reservoir 2002 is transparent to permit visualization of cement 2020. A transparent reservoir 2002 may be marked with graduations indicating the amount cement 2020 in the reservoir. Optionally, this permits a user to ascertain how much cement is being injected.

Pressure source 2004, optionally attached to tube 2006, may be provided filled with liquid. Optionally, air has already been flushed from these components. Such an arrangement may expedite operating room procedures. According to this arrangement, a quick connector 2016, connecting the reservoir 2002 to the flexible tube 2006, is equipped with a uni-directional valve 2017 that seals the tube 2006 until it is connected to the reservoir 2002.

Optionally, a uni-directional valve (not shown in the Figures) is incorporated into the reservoir piston 2018, and is opened/released toward reservoir portion that does not contain cement 2028. For example, systems of the type illustrated in fig 7G, the cement is loaded from a distal side (covered by cap 2014) of container 2002. Prior to attaching connector 2016, air can be drained by a one-way valve in piston 2018, so entrapped air can flow from container 2020 through the one-way valve in piston 2018, and through escape valve 2017.

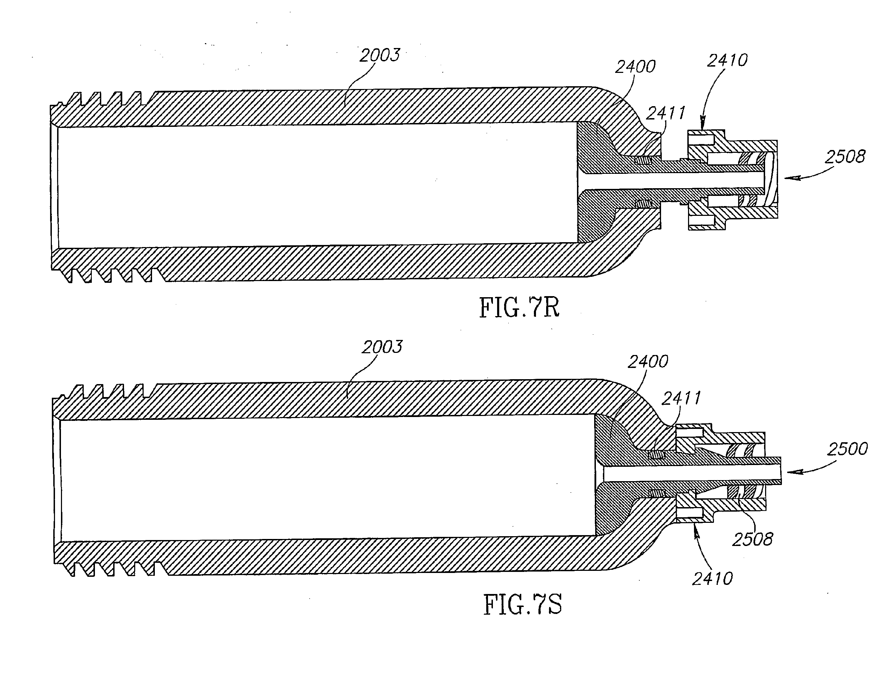

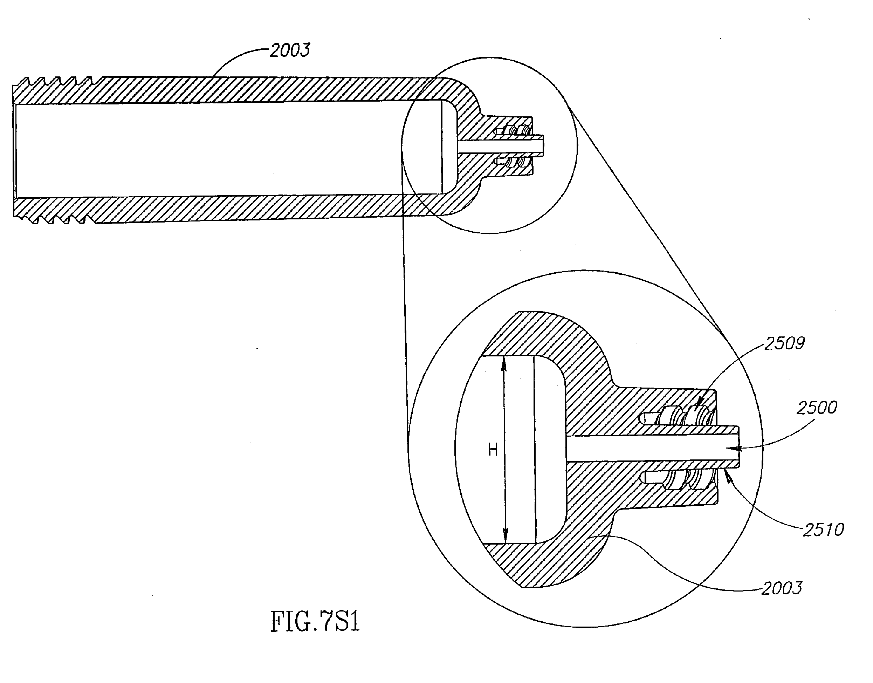

In Figs. 7P and 7S air escapes from opening 2500 directly. Optionally, no valves are provided. Although a hand operated handle is pictured, handle 2012 may be replaced by a foot pedal that is used to actuate piston 2024. Alternatively or additionally, pressure source 2004 may rely upon electric power for actuation. Electric power may be supplied, for example, by a battery. A battery powered motor may turn screw threads 2022 to advance piston 2024.

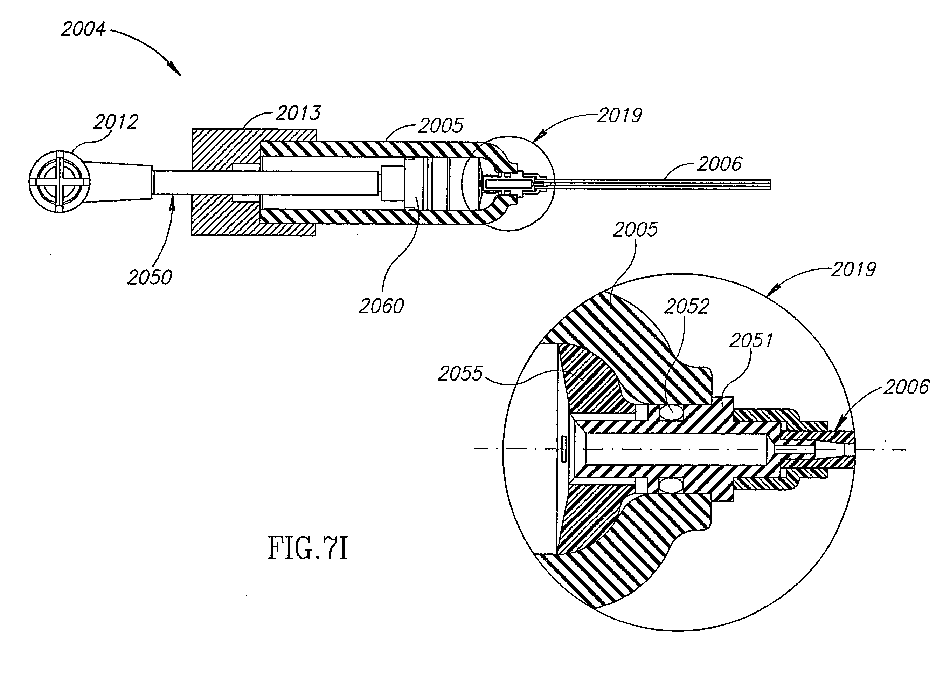

The construction and operation of exemplary hydraulic pressure sources for use in systems of the general type depicted in Figures 7F and 7G is depicted in greater detail in Figs. 7I through 7O.

**Exemplary pressure source**

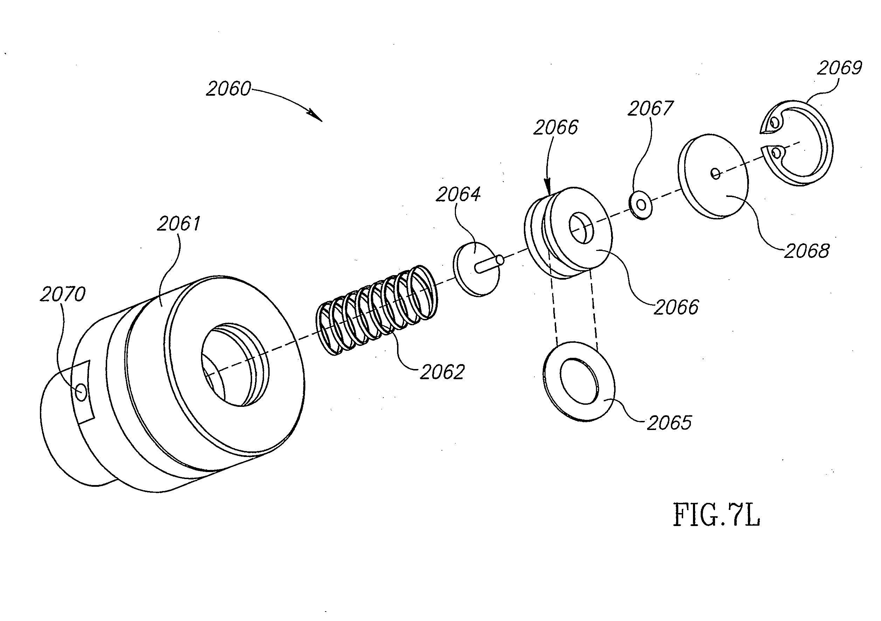

Fig. 7L illustrates a hydraulic pressure source 2004 including an actuation handle 2012 connected to a drive shaft 2050 and piston 2060. Drive shaft 2050 passes through hydraulic chamber cap 2013 and is insertable in hydraulic pressure body 2005. Piston 2060 is connected to, or constructed as part of, a distal end of drive shaft 2050. Piston 2060 forms a circumferential seal with respect to an inner surface of a lumen of hydraulic pressure body 2005. Hydraulic pressure body 2005 may be constructed of Amorphous Nylon or Polycarbonate.

A small step of the threading (eg 1 mm/rotation), a small radius of bolt (eg 4mm) and materials employed in construction for bolt and nut (eg Nylon for nut 2013 and stainless steel for bolt 2050) may produce a low friction coefficient.

Amorphous Nylon provides the requisite strength to resist high internal pressures with a low weight when compared to previously available steel hydraulic pressure sources. An amorphous nylon reservoir with an OD of 20, optionally 25, optionally 30, optionally 35 mm (or lesser or intermediate or greater values) may resist an internal pressure of as much as 300 atmospheres or more. Optionally, it can be transparent. Optionally, a transparent pressure body 2005 allows an operator to observe progress of piston 2060. Progress of the piston may be gauged against calibration markings on body 2005.

Pressure body 2005 may be connectable to a flexible tube 2006. Optionally, tube 2006 is glued to reservoir 2002 directly (eg by UV curing glue). The inset 2019 shows the connection in greater detail. A funnel 2055 is seated in a distal aperture of hydraulic pressure body 2005 and sealed by means of an O-ring 2052 and adapter plug 2051. As pressure on hydraulic fluid in hydraulic pressure body 2005 increases, funnel 2055 is more firmly seated against the distal aperture of hydraulic pressure body 2005. Optionally, this arrangement spreads the stresses radially over a distal end of the reservoir. Optionally, this arrangement prevents leaks at operating pressures of 100 to 300 atmospheres. The liquid is forced through tube 2006 as the pressure increases. A hydraulic unit including body 2005, cap 2013, drive shaft 2050 and piston 2060 may be provided as a unit pre-filled with a sterile liquid. Optionally, walls 2005 are transparent so that piston 2060 is visible to an operator of the unit. Transparent walls 2005 may be marked with graduations which indicate an injected volume of cement.

Specific materials for the cover 2013 and drive shaft 2050 may be chosen to reduce the friction coefficient ($\mu$). Optionally, cover 2013 is made of a polymer/plastic while shaft 2050 is made of steel. Optionally, Radius of Friction (R) is reduced by reducing the thread diameter on shaft 2050 and/or cap 2013.

$$M=R.f$$
$$f = \mu.F$$

So M=R*$\mu$*f. Therefore, a reduction in the moment needed for every normal force F is optionally achieved by reducing R and/or $\mu$.

Working moment (M) is the product of R and the radial force (f) of the hydraulic liquid. The force (F) between cap 2013 and shaft 2050 is the axial force applied on the piston 2060.

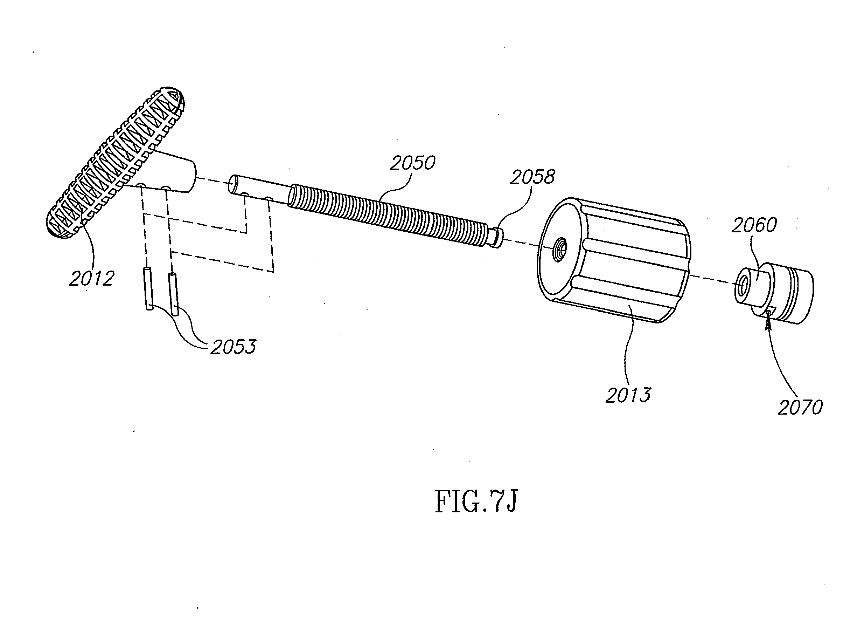

Fig. 7J is an exploded view of a handle and piston assembly as depicted in Fig. 7I. Pins 2053 anchor handle 2012 to drive shaft 2050. Optionally pins 2053 are designed to break if excessive torque is applied. Breaking of pins 2053 may be a safety feature. Cap 2013 and shaft 2050 are each threaded so that rotation of handle 2012 can cause shaft 2050 to advance or recede through cap 2013. Optionally, cap 2013 is constructed of a plastic polymer. Optionally, shaft 2050 is constructed of metal, for example stainless steel. Threaded shaft 2050 may be constructed of stainless steel and has a diameter of 6 mm, optionally 5 mm, optionally, 4 mm or lesser or greater or intermediate diameters. Optionally, stainless steel threads mated to plastic polymer threads provide a low friction connection which makes it easy to operate handle 2012 manually. A narrow diameter drive shaft 2050 may be employed as a means of reducing the amount of friction against threads of cap 2013. Threads on shaft 2050 and 2013 may each engage a set of ball bearings to provide a ball screw mechanism. In this figure, an optional pressure release port 2070 on piston 2060 is visible.

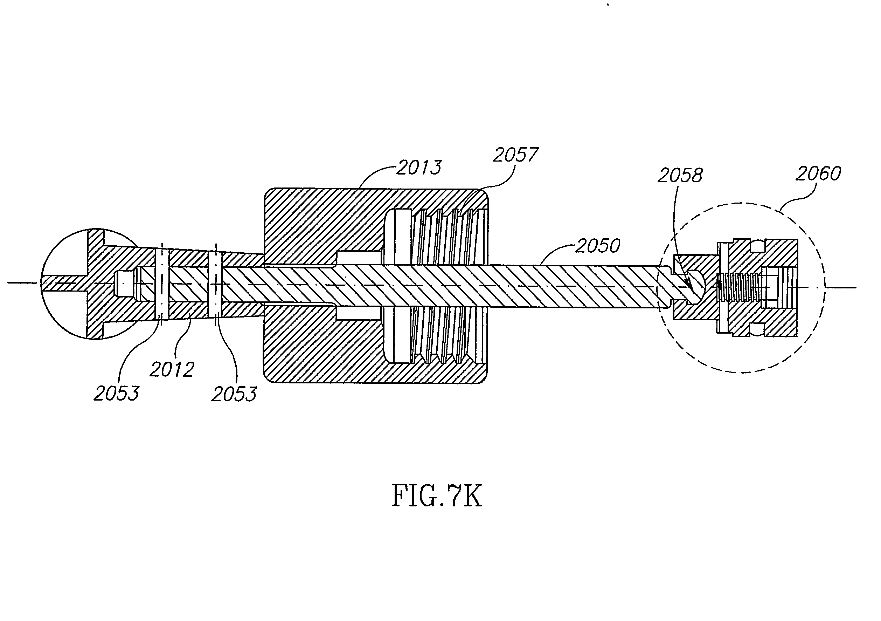

Fig. 7K is a cross sectional view of a handle and piston assembly as depicted in Fig. 7J. Fig. 7K shows a second set of threads 2057 on cap 2013. Threads 2057 are one exemplary means to engage cap 2013 to hydraulic pressure body 2005. Once cap 2013 is engaged to hydraulic pressure body 2005, operation of handle 2012 will cause advancement of piston 2060 in hydraulic pressure body 2005. Optionally, threads 2057 are in the same direction, as threads on shaft 2050 to prevent disassembly. Optionally, threads 2057 are locked once the cap 2013 is completely on.

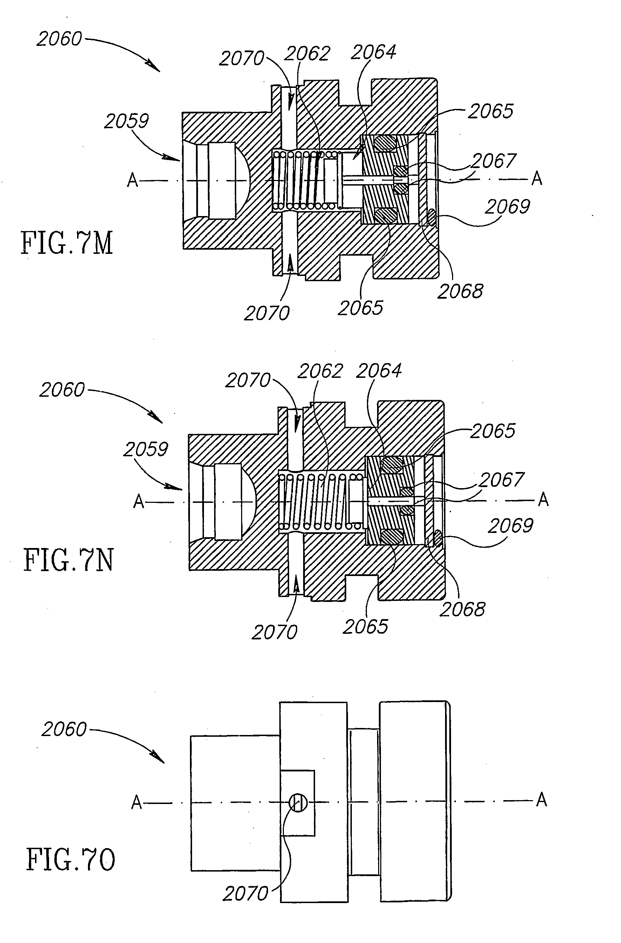

Fig. 7K also shows an exemplary engagement mechanism by which drive shaft 2050 may be coupled to piston 2060. The pictured engagement mechanism relies upon a ball 2058 which snaps into a matching socket (2059; Fig. 7M) in piston 2060. This arrangement may assure that piston 2060 remains attached to ball 2058 as shaft 2050 advances. Optionally, a ball/socket connection of this type permits piston 2060 to advance without rotating as bolt 2050 rotates. This lack of piston rotation may increase valve life and improves sealing. Optionally, the ball/socket type connection provides a sufficiently strong engagement force so that back turning of shaft 2050 will retract piston 2060. Optionally, a ball socket connection is economical yet reliable.

Operation of handle 2012 in an opposite direction may cause retreat of piston 2060 in hydraulic pressure body 2005. Optionally, operation in a reverse direction ceases injection of cement.

**Pressure source safety valve**

Fig. 7L is an exploded view of a piston 2060 including a pressure release valve which can relieve excess pressure of hydraulic fluid by venting hydraulic fluid through one or more release ports 2070 on piston 2060. The pressure relief valve may include a spring 2062, a pressure release aperture element 2064, and a sealing gasket 2067. In the figure, optional washers 2065, 2066, 2068 and 2069 are depicted. The pressure release valve may be triggered at a pressure of 160 atmospheres. Optionally, a threshold pressure of 160 atmospheres protects a system designed to withstand 200 atmospheres.

Figs. 7M and 7N are cross sectional views of the valve of Fig. 7L in an open and a closed operational state respectively. Fig. 7N shows spring 2062 in a fully extended configuration. As distal end 2058 of drive mechanism is forced into receptacle 2059, piston 2060 advances through hydraulic pressure body 2005. Advancement of piston 2060 causes an increase in pressure of a fluid residing in hydraulic pressure body 2005. Advancement of piston 2060 causes an increase in pressure of a fluid residing in hydraulic pressure body 2005. When the pressure inside the hydraulic pressure body 2005 reaches a predetermined threshold (e.g., 150, 160 or 210 atmospheres), the retraction of spring 2062 causes apertured element 2064 to protrude through seal 2067. Apertured element 2064 provides a channel of fluid communication between pressurized fluid 2020 (Fig. 7G) and lower pressure compartment (2028). This permits hydraulic fluid to flow to the rear chamber 2028 behind piston 2060 via release ports 2070. Release of hydraulic fluid causes the pressure to drop below the threshold, spring 2062 to expand and seal the valve. Released hydraulic fluid may be visible in transparent pressure body 2005.

Fig. 7O is a side view of piston 2060 showing release port 2070. Opening of the valve may reduce the hydraulic pressure to the threshold pressure.

Pressure at the defined threshold may indicate that the procedure should be stopped because the cement has solidified. If the physician wants to continue with the procedure, replacement of reservoir 2002 is indicated.

**Injection Reservoir**

Figs. 7P and 7Q are side cross-sectional views of an injection reservoir 2002. Reservoir 2002 may be constructed of, for example, amorphous nylon e.g. Durethan$^R$ (LANXESS, Leverkuzen, Germany), Grilamid$^R$ (EMS-Grivory, Reichenauerstrasse, Switzerland) or Topas$^R$ (Ticona GmbH, Kelsterbach, Germany). Materials for construction of reservoir 2002 are selected so that they are not corroded by the relevant cement. The amorphous nylon may be transparent. The thickness of walls 2003 of reservoir 2002 may be greater than 3 mm, optionally greater than 4 mm, optionally greater than 5 mm optionally greater than 6 mm or intermediate or greater values. Reservoir 2002 may be characterized by a wall thickness to internal diameter ratio of about 0.23, optionally 0.25, optionally, 0.27, optionally 0.29 (e.g., wall thickness of about 5 mm and ID of about 18 mm). This ratio provides sufficient strength to withstand pressures of 100 to 300 atmospheres.

Walls 2003 of reservoir 2002 may be transparent and marked with a scale indicating volume. Optionally, this permits an operator of the system to ascertain how much cement has been injected at any given moment. Optionally, ribs provided to add strength serve as a scale indicating volume.

Cement reservoir 2002 may be loadable with sufficient cement to treat at least one vertebra with a single injected aliquot. Optionally, 5 ml optionally 10 ml or intermediate or greater volumes are typically employed to treat a single vertebra. Optionally, cement reservoir 2002 is loadable with sufficient cement to treat at least two vertebrae, optionally at least 3, optionally at least 4 vertebrae without re-filling. Optionally, this reduces a number of access procedures for each vertebra, optionally to a single access procedure. A single access procedure may be employed to treat at least two locations in a vertebra.

Reservoir 2002 may be loaded with a cement characterized by a long "working window" during which the cement is characterized by a viscosity above 500 Pascal second but is not yet solidified. Optionally, the working window is greater than 5, optionally 8, optionally 12, optionally 15 minutes or intermediate or greater times.

Reservoir 2002 may serve also as a mixing chamber. Optionally, a polymer component and a monomer component of the cement are mixable in reservoir 2002. Alternatively, mixing is performed in a separate mixing apparatus and cement is transferred to the reservoir after mixing is complete.

**Reservoir assembly**

Fig. 7P illustrates assembly of an exemplary injection reservoir for injection of bone cement. While this reservoir is suited for use in a system of the general type depicted in Figs. 7F and 7G, the exemplary arrangement of Fig. 7P features a proximal reservoir cap 2100 as opposed to the distal reservoir cap of Figs 7F and 7G.

A reservoir cap 2100 includes a connector plug 2110 equipped with a tube connection 2310 to facilitate connection to tube 2006 which contains hydraulic fluid. Plug 2110 and tube connection 2310 form a contiguous lumen 2300 which facilitates delivery of hydraulic fluid from tube 2006. Plug 2110 optionally rotates within cap 2100 so that an angle of tube connection 2310 with respect to reservoir 2002 can be adjusted.

The angle of connection 2310 with respect to reservoir 2002 may be adjusted so that tube 2006 is out of the field of view of an X-ray imaging device. Rotation of plug 2110 may make connection of 2006 more convenient. Plug 2110 optionally includes a coupling portion 2115 which mates with a complementary coupling portion 2215 (Fig. 7Q) on delivery piston 2200. Optionally, piston 2200 is mounted on plug 2110 during assembly by snap coupling portions 2115 and 2215.

Cement reservoir 2600 is optionally filled with cement prior to closing of reservoir body 2003 with cap 2100. Cap 2100 can then be attached to body 2003 by means of, for example, mated threads on the two pieces. At this stage, both plug 2100 and piston 2200 are sealed to an inner side of walls 2003, for example by O-rings 2211. During operation, hydraulic actuator 2004 causes a fluid to flow through tube 2006 under pressure and enter lumen 2300. As the applied pressure increases, this fluid accumulates in portion 2700 of reservoir 2002 (Fig. 7Q). In an exemplary embodiment of the invention, plug 2100 and piston 2200 disengage when the fluid pressure developed in 2700 supplies enough force to advance piston 2200 against resistance supplied by cement in portion 2600 of reservoir 2002.

At the distal end of reservoir 2002, an optional inner plug 2400 engages an outer connector 2410 and holds outer connector 2410 to the distal end of the injection reservoir. Outer connector 2410 is constructed to engage an injection cannula and to remain engaged at a relevant operating pressure of the delivery system. Inner plug 2400 and outer connector 2410 form a channel of fluid communication 2500 which can facilitate a flow of cement from reservoir 2600 to an inner lumen of a cannula connected to connector 2410. The function of plug 2400 during filling of reservoir 2002 is described with regard to Figs. 7R and 7S, hereinbelow.

Fig. 7Q shows that fluid 2700 accumulates within 2005 and pushes piston 2200 away from plug 2110 and towards plug 2400. As the volume of fluid 2700 increases, the volume of cement 2600 decreases and the cement is pushed outwards through channel 2500 into a cannula (not shown in this figure). Piston 2200 may be a floating piston which is moved by a column of fluid 2700. Optionally, walls 2005 are transparent so that piston 2200 is visible to an operator of the unit, for example as an indicator of cement volume. Transparent walls 2003 may be marked with graduations which indicate an injected volume of cement.

Cement reservoir 2600 may be supplied as a separate unit comprising walls 2003, inner plug 2400 (Fig. 7R) and outer connector 2410. Figs. 7R and 7S illustrate one exemplary type. Plug 2400 contains an aperture 2500 which is initially covered by connector 2410 during filling of the reservoir with cement and subsequently opened to permit attachment of a cannula.

Fig. 7R illustrates outer connector 2410, inner plug 2400 and walls 2003 in cross section. In this figure, connector 2410 is only partially pressed onto a protruding portion of plug 2400. Seal 2508 remains unbroken. O-ring 2411 provides a tight seal between walls 2003 and plug 2400. This arrangement permits introduction of cement into the reservoir from a proximal end before cap 2100 (Fig. Q) is attached. Once the reservoir is filled, cap 2100 may be applied as described above. Optionally, this arrangement permits a more efficient seal to a cannula and/or provides the possibility to rotate the cannula with respect to the reservoir. The cannula may be deformed from a straight line and/or be directional.

Once the reservoir is filled and capped, seal 2508 may be broken (Fig. 7S) by advancing connector 2410 towards plug 2400. Breaking of seal 2508 creates channel of fluid communication 2500 which can facilitate a flow of cement outwards from the reservoir into a cannula (not shown in this figure) connected to connector 2410. Breaking of seal 2508 may expose a threaded and/or luer connection which is adapted to engage a cannula.

Fig. 7S1 depicts an exemplary reservoir 2600 in which walls 2003 are formed as a contiguous unit which includes some of the functional characteristics of outer connector 2410, inner plug 2400 in Fig. 7S. In Fig. 7S1, aperture 2500 is not sealed. Walls 2003 include threads 2509 and/or luer connector 2510 for attachment to a cannula.

**Foot operable actuator**

Fig. 7T is a perspective view of a foot operable hydraulic actuator 4004. A foot operable actuator may be used in place of a hand operable actuator. A foot operated actuator may permit an operator to employ both hands for other tasks and/or reduces the need for an assistant. In the figure, hydraulic reservoir 4005 is functionally similar to hydraulic reservoir 2005 as described hereinabove. Locking ring 4013 is functionally analogous to reservoir cap 2013 as described hereinabove. The pictured foot operable actuator contains a drive pedal 4100 which moves angularly with respect to an axle 4300. Each depression advances a hydraulic piston in hydraulic reservoir 4005 by a fixed increment. After pedal 4100 is depressed for actuation, it returns to a pre- actuation position automatically. This return may be achieved, for example, by use of a spring which provides a resistive force. Optionally, actuator 4004 includes an additional pedal 4200 for pressure release which moves angularly with respect to an axle 4300. Pedals 4100 and 4200 may be clearly marked so that an operator can distinguish between them easily. Markings may be, for example, in printed symbols and/or by pedal color and/or by pedal size and/or by pedal shape and/or by pedal position.

Walls of hydraulic pressure chamber 4005 may be transparent and marked with a scale. Optionally, the scale indicates indicating volume. Optionally, this permits an operator of the system to ascertain how much cement has been injected at any given moment. Optionally, ribs provided in the walls to add strength serve as a scale indicating volume. Pressure inducing levers may apply increments of hydraulic pressure using any clutch/drive mechanism known in the art to advance a hydraulic piston within hydraulic reservoir 4005. Examples of suitable drive mechanisms include, but are not limited to ratchet pawl mechanisms, sprag clutches, roller ramp clutches and mechanical diodes, cam followers and roller bearing cam followers. Sprag clutches are commercially available (e.g. from JTEKT Co.; Osaka/Nagoya; Japan). Mechanical diodes are available from, for example, Epilogics (Los Gatos; CA; USA). One of ordinary skill in the art of mechanical engineering will be able to select a suitable commercially available drive mechanism, or construct a suitable drive mechanism from commercially available parts in consideration of desired performance characteristics for any particular contemplated arrangement.

Release pedal 4200 may be provided to release some or all applied hydraulic force. Such a release may be desired, for example to change or replace cement reservoir 2004, at the end of a surgical procedure and/or in the event of an emergency. Optionally, release pedal 4200 may open a valve which vents hydraulic fluid from reservoir 4005. Alternatively or additionally, release pedal 4200 may act by permitting a threaded drive shaft to move backwards so that a hydraulic piston in reservoir 4005 is retracted.

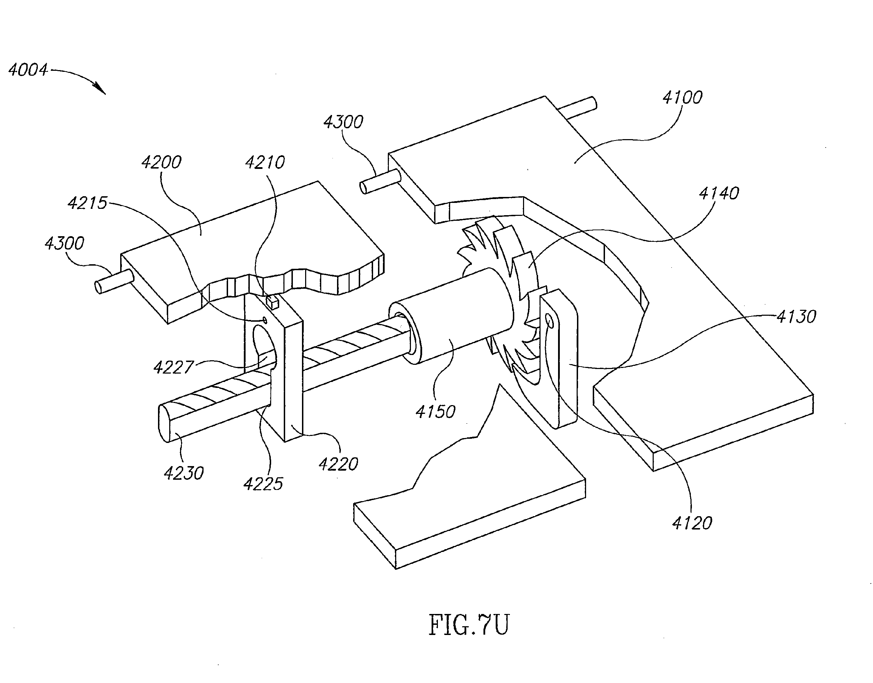

Fig. 7U illustrates a pedal operated drive mechanism with a companion release pedal. When drive pedal 4100 is depressed by a foot, it rotates with respect to axle 4300 and depresses drive arm 4130. Drive arm 4130 engages gear 4140 causes it to rotate angularly by a single increment. The increment may be varied by varying the number of teeth on gear 4140. Gear 4140 is mounted on a drive nut 4150 so that rotation of gear 4140 causes rotation of drive nut 4150. Drive nut 4150 is equipped with an inner threading mechanism (not visible in the figure) which can advance driveshaft 4230 without rotating a distal end thereof. As drive nut 4150 is rotated it operates the inner threading mechanism and drives drive shaft 4230 outwards through a narrow portion 4225 of a hole in clutch plate 4220. Drive arm 4130 and gear 4140 are depicted to generally indicate the presence of a ratcheted gear or functionally similar mechanism and their pictured shapes should not be viewed as a limit of the invention. When drive pedal 4100 is released, it is raised by a spring (not shown). Drive arm 4130 disengages from gear 4140. Drive arm axle 4120 permits drive arm 4130 to rotate slightly as it is raised and lowered so that it disengages and re-engages teeth of gear 4140.

A distal end of drive shaft 4230 pushes a piston (not shown in this view) in hydraulic pressure reservoir 4005 (Fig. 7T). Pressure in the reservoir tends to force drive shaft 4230 to return towards drive nut 4150. A narrow aperture 4225 in clutch plate 4220 prevents rotation of drive shaft 4230 while drive arm 4130 is disengaged from gear 4140 and prevents the shaft from retuning towards nut 4150. Clutch plate 4220 is optionally attached to pedal 4200 by rod 4210 and pin 4215.

When release lever 4200 is depressed, it lowers clutch plate 4220 so shaft 4230 passes through a wide portion 4227 of the hole. Shaft 4230 is then free to retract towards nut 4150 because it can rotate in wide portion 4227 of the hole. Optionally, shaft 4230 is constructed with sectorial threading (not pictured in this view) so that when clutch plate 4220 descends shaft 4230 rotates against a nut that defines a desired resistance. Optionally, operation of lever 4200 releases all of the pressure in the system or only a portion of it. Optionally, release may be sudden or gradual.

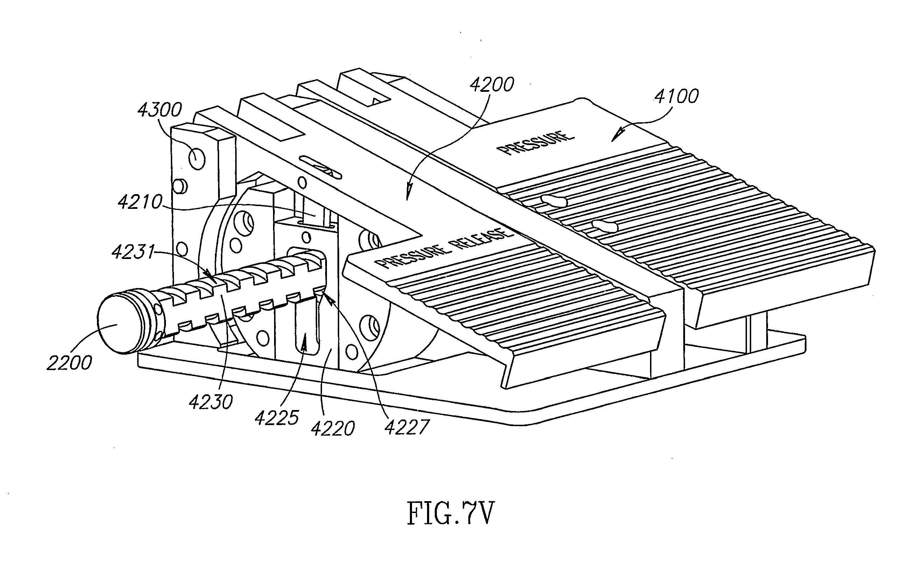

Fig. 7V illustrates a foot operable hydraulic actuator as seen in Fig. 7T with hydraulic reservoir 4005 removed. In this view, drive shaft 4230 is characterized by optional sectorial threading 4231. Shaft 4230 is pictured in wide portion 4227 of a hole in clutch plate 4220 so that it can retract. A distal end of shaft 4230 is fitted with a piston 2200. As shaft 4230 advances it drives piston 2200 into pressure reservoir 4005 (not pictured in this view) and increases the hydraulic pressure.

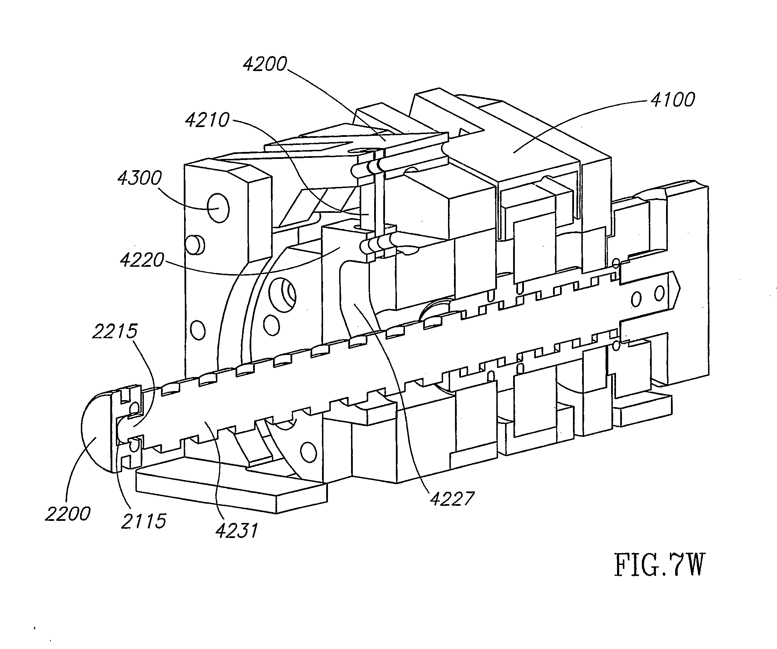

Fig. 7W is a cross sectional view of the exemplary actuator depicted in Fig. 7V. In this view release lever 4200 is raised so that wide portion 4227 of the hole in clutch plate 4220 is raised off of sectorial threads 4231 on shaft 4230. This causes the clutch plate to engage the drive shaft and prevent retraction of the drive shaft.

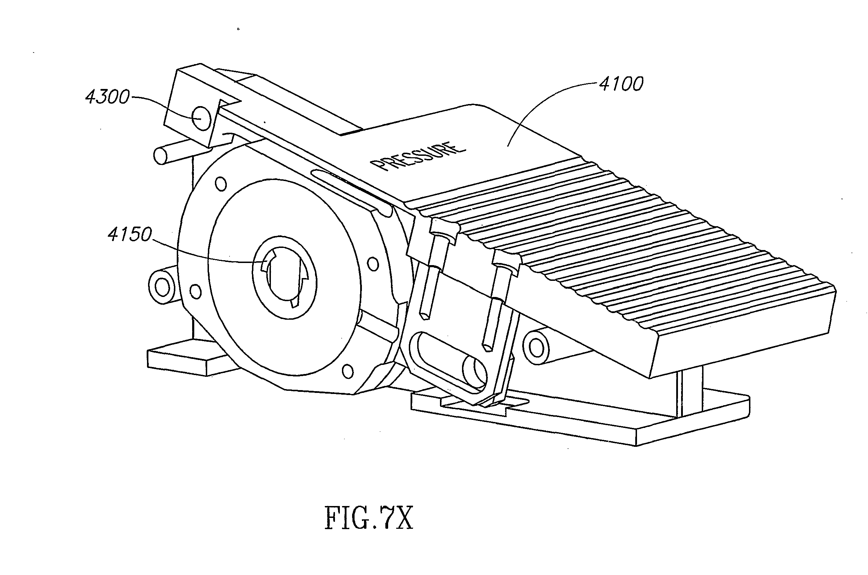

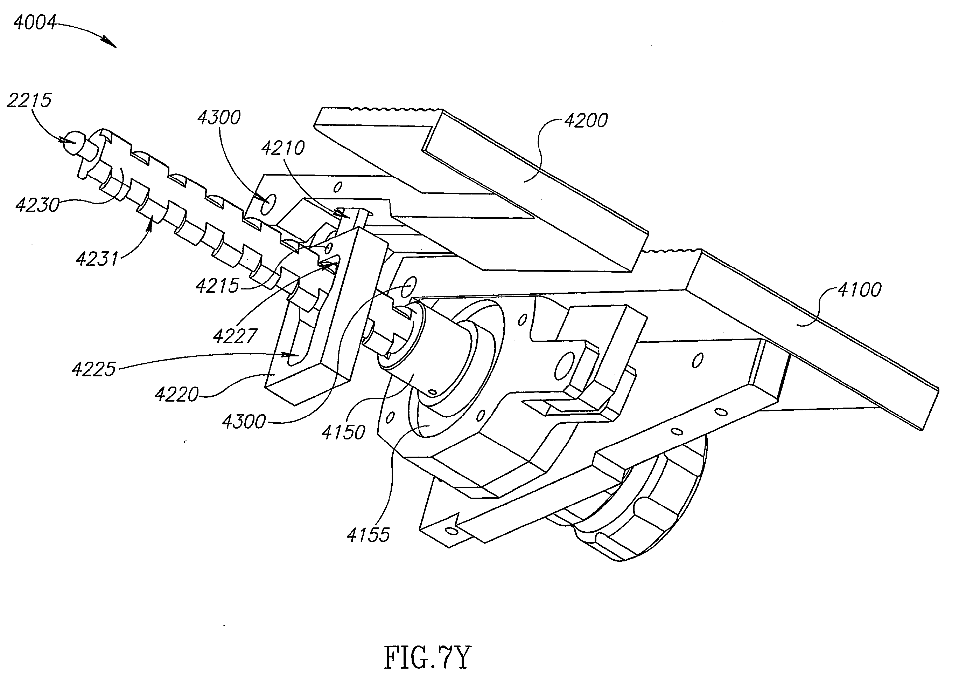

Fig. 7X illustrates an exemplary drive nut 4150 adapted to engage and rotate sectorial threads such as those illustrated in Figs. 7V and 7W. Fig. 7Y is a perspective view of the exemplary foot activated actuator depicted in Figs. 7V, 7W and 7X from below. This view illustrates clearly a housing 4155 of the inner threading mechanism which advances shaft 4230 by engaging and turning sectorial threads 4231.

**Additional exemplary hydraulic mechanism**

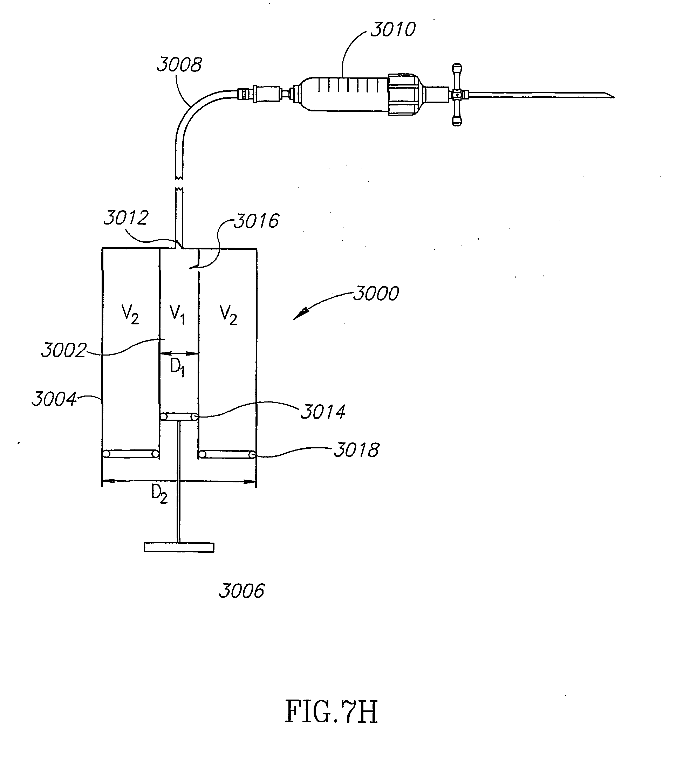

Fig. 7H illustrates a hydraulic delivery system. A hydraulic pump 3000 includes a smaller syringe 3002 within a larger syringe 3004. Smaller syringe 3002 is characterized by a first volume (V1) volume and a first diameter (D1), and larger syringe 3004 is characterized by a second volume (V2) volume and a second diameter (D2); where $V_1 << V_2$ and $D_1 << D_2$. Each activation of an actuator 3006 (e.g., handle rotation or foot pedal pressing) may result in injection of defined amount of liquid from smaller syringe 3002 into reservoir 3010, optionally via flexible tube 3008. A uni-directional valve 3012, located at the distal end of the small syringe 3002 may assure that liquid flows only towards reservoir 3010. When activation of the actuator ceases, piston 3014 of the small syringe 3002 automatically returns to its original position. The automatic return of piston 3014 may be achieved, for example by use of a spring or an elastic band which applies force in a direction opposite to a direction of actuation. A second uni-directional valve 3016 is located in the wall between smaller syringe 3002 and larger syringe 3004. When piston 3014 returns to its original position, a vacuum is created inside syringe 3002. The vacuum and/or a force and/or a spring that presses piston 3018 opens valve 3016 and liquid from larger syringe 3004 flows into the barrel of the small syringe 3002. Liquid in larger syringe 3004 may serve as a reservoir for the refilling of the small syringe 3002. A piston 3018 of larger syringe 3004 may advance as V2 decreases. Optionally, this can provide force amplification (if D1 is greater than an ID of 3010; see detailed explanation below) and/or facilitates delivery of small and/or defined aliquots of liquid upon each activation of actuator 3006.

In many cases, the system is designed to assure that the hands of an operator is outside an X-ray radiation zone of an imaging or monitoring system employed in conjunction with the cement delivery system.

**Pressure Amplification**

Referring again to Fig. 7G, piston 2024 of pressure source 2004 is characterized by a first diameter (D1) and piston 2018 of cement reservoir 2002 is characterized by a second diameter D2.

If hydraulic fluid is present in 2026 and in 2028, the two chambers function as a single chamber and no hydraulic amplification is achieved even if D1 and D2 are different.

Piston 2018 may be pushed by a drive shaft (not shown) instead of by hydraulic fluid in 2028. Accordingly hydraulic amplification may be calculated as follows:

The applied force (F) supplied by each piston can be calculated from relevant pressures (P) and diameters (D):

$$F = P*A; \text{ where}$$

$$A = \Pi*D^2/4 \text{ thus}$$

$$F = P* \Pi *D^2/4$$

If pressure amplification is defined as pressure in cement reservoir 2002 (P2) divided by pressure in pressure source 20042004 (P1), the pressure amplification is equal to $(D1/D2)^2$.

Pressure source 2002 may be designed to be held in one hand, while a second hand operates handle 2012. This design is compatible with an internal diameter D1 of 5 cm. A typical cement reservoir has in internal diameter of 1.8 cm. This exemplary configuration produces a pressure amplification of 7.72.

For foot operations, D1 may be considerably larger (e.g. 10 cm, 15 cm, 20 cm or intermediate or larger sizes) and a greater pressure amplification may be achieved.

Alternatively or additionally, mechanical amplification applies as in any manual device that uses a rotational drive with a lever arm (e.g. handle 2012 and cap 2013 in Fig. 7G or lever 4100 and gear 4140 in Fig 7U). These mechanical amplifiers further reduce the amount of input force required to achieve a large force to drive the piston in the cement reservoir (e.g. 2002 or 4005).

**Unit material provision system**

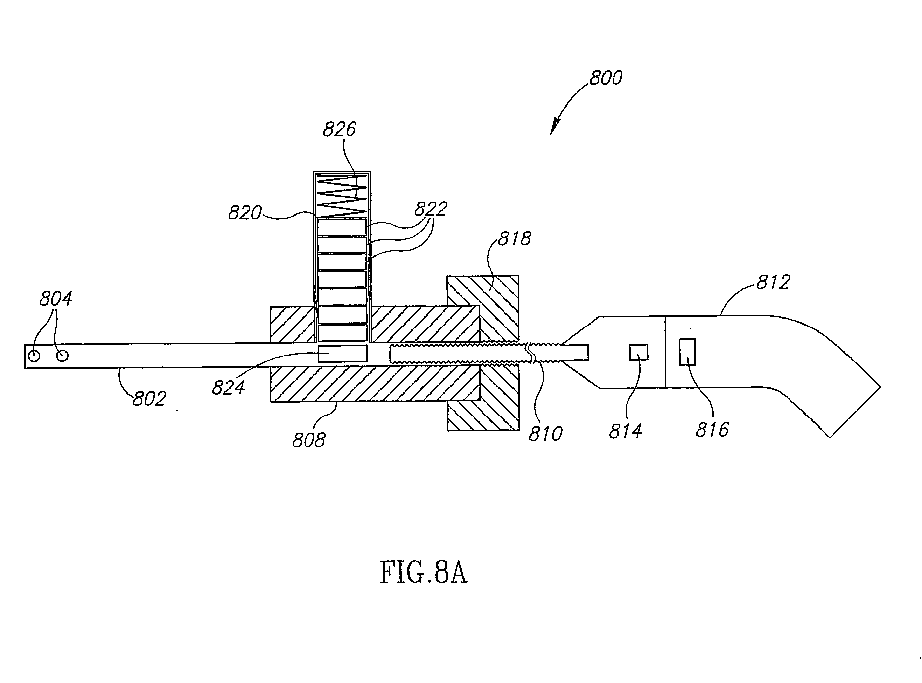

Fig. 8A shows a delivery system 800 in which material is provided as discrete units, each of which is of relatively small volume, for example, 1/2, 1/4, 1/7, 1/0 or less of the amount required for treatment. One potential advantage of working in units is that an operator is more aware of the effect of his/her actions as each action can only inject one unit. Another potential advantage of working in units is that units with different material properties may be provided during a procedure. Another potential advantage is that units being small will generally exhibit a smaller friction with the delivery system.

System 800 comprises a delivery tube 802 having one or more extrusion apertures 804 at its tip. A barrel 808 on which tube 802 is mounted, also includes an optional magazine 820, described below. A body 818 with an optional nut threading is optionally attached to barrel 808. A pusher 810 lies within delivery tube 802 and/or barrel 808.

A handle 812 may be provided which includes a battery powered mechanism for advancing pusher 810. A hydraulic mechanism such as described above may be used instead. Optionally, one or more switches are provided, for example, an on/off switch 816 and a direction switch 814. Optionally, when pusher 810 completes its forward motion, it is automatically retracted. Optionally, only a single switch is needed, activation of which causes extrusion of one unit. Handle 812 may be rotationally locked to body 818, for example using one or more guide pins.

Handle 812 may comprise a motor and a battery that rotate pusher 810. An alternative mechanism is described below.

Referring to magazine 820, the magazine may comprise discrete units 822 of material (a unit 824 is shown inside tube 802). Optionally, a spring 826 is used to push the units towards tube 802. Optionally, the magazine is filled with a contiguous mass of material and the units are defined by the cutting action caused by pusher 810 pushing a unit of material away from the magazine.

A magazine may be prepared ahead of time, for example, by a manufacturer, who fills the magazine with a non-setting material. The magazine may be loaded with a series of units of different properties, for example, responsive to an expected progress of a procedure, for example, first providing a soft material and then providing a harder material, or vice versa. Alternatively, a rotating magazine is used, in which a user can select which of several compartments will load barrel 808 next. This allows fine control over the injected material. An operator may remove magazine 820 at any time and replace it with a different magazine. Optionally, this is done while pusher 810 is forward, so that there is no danger of backflow from the body.

Optionally, one or more of the units comprises or is an implant device (rather than an amorphous and/or homogenous mass), for example, an expanding implant or an implant whose geometry does not change. Optionally, one or more of the units comprises a cross- linked material.

The delivery system may comprise two or more delivery tubes (optionally the combined geometry has a cross-section of a circle or of a figure eight). Optionally, each tube has a separate pusher mechanism and/or a separate material source (e.g., a magazine). Optionally, the two tubes are used simultaneously. Optionally, an operator can selectively use one tube. Optionally, the materials provided in each tube are components that react chemically one with another. Optionally, electronic control is provided to control the relative provision rates of the two tubes. Optionally, this allows control over the final material properties. Optionally, the use of two or more tubes allows a layered structure to be built up in the body. Optionally, one of the tubes delivers a setting material and the other tube delivers a non-setting material. In an alternative embodiment, each tube is used to provide a different component of a two component material. Optionally, the two tubes meet at their distal end, to ensure mixing of the components.

The delivered material may be CORTOSS by Orthovita Inc. (US) a composite of Bis-GMA, Bis-EMA and TEGDMA. This material is optionally mixed along the path in the delivery tube.

Instead of the units being provided by a magazine or by a cutting mechanism, a partial unit behaviour may be provided by the motor of handle 812 stopping after every "unit" advance. Optionally, mechanical stops are provided for a hydraulic mechanism, if used. Optionally, instead of stopping, a sound is provided when a unit is injected or based on a different logic, for example, when 50% or another percentage of planned amount of material is provided. Optionally, a CPU is provided which analyses an image provided by an imaging system and generates a signal when a sufficient and/or near sufficient and/or over-load amount of material is provided. Other circuitry may be used as well.

Optionally, circuitry is provided for controlling the rate and/or pressure of material provision. Optionally, the circuitry stops advancing if a sudden change in resistance is perceived.

The delivery system may include pre-heating or pre-cooling of the injected material and/or of tube 802. A Peltier cooler and/or a resistance heater may be provided in barrel 808. Other cooling or heating methods, such as based on chemical reactions or phase changing materials, may be used.

The magazine may be a long coiled magazine. Alternatively or additionally, the deformable material is folded in the magazine. Optionally, the magazine is elongated. Optionally, separate loading and pushing mechanism are provided. For loading, a unit may be inserted through a slot in the side of the barrel. For pushing, the unit is advanced under a low pressure past the slot (or the slot is sealed) and only then is significant pressure required to advance the unit, for example, once the leading edge of the unit reaches the extrusion apertures.

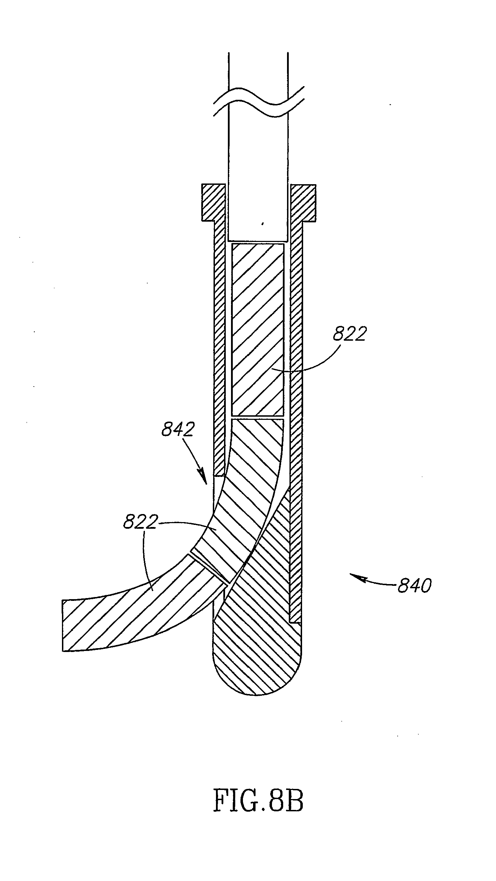

Fig. 8B shows the implementation of a unit delivery method even without a cassette. A delivery tip 840 of the cannula is shown with a lateral aperture 842 through which multiple units 822 are shown exiting. Optionally, an indication is provided to the user as a unit exits, for example, based on motion of a pusher used. Optionally, the system of Fig. 8A is used to load a series of units 822 into the barrel, for example, pulling back the pusher after each unit is advanced past the cassette. A tip of the cannula may be closed, optionally permanently closed, so that cement 822 is forced laterally outwards via aperture 842.

**Battery powered pusher**

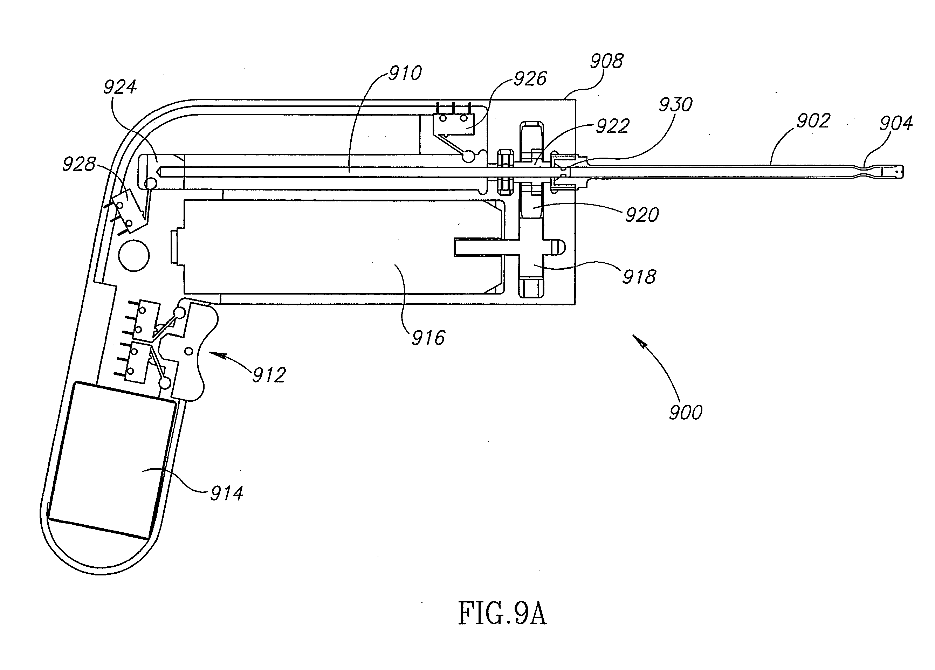

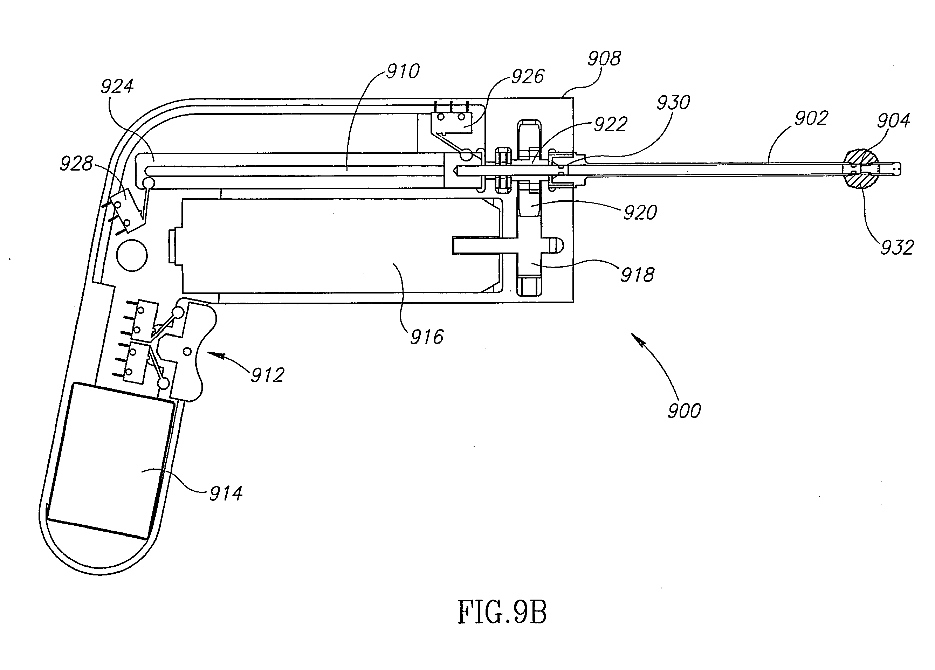

Figs. 9A and 9B show a material pusher 900 with reduced material twisting.

As in the delivery systems described above, pusher 900 comprises a delivery tube 902 having one or more apertures 904 near its end. Optionally, an offset is provided between the apertures and the far tip of tube 902, for example, to ensure centering (or other positioning) of the extruded material, for example preventing the material from being provided too close to a far end of the vertebra, if the delivery system is pushed forward. Tube 902 is mounted (e.g., optionally replaceably) to a body 908. A pusher 910 is used to advance material through tube 902.

In use, an operator may press a switch 912, for example, to select between forward, backwards and no motion of pusher 910. Power from a battery 914 (or a hydraulic or other source) is conveyed to a motor 916. Rotation of the motor causes a nut 922 to rotate relative to pusher 910. Optionally, a series of gears are used which may or may not provide a mechanical advantage, depending on the implementation. Motor 916 may rotate a gear 918 that rotates a gear 920, which rotates nut 922 which is coaxial thereto. Optionally, a rotation preventing element 924, for example, a rectangular element 924 is mounted on pusher 910 and prevents rotation thereof.

Optionally, one or more sensors are used to detect the extremes of positions of pusher 910, when it is advanced and when it is retracted. In the example shown, a micro-switch 926 and a micro-switch 928 detect the ends of motion of pusher 910, for example, using a bump or electrically conducting section 930 (depending on the sensor type used).

Alternatively or additionally, a positional encoder is used, for example, by counting rotation, or a separate encoder as known in the art of encoders.

Fig. 9B shows system 900 after extrusion is effected, showing extrusions 932. Optionally, extrusions 932 are an extension to tube 902, prior to them being cut off by pusher 910. Rotation of tube 902 may cause extrusions 932 to act as a reamer. The viscosity and shear strength of the material may be selected to effect a desired limitation on the reaming abilities, for example, to prevent damage to bone.

Optionally, one or more gears are provided to rotate and/or oscillate the delivery tube as the material is advanced. Optionally, periodic or ramp axial motion is provided, by motor means. Optionally, the distal tip of the delivery tube is made soft, for example by attaching a soft tip thereto, to reduce or prevent damage to the vertebra. Sleeve provision system

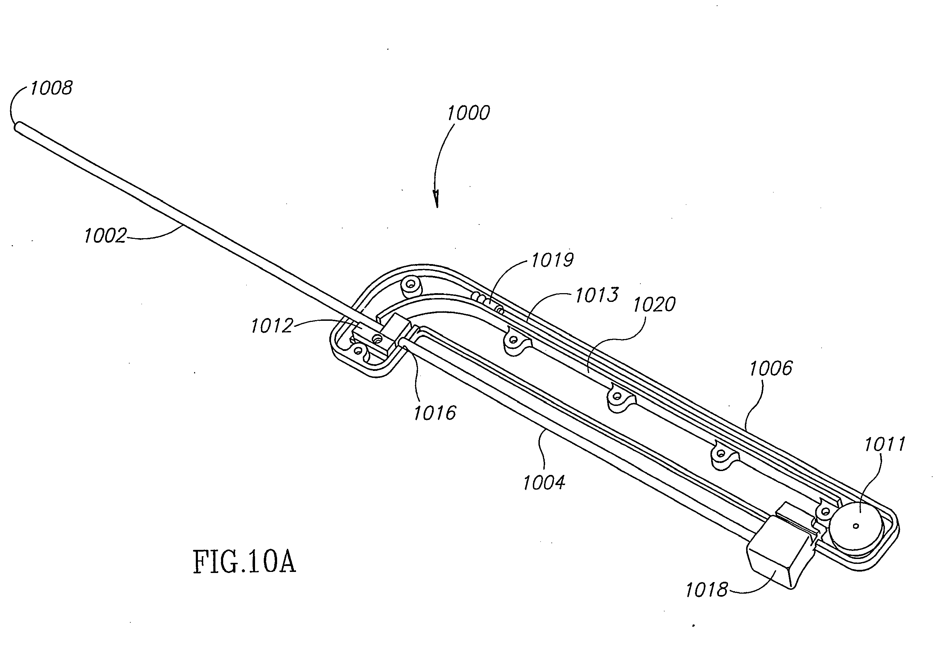

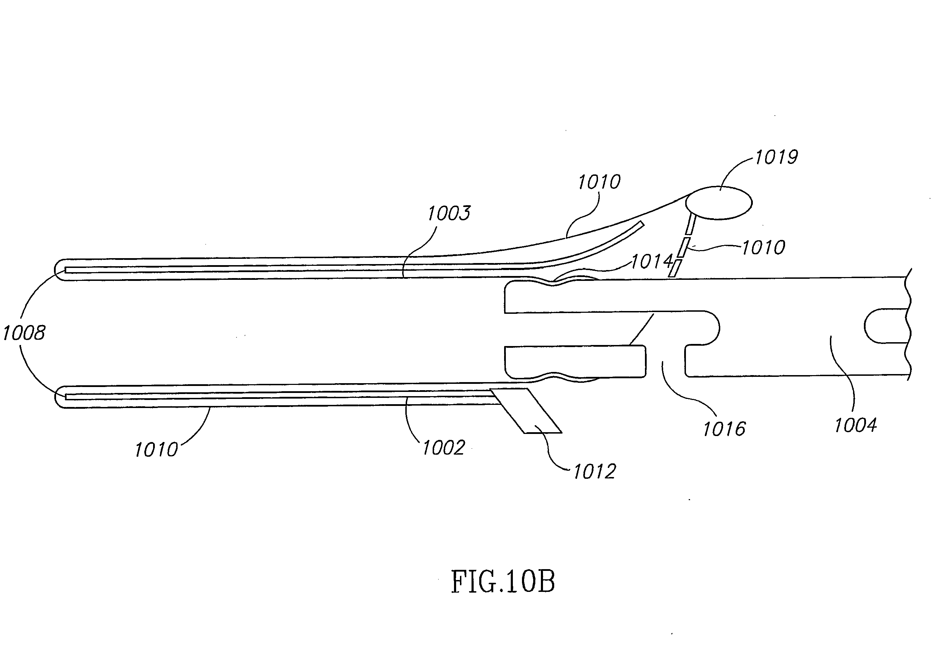

Figs. 10A and 10B shows a sleeve based delivery system 1000. Fig. 10A is a general cut-open view of system 1000, in which a sleeve 1010 is not shown. Fig. 10B shows the distal portion of system 1000, including sleeve 1010 mounted thereon. Figs. 10A-10B also illustrate a refilling mechanism by which the delivery tube includes a port to which a refill system can be connected to refill the delivery tube with material to be injected into the body.

A pusher 1004 pushes material that is found inside a delivery tube 1002. In the embodiment shown, the material is ejected past a tip 1008 of delivery tube 1002. A sleeve 1010 is provided so that the sleeve lies between the material and delivery tube 1002. An optional tube cutter 1012, such as a knife is shown to optionally split the tube after it exits the body. A pulley system 1011 for collecting the split tube is also shown.

In operation, an amount of material is either provided in tube 1002 or is injected into it, for example, via a port 1016 in pusher 1004. Advancing of pusher 1004, for example, by applying force to a knob 1018 attached thereto, for example manually, using a motor or using other mechanisms described herein, pushes against the material in tube 1002. At the same time, sleeve 1010, which is attached to pusher 1004, for example, by a crimping 1014, is pulled along with the material. Portions of sleeve 1010 reaching distal tip 1008 of tube 1002, fold back towards a body 1006 of delivery system 1000. When sleeve 1010 reaches knife 1012, it is optionally split so that it can pass over tube 1002 and pusher 1004. A thread or wire or other coupling 1013 is attached to the proximal (split) side of sleeve 1010 (e.g., via a connector 1019) and via a pulley 1011 is pulled as pusher 1004 advances. A slide 1020 is optionally provided to guide the motion of the split sleeve It should be appreciated that such a sleeve system can also be used for delivering implants rather than material. In one example, a compressed plastic implant, for example, polyurethane, which is compressed radially (and extended axially) is advanced using a sleeve system, to reduce friction. Optionally, the sleeve material is selected according to the material being used and/or the tube material. In another example, the sleeve system is used to deliver a self-expanding implant, for example, as described in WO 00/44319 or in WO 2004/110300.

It is noted that a sleeve system may also be flexible. Optionally, the sleeve is formed of a chain-link or a knitted material, rather than an extruded plastic polymer tube. Optionally, the sleeve is formed of multiple layers of materials, for example by extrusion or by lamination. Optionally, fibers or other strengthening means are provided to reduce elongation. Optionally, the sleeve is formed of a material that withstands heat and/or chemical by-products caused by PMMA. Optionally, the sleeve is preformed to elastically expand when it exits the delivery tube. Optionally, the sleeve is perforated or includes a plurality of apertures therein. Optionally, the sleeve elutes one or more treatment materials. Optionally, the sleeve elutes one or more catalysts or catalysis retarding materials, for example, to prevent or slowdown reactions in the delivery system and/or speed them up out of the delivery system.

Optionally, a layer of oil or other lubricant is provided in addition to or instead of the sleeve.

Optionally, the sleeve remains inside the body, for example, being formed of a bio-degrading materials or maintaining its form. Optionally, when degrading, strengthening fibers or other elements remain to enhance the strength of the extruded material or implant.

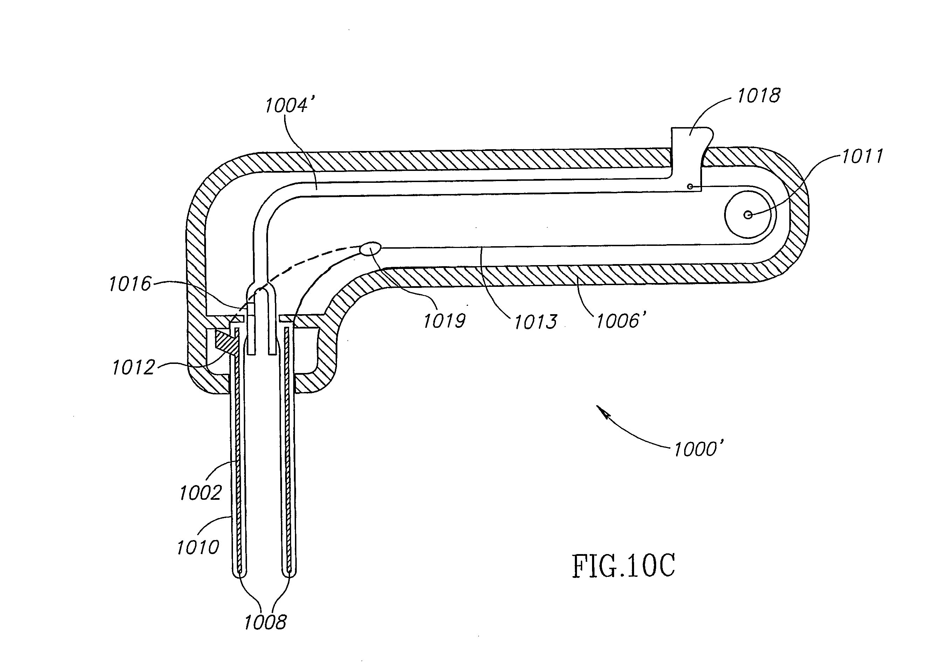

Fig. 10C is a cross-sectional view of a variant system 1000' in which a pusher 1004' is flexible enough to bend. This allows a body 1006' of the device to be manufactured in a nonlinear shape, for example, in the shape of revolver, which may be easier to hold. Optionally, one or more wheels, bearings or slides (not shown) are used to guide pusher 1004'. Optionally, pusher 1004' can be made more flexible as some of the motive force used to move the material is provided by the sleeve pulling the material forward. Alternatively or additionally, some reduction is supported by the reduced friction.

Optionally, a sleeve system is used with a magazine system, for example, the units being provided through port 1016.

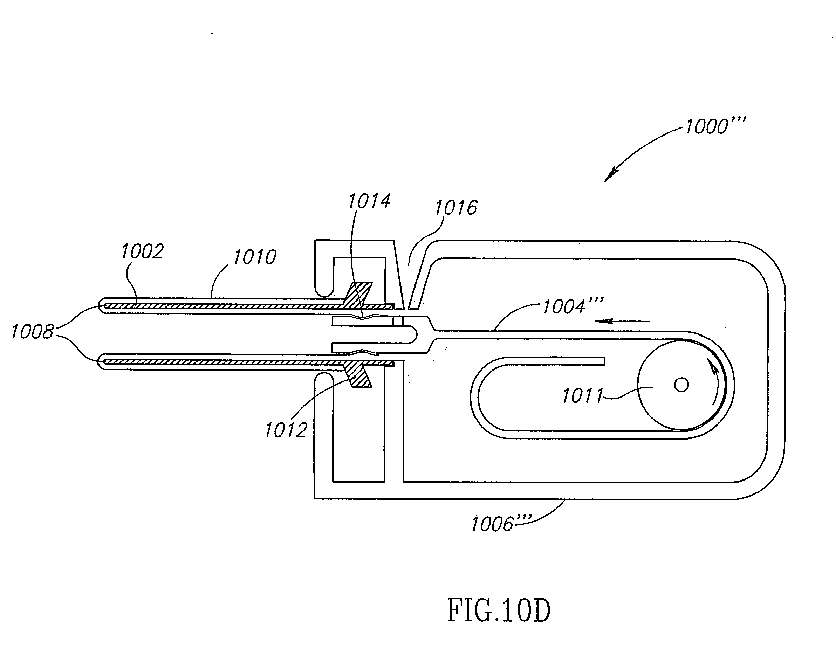

Optionally, the sleeve is pre-split and includes an overlap to prevent friction in the delivery tube. Optionally, this allows a magazine to load the sleeve from the side. Fig. 10D shows a further, compact, variant 1000" in which a pusher 1004" is made flexible enough to fold over itself, so body 1006" can be of smaller dimensions. It should be noted that these more compact and/or non-linear embodiments can also be practiced without the sleeve feature. The sleeve pullback mechanism is not shown here.

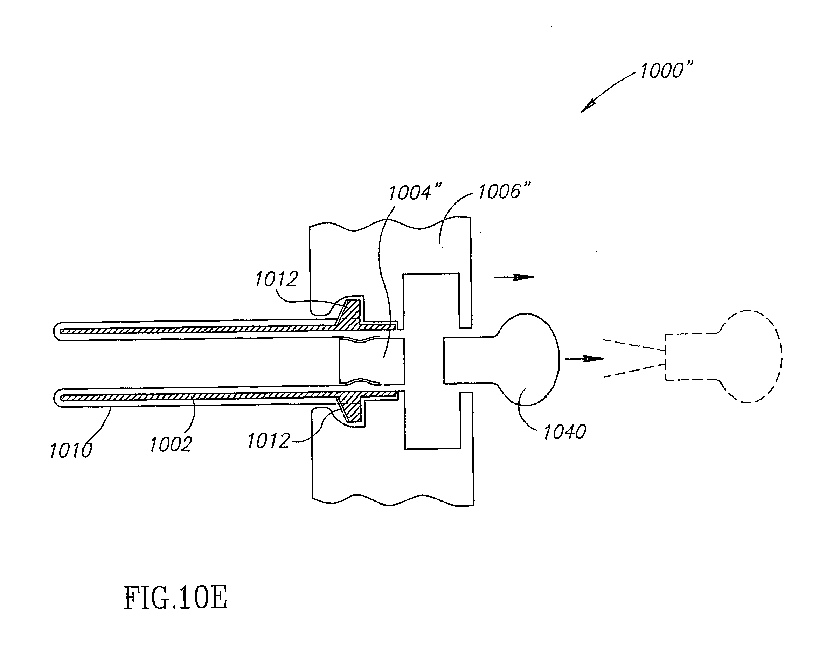

Fig. 10E shows a variant system 1000''' in which a pusher 1004''' is reduced in size axially. In this design the motive force is provided by pulling back the cut sleeve 1010 using a knob 1040 (or a motorized or mechanical gain or other means). This pulling back advances a shortened pusher 1004'''. Optionally, pusher 1004''' is provided as a sealed end of sleeve 1010. A body 1006'" of the system can be very compact, depending on the method of pulling back on knob 1040. Two or more symmetrically positioned knifes 1012 are provided, to allow for proper mechanical support of tube 1002 by body 1006'". Optionally, the tube is precut.

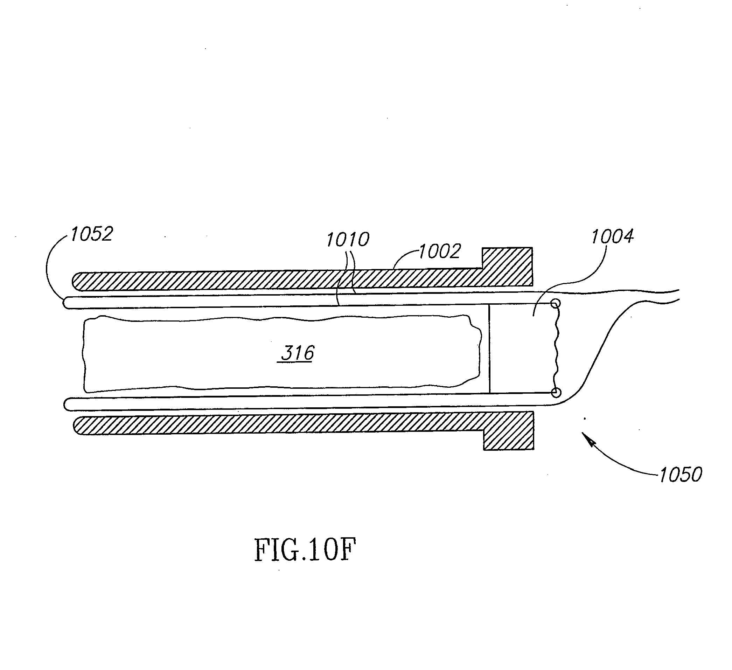

It is noted that pusher 1004 may be separated from the injected material by the sleeve. Optionally, a hydraulic system is used to advance the pusher, for example (in Fig. 10F) attaching a flexible tube to pusher 1004'" in tube 1002.

Sleeve 1010 may be used to isolate the body itself from the hydraulic system, possibly allowing for a system with a higher probability of leaking.

The material exited from the distal end 1008 of tube 1002. Optionally, a stop is provided at the end, so that the material is forced sideways. Optionally, the stop is not attached to tube 1002 at end 1008 thereof. Rather a thread, running through tube 1002 and/or outside thereof (or more than one thread) attaches the stop to the body of device 1000. Optionally, the thread runs through a narrow lumen formed in pusher 1004.

Alternatively, one or more elements which attach the stop to tube 1002, serve to split sleeve 1010, at tip 1008 of tube 1002. The stop may be attached to tube 1002 after the sleeve is mounted thereon. Alternatively, the sleeve is pre- split, pulled through tube 1002, past the elements and attached to connector 1019.

In an alternative arrangement, the sleeve is provided totally within the delivery tube. In one arrangement (not shown), the delivery tube comprises two coaxial tubes and the inner tube serves as shown by tube 1002 in Figs. 10A-10E.

In another arrangement, the fact that the delivery tube is full of material is taken advantage of, in that the material (316) serves to prevent the tube from collapsing when it is simultaneously pushed from one end and pulled from the other. This may depend on the viscosity of the material and/or on the shape of the distal tip of the delivery system. Optionally, the distal end is slightly flared to define a folding over location for the sleeve.

Fig. 10F shows a delivery system 1050, in which sleeve 1010 is provided within delivery tube 1002. As can be seen a folding over location 1052 for the sleeve is provided past the end of tube 1002. A ring (not shown) may be provided past the end of tube 1002 and around which the sleeve is folded. This ring serves as a scaffold for the folding, but due to its having a diameter greater than an inner diameter of tube 1002 (or at least being misaligned if the ring and/or tube are not circular in cross-section), cannot be pulled into the tube by retraction of sleeve 1010.

Sleeve 1010 may not fold back towards system 1000. Rather, the sleeve is pushed into the vertebra with the material. Optionally, once out of the confines of tube 1002, the material can tear the tube. In an alternative embodiment, the sleeve remains intact and encloses the material, sausage-like, in the body. The sleeve may be formed of biocompatible, bioabsorbable and/or implant grade material.

### Squeeze based material provision

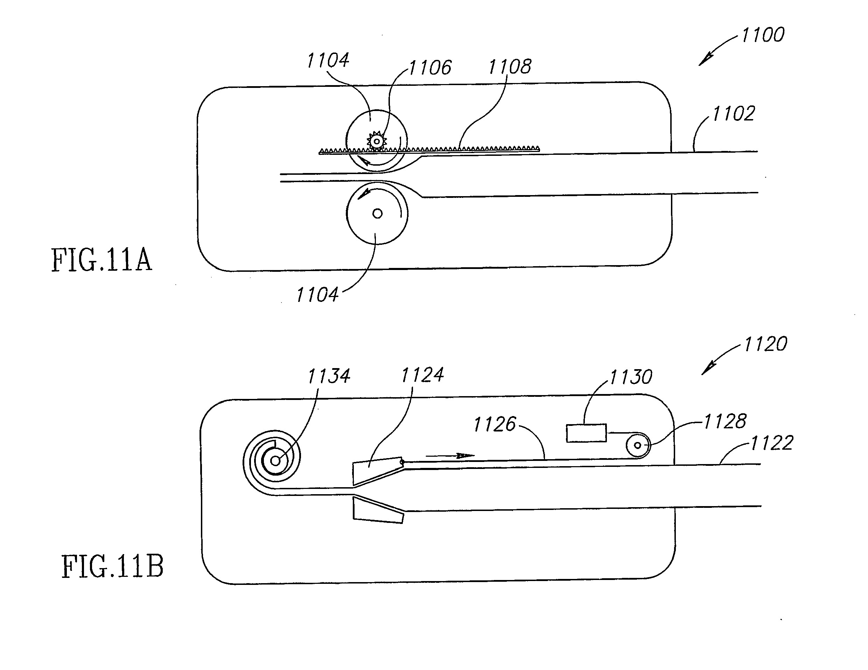

The material may be squeezed out of the delivery system rather than pushed. Fig. 11A shows a squeeze based system 1100, in which a delivery tube 1102 is made out of a squeezable material, such as a polymer or annealed metal.

A pair of rollers 1104 (or one roller and an opposing anvil, not shown) advance towards the distal side of tube 1102, squeezing it flat and forcing material that fills the tube to migrate distally. Various motion mechanism can be used. In the figure, the motion mechanism is a linear gear 1108 which engages a gear 1106 that is coaxial with roller 1104. When the roller is rotated, the linear gear advances the roller. Various power sources may be used, for example, electric motors and hydraulic power. Also, other power trains may be used. The rollers are optionally made of stainless steel.

Fig. 11B shows a delivery system 1120, in which a squeeze element 1124 slides rather than rolls against a delivery tube 1122. Tube 1122 is optionally rolled around a pin 1134. Various mechanisms can be used to move squeeze element 1124, for example a motor 1130 attached to a cable 1126 via an optional pulley 1128.

### Tamping method

Friction may be reduced by reducing the length of motion of the material inside a delivery tube. In one method, a small amount of material is provided into a distal side of a delivery tube (while outside the body). Then the distal part is inserted into the body and a tamping tool is provided into the proximal part. This process may be repeated several times until a desired amount of material is provided into the body.

### Penetrating delivery system

The delivery system may also penetrate to the bone and/or penetrate the bone. Optionally, this obviates the need for a separate cannula and/or may simplify the procedure. Optionally, the delivery tube is kept in the body when it is being refilled with material to be injected.

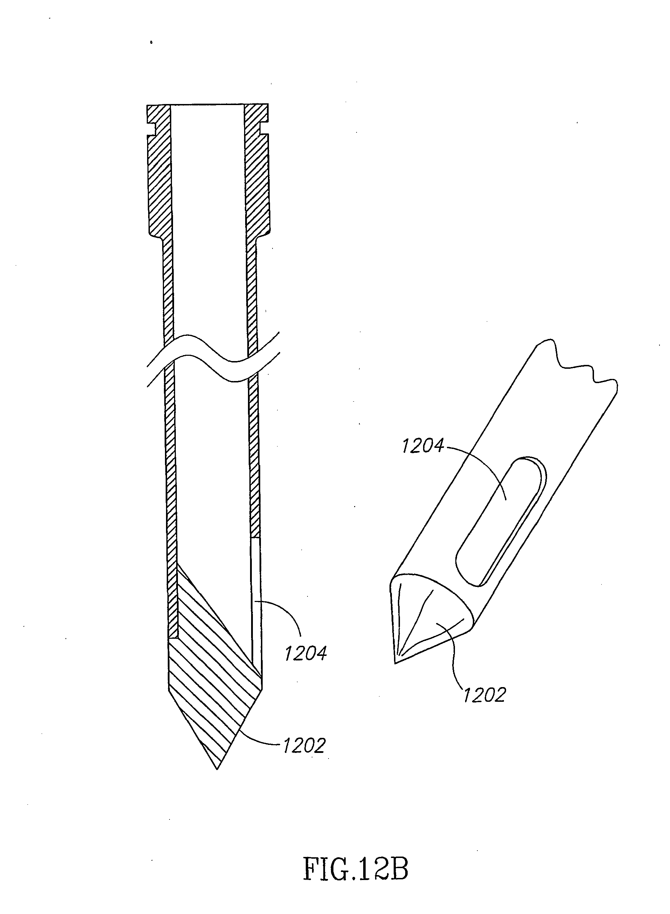

Fig. 12A shows a penetrating delivery system 1200. A distal tip 1202 is formed in a manner suitable for drilling in bone. This is shown in greater detail in Fig. 12B which a bone cement cannula with a lateral ejection aperture 1204 and a permanently closed distal tip 1202.

A hydraulic pump or mechanical ratchet advance mechanism is optionally used, with a handle 1206 used for pumping shown.

A potential advantage of a one piece system is that fewer parts are needed. If the system is preloaded with all the material needed, for example, at a manufacture, no equipment changes are needed. Optionally, the use of a side aperture 1204 allows the tip to be a drilling tip. Optionally, the use of smaller diameter tubes allows fewer parts to be used, as drilling is simplified.

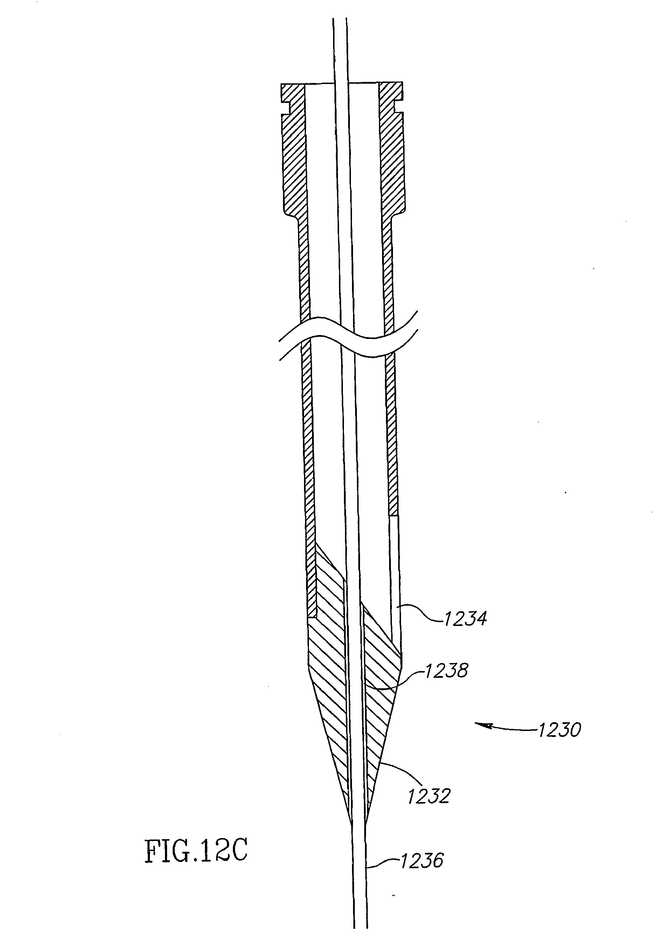

Optionally, the proximal end of system 1200 is adapted for tapping with a mallet. Fig. 12C shows an alternative system, 1230 adapted to ride on a guidewire 1236, for example, a K-wire. A bore 1238 may be formed in a drilling section 1232 of system 1230. Alternatively, the bore is to the side of the drilling head, for example, exiting through an aperture 1234 which may also be used for extruding material. Optionally, the pusher (not shown) is drilled as well. Optionally, the diameters of the drilled holes are too small for the material to exit through. Alternatively, bore 1238 is used for extruding material, after the K-wire is removed.

The material may be predrilled with a bore, to allow passage of the guidewire therethrough. Optionally, this bore is provided with a sleeve. It is noted that absent axial pressure on the material, the material will generally not flow into the drilled bore. Alternatively or additionally, the guidewire is coated with a suitable friction reducing coating, solid or fluid.

Optionally, the delivery tube is loaded after the delivery tube is guided into the body (and the guidewire removed), for example using a barrel storage means or a unit magazine as described above.

Optionally, a separate lumen is defined for a K-wire. Optionally, that lumen is a collapsible lumen. However, until pressure is applied to the material to be delivered, it remains un-collapsed. Once the guidewire completed its task, it is removed and pressure applied to the material, collapsing the guidewire channel and improving the flow characteristics (by increasing effective inner diameter of the delivery tube.

A cannula may not be needed, for example, if the delivery system rides on the guidewire or if the delivery system is used to directly penetrate the bone. Optionally, the delivery tube of the delivery system is not removed once inserted into or to the bone, for example, using a barrel or pumping mechanism as described above to reload the delivery mechanism if required. Once the system is reloaded, the pusher can advance the material into the delivery tube where it can then be advanced into the bone.

**Mixing Apparatus**

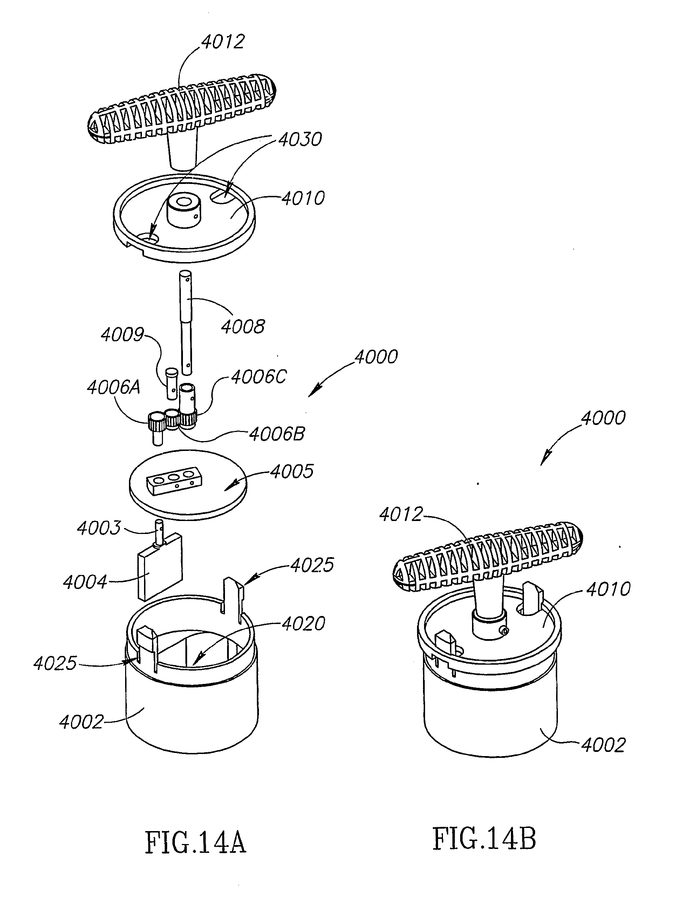

Figs. 14A-14B illustrate an exemplary high shearing force mixing apparatus 4000 adapted for mixing a viscous mixture. Fig. 14A is an exploded view of apparatus 4000 and Fig. 14B is a perspective view of the same apparatus after assembly. Apparatus 4000 may be used for mixing the components of high viscosity bone cement.

Mixing apparatus 4000 may comprise a container 4002, a mixing paddle 4004, a revolving plate 4005, gears 4006, axles 4008 and 4009, a cover 4010, and a handle 4012. Mixing element 4004 may have a large surface area of 400, optionally 600, optionally 800, optionally 1000 mm or intermediate or greater values. Optionally, mixing paddle 4004 is slotted or has holes distributed on its surface. Optionally, during operation mixing implement 4004 applies large shearing forces to a viscous mixture in well 4020. Optionally the large shearing force assures complete mixing of a liquid phase and a solid phase (e.g. powder or beads).

Paddle 4004 may be "wiped" on walls of container 4020. Optionally, shearing forces and stresses may vary with velocity of the revolving and/or paddle surface area and/or cement volume and/or cement viscosity. In a use scenario of mixer 4000, the cement components are inserted into a mixing well 4020 of container 4002. The cement components will typically initially include a solid phase (e.g. polymer beads or powder) and a liquid phase.

Closing cover 4010 may be closed by lowering it onto container 4002 so that tabs 4025 are engaged by slots 4030 prevents rotation of cover 4010 with respect to container 4002. Optionally, other rotational locking means are employed.

Revolution of handle 4012 turns axle 4008 and causes revolution of gears 4006A, 4006B and 4006C. Axle 4008 may be rotated by an electric motor, optionally a battery powered motor. Mixing element 4004 is attached via its axle 4003 to gear 4006A located on revolving plate 4005. When axle 4008 is turned, it causes revolution of gears 4006A, 4006B and 4006C. Revolution of revolving plate 4005 causes axle 4003 of mixing element 4005 to revolve about a center of mixing well 4020. The revolution without rotation of mixing paddle 4004 causes the mixing element to press the mixture against each of the four wells of mixing well 4020 in turn. This mixing pattern may reduce an amount of un-mixed material on inner walls of well 4020.

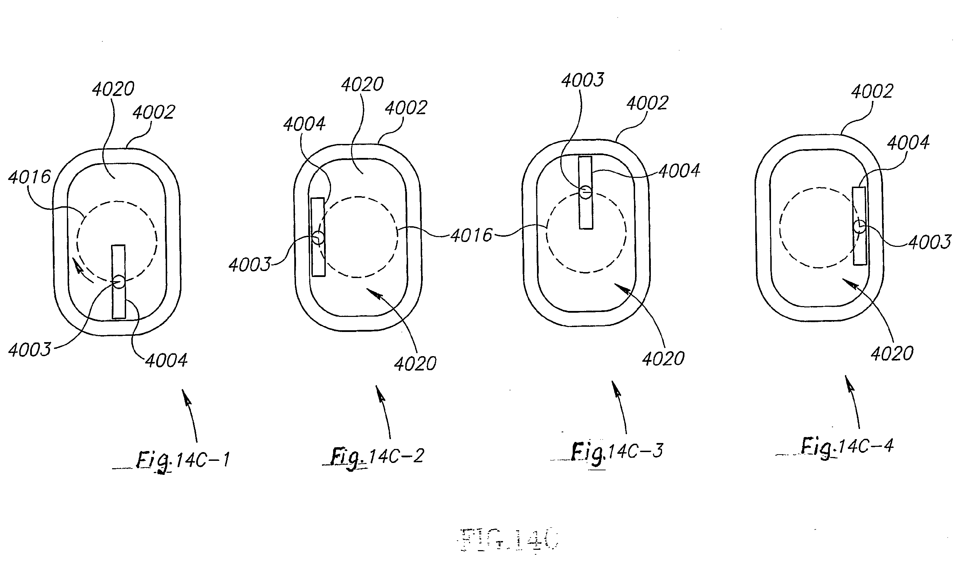

Figs. 14C1, 14C2:,14C3 and 14C4 are top views of mixing well 4020. The described sequential views of paddle 4004 describe how the apparatus successively presses material against walls of the mixing well. Axle 4003 may move along round path 4016. Gears 4006 A; 4006B and 4006C assure that paddle 4004 does not rotate around axis 4003. Thus, each of the four sides of the paddle 4004 always faces the same direction (relative to walls of mixing well 4020). Paddle 4004 may revolve without rotation because gears 4006A and 4006C each have the same number of teeth. Gear 4006B is interposed between gears 4006 A and 4006C to cause them to turn in a same direction. Optionally, gear 4006B has any desired number of teeth.

As illustrated in Fig. 14C1, as the paddle 4004 moves from the bottom wall towards the left wall, it applies pressure to a portion of the mixture located near the left wall presses it against the left wall of mixing well 4020 (Fig. 14C-2). The material being mixed tends to escape towards the upper and lower walls of well 4020. As paddle 4004 continues along its path (Figs. 14C-3) it contacts the upper wall of well 4020 and then presses the mixture against the right wall (Fig. 14C-4) of well 4020. This mixing pattern provides constant flow of the material being mixed and homogeneous mixing of the mixture components, even at high viscosity.

Mixing apparatus 4000 may be constructed of a wide variety of materials. A choice of construction materials optionally considers the particular type of bone cement to be mixed, its chemical characteristics and/or viscosity. Mixing well 4020 and/or container 4002 may be constructed at least partially of polypropylene and/or nylon. Paddle 4004 and/or axle 4003 may be constructed of stainless steel. Gears 4006A, 4006B and 400C are optionally constructed of plastic and/or metal.

Once mixing is complete, cover 4010 can be opened and the mixed contents can be removed from mixing well 4020.

**Transfer of viscous material**

Mixed viscous bone cement may be removed from mixing well 4020 and transferred to a reservoir of a delivery system. Optionally, the reservoir is a cement reservoir as described hereinabove. Optionally, the mixing well serves as a cement reservoir.

Viscous bone cement may be manually manipulated into a delivery system reservoir. Optionally, manual transfer includes shaping. Viscous bone cement may be manually shaped so that it roughly conforms to a configuration of a delivery reservoir. For example, the viscous material may be rolled into a roughly cylindrical form with a diameter slightly smaller than a delivery reservoir into which the material is to be introduced. Manual transfer may include use of a tool. For example, viscous bone cement is packed into a delivery reservoir using a tool. Optionally, the tool is a rod.

Viscous bone cement may be transferred to a delivery reservoir via an aperture in mixing well 4020. Optionally, the aperture is a lateral aperture in a wall of well 4020. Optionally, the same aperture is used to introduce cement into well 4020. The aperture may include a connector connectable to the delivery system reservoir. Optionally, the connector connects the mixing apparatus to the delivery system reservoir while the mixing apparatus operates.

**Transfer apparatus**

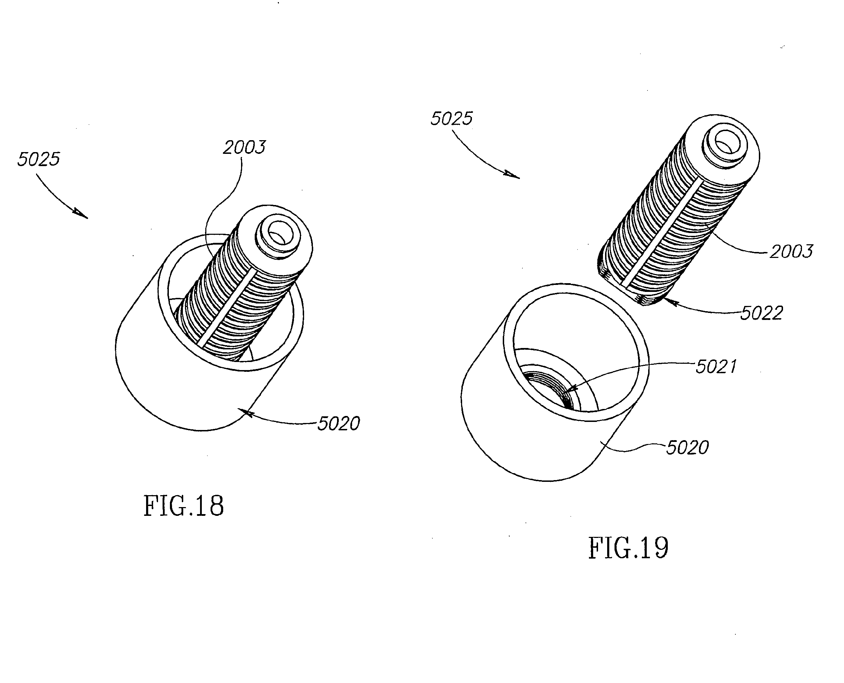

Figs. 18, 19, 20 and 21 illustrate a transfer apparatus 5000 for loading a viscous material into a container. The container may be a cement reservoir and the material is a viscous bone cement.

Fig. 18 illustrates cement reservoir 2003 assembled into a transfer piston 5020 to form a transfer assembly 5025. Fig. 19 illustrates that assembly of cement reservoir 2003 and transfer piston 5020 is optionally by means of matched threads 5021 and 5022.

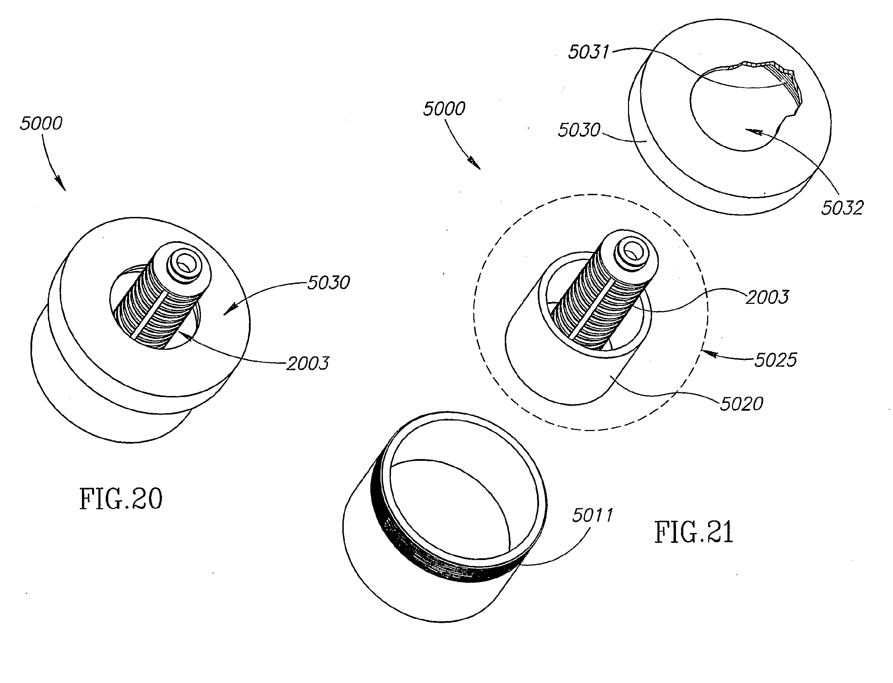

Fig. 21 illustrates assembly of transfer assembly 5025 into a container 5011 of a mixing apparatus. Not pictured is the viscous material (optionally bone cement) in container 5011. Transfer assembly 5025 is seated in container 5011 so that reservoir 2003 faces outwards. Cover 5030 is optionally applied to container 5011, for example using threads 5031 so that reservoir 2003 protrudes from hole 5032 as seen more clearly in Fig. 20.

Application of pressure to reservoir 2003 and/or an upper edge of piston 5020 causes piston 5020 to descend into container 5011. Cover 5030 may apply pressure to upper edge of piston 502o as it is attached to container 5011. Cement in container 5011 is displaced upwards into reservoir 2003. When the reservoir is sufficiently filled, it is removed from piston 5020. The reservoir may be transferred to a delivery system as described hereinabove.

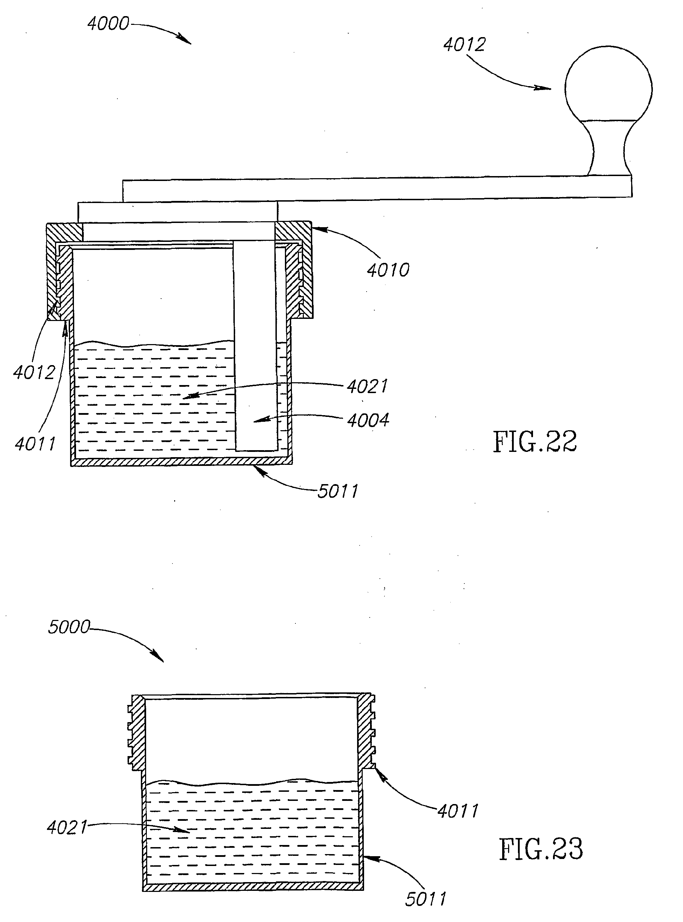

Figs. 22, 23, 24, 25 and 26 illustrate container 5011 which is a mixing well of a mixer 4000.

Fig. 22 is a cross sectional view of the mixer containing cement 4021. Cover 4010 is held in place by mated threads 4011 and 4012.

Fig. 23 is a cross sectional view of the mixer containing cement 4021 with cover 4010 removed. Mixing well 5011 becomes the basis for transfer apparatus 5000.

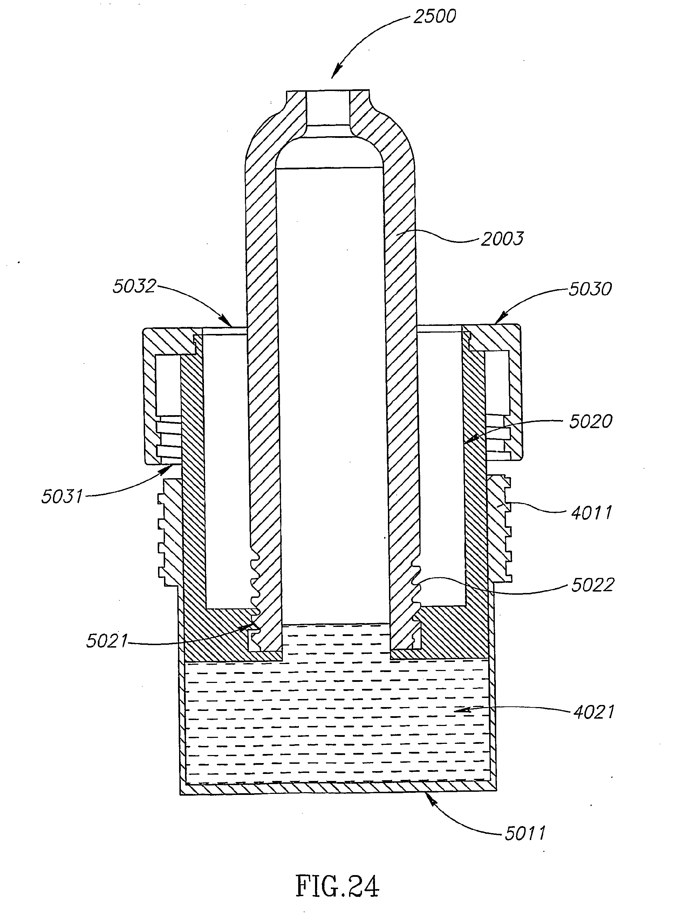

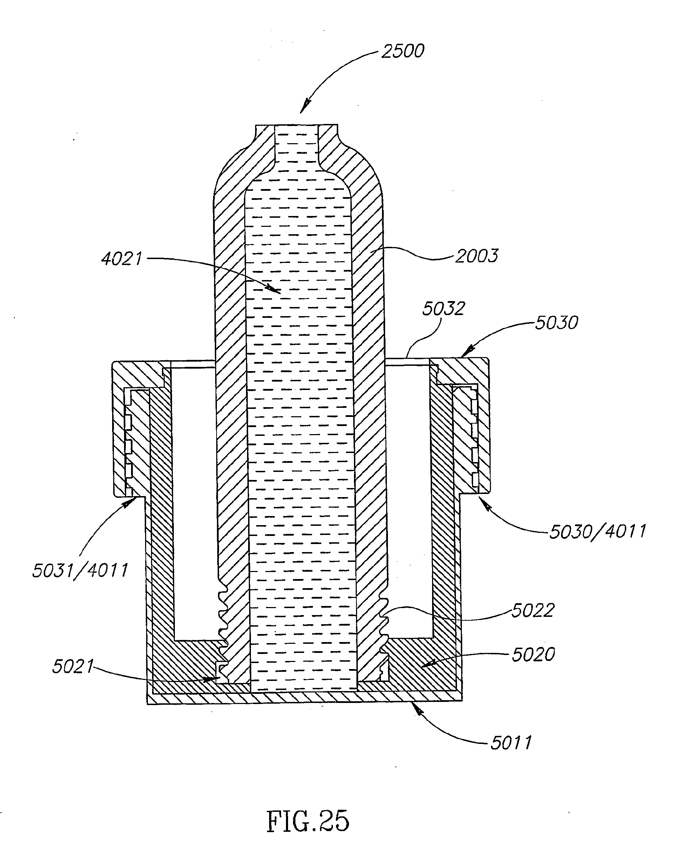

Figs. 24 and 25 are cross sectional views illustrating assembly of cement reservoir 2003 into transfer piston 5020. Optionally, assembly is via mated threads 5022 of reservoir 2003 and 5021 of transfer piston 5020. Cover 5030 is optionally employed to force transfer piston 5020 downwards into mixing well 5011. Cover 5030 may be threaded onto mixing well 5011 via complementary threads 5031 and 4011. Cover 5030 may be screwed onto well 5011 to force piston 5020 downwards onto cement 4021.

Fig. 25 illustrates that downward motion of piston 5020 forces cement 4021 to rise upwards into reservoir 2003 towards aperture 2500.

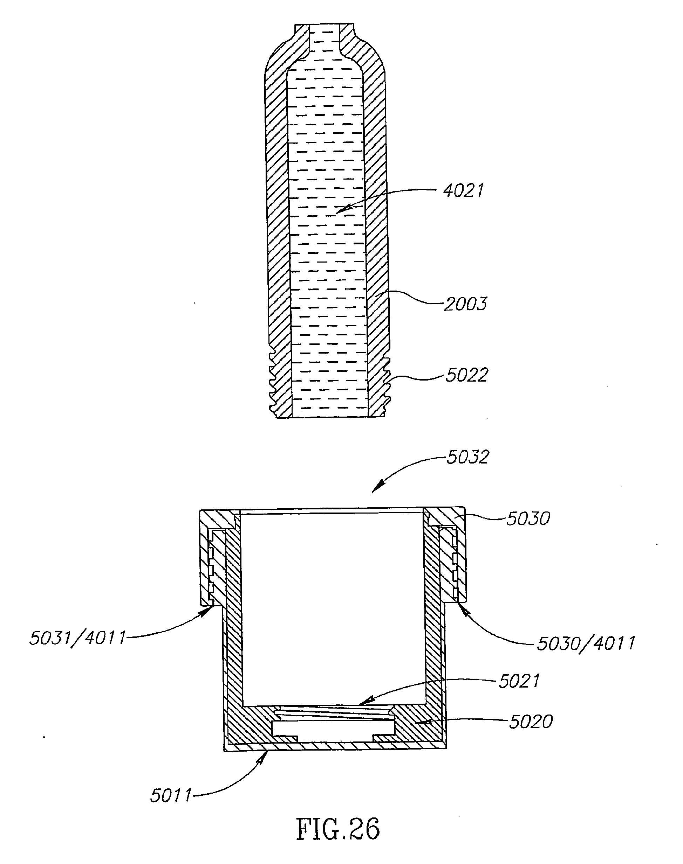

Fig. 26 illustrates removal of reservoir 2003 filled with cement 4022 by disengagement of threads 5022 from matching threads 5021. Optionally, a floor of mixing well 5011 and/or a base of transfer piston 5020 are not straight. This may reduce an amount of residual cement in well 5011 after transfer.

**Optional additional therapy**

The provision of material may be enhanced by additional therapy. Optionally, the additional therapy comprises thermal therapy. Optionally, the material is pre-heated or pre-cooled. Optionally, the pre-heating or pre-cooling also serves a purpose of controlling the material properties and/or setting behaviour.

Heating may be by contact heat (conduction) or by radiofrequency energy or light, for example a flash lamp or a laser source. Alternatively or additionally, the delivery system radiates heat. Optionally, a microwave or other wireless heating method is used.

Optionally, heating is provided separately from material provision. In one example, a heated guidewire is provided into the vertebra. Optionally, the guidewire extends one or more protrusions, to guide thermal energy into the nearby tissue. Optionally, a thermal sensor is provided to control the temperature in the vertebra and/or prevent over heating.

Temperature control may be applied to increase the handling and/or working time of the bone cement. Optionally, a temperature control unit operates on cement in an external reservoir and/or cement in a delivery system reservoir. The temperature control unit may include a resistive coil powered by an electric power source, optionally a battery.

**Exemplary Materials**

The materials which can be used in embodiments of the invention are known materials, for example, PMMA. However, they may be used at unusual conditions, for example at a semi-hardened condition. Also, while putty materials may be known, they are not typically used for injection through a small bore into bone.

It should be noted that while specific examples are described it is often the case that the material composition will be varied to achieve particular desired mechanical properties. For example, different diagnoses may suggest different material viscosities.

For non-hardening materials, the material can be allowed to set outside the body. After such setting the material may be washed or ventilated. In this manner, some materials with potentially hazardous by-products can be safely mixed and then used in the body. Optionally, a material is tested to make sure toxic by-products are removed to below a safety threshold. Optionally, a testing kit is provided with the delivery system.

The material may be selected so that its mechanical properties match the bone in which it will be implanted. The material may be matched to healthy or to osteoporotic trabecular bone. Optionally, the mechanical properties of the bone are measured during access, for example, based on a resistance to advance or using sensors provided through the cannula or by taking samples, or based on x-ray densitometers measurements.

In general, PMMA is stronger and has a higher modulus than trabecular bone. For example, trabecular bone can have a strength of between 3-20 megapascal and a Young modulus of 100-500 megapascal. Cortical bone, for example, has strength values of 170-190 gigapascal and Young modulus of 13-40 gigapascal. PMMA typically has values about half of Cortical bone.

The material may be selected to be less than 120% as strong and/or Young modulus as the expected bone to be treated. Optionally, the values of one or both of strength and Young modulus are 10%, 20%, 30%, 40% or less reduced from that of trabecular bone. It should be noted that if less of the vertebra is filled, the injected material will be supported, at least in part, by trabecular rather than cortical bone, depending for example on the method of filing of interior 308.

**Exemplary non-hardening material**

The material used may be a putty-like material. One example of a putty-like material is a hydroxyapatite with an increased ratio of sodium alginate. For example, the increased ratio can be 8% or 10%. While this material does harden in the body, it does not set to a hardened condition absent humidity. Thus it can be prepared ahead of time and pre-stored in a delivery system, for example by a manufacturer. The added material may slow down water absorption so that while sufficient water enters the material to initiate setting, not enough enters to cause dissolution. An example of this material is described in Ishikawa et al., "Non-decay fast setting Calcium phosphate cement: Hydroxyapatite putty containing an increased amount of sodium alginate", J Biomed Mater Res 36 1997, 393-399. More details may be found in "Effects of neutral sodium hydrogen phosphate on setting reaction and mechanical strength of hydroxyapatite putty", by Kunio Ishikawa, Youji Miyamoto, Masaaki Takechi, Yoshiya Ueyama, Kazuomi Suzuki, Masaru Nagayama and Tomohiro Matsumura, in J Biomed Mater Res, 44, 322-329, 1999.

Other calcium derivative cements, bone chips and/or fillers may be used as well. Bone chips, depending on processing may have a limited shelf life. Some of these materials generally harden (or combine with bone growth) after a relatively long time, such as more than a week, more than a month or more than 3 months.

**Additional exemplary non-hardening material**

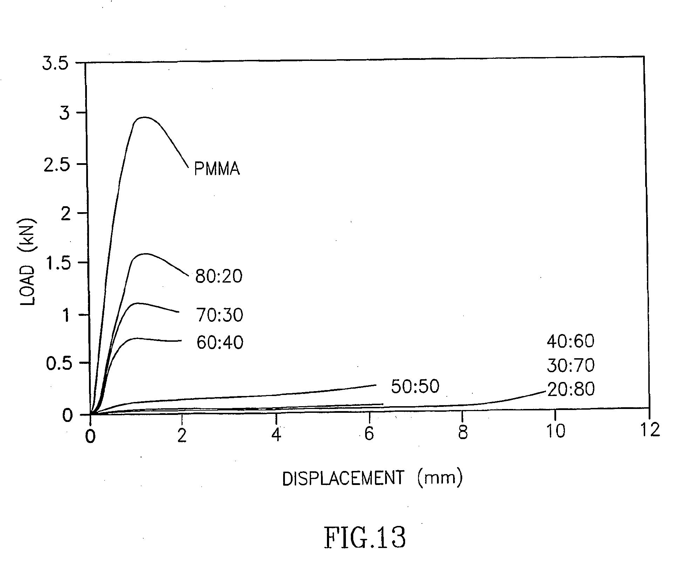

The material used may be a mixture of LMA (lauryl methacrylate) and MMA (methyl methacrylate). Depending on the ratio used, different mechanical properties and viscosities can be achieved. Fig. 13 is a graph showing the relative viscosities of PMMA and various ratios of the copolymer material. In the example shown, as the ratio of LMA decreases, viscosity goes down.

Diblock copolymers of MMA and LMA were synthesized by anionic polymerization using DPHLi as initiator in THF at -40°C with the sequential addition of monomers. The molecular weight distribution of the polymers was narrow and without homopolymer contamination when LMA was added to living PMMA chain ends.

The ratio used may be 80:20, 70:30, 60:40, 50:5.0, 30:70, 20:80 or intermediate, smaller or larger ratios (by volume).

**Experiment: Materials and Methods**

*Starting materials*

Medicinal distillate methyl methacrylate and lauryl methacrylate stabilized with 10-100 ppm of the monomethyl ether of hydroquinone were used as received from Fluka, Germany. Benzoyl peroxide (BPO) was purchased from BDH Chemicals, England. N Barium sulfate (BS) was obtained from Sigma-Aldrich (Israel). All solvents were analytical-grade from Biolab (Jerusalem, Israel) and were used as received.

*Polymerization*

Polymerization reactions were carried out in a single necked round bottom flask equipped with magnetic stirring. In a typical reaction, 60ml MMA (0.565mol), 50 ml LMA (0.137mol), 220 mg of Benzoyl Peroxide (0.9mmol) and 100 ml THF were transferred. The amount of BPO was adjusted to each of the compositions according to the total amount of mols of the monomer. The amount of the THF was equal to the total volume of the monomers (table 1). The content was heated to a polymerization temperature of 70-75°C for 20 hours, then the solution was precipitated in sufficient amount of methanol and left to mix for four hours. Finally the polymer was dried in an oven at 110°C under vacuum.

Table I: copolymers composition

| Copolymer (MA:LMA) | MA (ml/mol) | LMA (ml/mol) | BPO (mg/mol) | THF (ml) |
|---|---|---|---|---|
| 100:0 | 100 (0.94) | 0 (0) | 285 (1.18) | 100 |
| 80:20 | 80 (0.75) | 20 (0.07) | 258 (1.06) | 100 |
| 70:30 | 70 (0.66) | 30 (0.10) | 239 (0.99) | 100 |
| 60:40 | 60 (0.56) | 40 (0.14) | 220 (0.9) | 100 |
| 50:05 | 50 (0.47) | 50 (0.17) | 201 (0.83) | 100 |
| 40:60 | 40 (0.38) | 60 (0.20) | 182 (0.75) | 100 |
| 30:70 | 30 (0.28) | 70 (0.24) | 163 (0.67) | 100 |
| 20:80 | 20 (0.19) | 80 (0.27) | 144 (0.6) | 100 |
| 0:100 | 0 (0) | 100 (0.34) | 107 (0.44) | 100 |

The dried polymer was milled to a fine powder (Hsiangtai Sample mill, model sm-1, Taiwan) and mixed with barium sulfate (30%w/w). The mixture was heated in a glass inside a sand bath to 140°C, until melting of the polymer. The mixture was left to cool, and milled again. This procedure was repeated at least three times, until a homogeneous off-white polymer was received, which could be melted into loadable slugs for the delivery systems and magazines described above.

*Characterization*

Molecular weight and polydispersity were analysed by Gel permeation chromatography, GPC system consisting of a Waters 1515 isocratic HPLC pump with a Waters 2410 refractive-index detector and a Rheodyne (Coatati, CA) injection valve with a 20-ML loop (Waters Ma). The samples were eluted with $CHCl_3$ through a linear Ultrastyragel column (Waters; 500A pore size) at a flow rate of 1 mL/min.

$^1$H-NMR spectra were recorded on a Varian 300MHz instrument using $CDCl_3$, as solvents. Values were recorded as ppm relative to internal standard (TMS).

A Cannon 1C A718 Ubbelhold viscometer was used for the viscosity measurements of the polymer. The measurements were performed at 30°C with toluene as a solvent.

*Water Absorption Capacity*

Swelling behaviour of acrylic bone cements was carried out from accurately weighed films of 0.8 mm thickness. Films were introduced in 0.9 wt% NaCl solution (20 ml) and kept at 37°C. The water sorption kinetics in 20ml saline solution were evaluated in two specimens of each bone cement (containing 30% barium sulphate).

Equilibrium gain was determined gravimetrically at different periods of time. The uptake of water was recorded at 30 min intervals in the beginning and spacing out these intervals until the equilibrium was attained. At appropriate times, the samples were removed, blotted with absorbent paper to remove the water attached on its surface and weighed. The percentage of equilibrium gain was obtained from each specimen using the following expression

$$\text{Hydration Degree (\%)} = (\text{weight of swollen specimen} - \text{initial weight of specimen} / \text{initial weight of specimen}) \times 100$$

*Results:*

100% PMMA: Average 1.845% (+0.045)
Initial weight (g) 0.2156 and 0.2211
Weight of specimen at equilibrium (g) 0.2195 and 0.2253
Equilibrium gain (%):1.8 and 1.89;

60% PMMA, 40% PLMA: Average 1.65 % (+0.235)
Initial weight (g): 0.1161 and 0.1402
Weight of specimen at equilibrium (g) 0.1183 and 0.1422
Equilibrium gain (%):1.42 and 1.89;

50% PMMA, 50% PLMA: Average: 1.02 % (+0.28)
Initial weight (g): 0.2700 and 0.2371
Weight of specimen at equilibrium (g) 0.2720 and 0.2400
Equilibrium gain (%): 0.74 and 1.3.

*Compression Testing*

These tests were conducted using an Instron 4301 universal testing machine provided with a load cell of 5 kN, and at a cross-head speed of 20 mm/min. A known weight of polymer was melted in a glass inside a sand bath. The bath was heated at 150°C for two hours, and then barium sulfate was added (30% w/w) and mixed well several times, until homogenous dough was received. Cylindrical specimens of 6 mm in diameter and 12 mm high were prepared by forcing the melted copolymers into the holes of a Teflon mold. One side of the mold was covered with Teflon plates and secured with clamps. The specimens were cooled for 20 minutes in the mold, then the upper side was cut to the mold shape, and the specimens removed from the mold, finished to a perfect cylindrical shape. The test took place at least 1 week after aging in air at 23 $\pm$1 °C. For each cement composition, six specimens were tested. The elastic modulus and the maximal strength force were obtained.

Results:

*Molecular Weights and Viscosity Measurement*

The number and weight average molecular weights of poly (La-MA), poly (MMA) and their copolymers were obtained from gel permeation chromatography. The polydispersity index varies in the range 1.6 to 2.87. The viscosities of the polymers are obtained using Toluene as solvent at 25°C. The intrinsic viscosities ($\acute{\eta}$) were obtained by extrapolating $\acute{\eta}_{sp}c^{-1}$ to zero concentration. The molecular weights and viscosities are presented in Table II.

Table II: composition

| Feed Ratio MMA:LMA | NMR Analysis | GPC Analysis of Polymers | | | |
|---|---|---|---|---|---|
| **Vol % (mol %)** | **[MMA]:[LMA]** | **$M_n$** | **$M_W$** | **Polydispersity** | **[η]** |
| 100:0 (100:0) | 100:0 | 65190 | 119544 | 1.833 | 0.544 |
| 8:2 (91.5:8.5) | [88]:[12] | 69118 | 119194 | 1.724 | 0.421 |
| 7:3 (87:13) | 87:13 | 63006 | 112442 | 1.78 | 0.393 |
| 6:4 (84:16) | 84:16 | 73295 | 118384 | 1.615 | 0.366 |
| 1:1 (74:26) | 69:31 | 94167 | 135880 | 1.44 | 0.351 |
| 4:5 (69:31) | 70:30 | 55455 | 104711 | 1.888 | 0.316 |
| 4:6 (64:36) | 62:38 | 75648 | 134745 | 1.781 | 0.305 |
| 3:7 (56:44) | 56:44 | 35103 | 79986 | 2.27 | 0.221 |
| 2:8 (40:60) | 40:60 | 23876 | 68720 | 2.87 | 0.178 |
| 0:100 (0:100) | 0:100 | 27350 | 75146 | 2.74 | 0.083 |

*Compressive Test*

The results of the compressive test are collected in Table III as a function of compressive strength and modulus. The influence on the mechanical behaviour of adding lauryl methacrylate monomers can be clearly observed. The introduction of higher percentages produces a decrease that is more pronounced at 50% (v/v) LA. The compressive modulus shows a drastic decrease as the content of LA increases. This drop may be related to the structure modification of the matrix by the introduction of LMA. This drop may also limit the use of some compositions for some applications.

Table III: compression test results

| Composition MA:LA (V%) | Max Strength (MPa) | Modulus (MPa) |
|---|---|---|
| 1:0 | 106.8 (9) | 2478 (220) |
| 8:2 | 82.5 (17.1) | 1100.7 (129) |
| 7:3 | 63.3 (13.2) | 634.5 (116) |
| 6:4 | 48 (11) | 550 (250) |
| 5:5 | 18.9 (4.5) | 69.6 (20) |
| 4:6 | 1.9 (0.2) | 49.5 (11.8) |
| 3:7 | 19.19 (3.42) | 8.3 (1.2) |
| 2:8 | 0.253 (0.06) | 1.71 (0.417) |

*Material modifications*

Optionally, various additives are added to the materials described herein, to modify their properties. The adding can be before setting or after setting, depending on the material. Exemplary materials that can be added include fibers (e.g., carbon nanotubes or glass fibers) of various lengths and thicknesses, aggregates and/or air bubbles.

If the material is manufactured to be anisotropic, it can be advanced into the body in a desired direction, for example, by selecting a delivery path (e.g., storage, tube, aperture) to reduce twisting and/or deformation. Optionally, such materials are provided as short units (Fig. 8).

**Softening and semi-hardening materials**

The material used may soften after provision into the body. The material may comprise an additive that disperses or weakness in water or body fluids, for example, salt. A softening material may be useful if the forces required for height restoration are smaller than the forces required for maintaining height. Softening times are optionally controlled by mixing in a gel material which slows down water penetration into the extruded material.

**Semi-hardening materials**

The material used may set to non-hardened condition. The material may comprise MMA, LMA and NMP. NMP solvates in water, allowing the material to set somewhat. A hardened condition may be avoided, possibly preventing the induction of fractures in nearby vertebra.

**Use of hardening materials**

The above described devices (e.g., delivery) may be used with a material which sets to a hardened condition, for example, PMMA or other bone cements and fillers. The material may be provided in a kit that includes a timer and/or a viscometer, so that an operator can estimate the workability and viscosity of the material and its usefulness for height restoration without leakage. Optionally, the time includes a temperature senor and provides an estimate of workability time based on the temperature and the time the components of the PMMA were mixed.

Optionally, styrene may be added.

In general bone cements polymerize by radical-initiated addition reactions. According to the invention, the cement is prepared from two separate components: a powder component containing prepolymerized beads of PMMA or a PMMA/styrene copolymer and a liquid component containing MMA.

In an exemplary embodiment of the invention, an initiator (e.g. benzoyl peroxide (BPO) is incorporated into the powder component and a chemical activator (e.g. DMPT) is incorporated into the liquid component. Optionally, an easily oxidized molecule (e.g. hydroquinone) is added to the liquid component to prevent spontaneous polymerization during storage. Optionally, cement may be rendered radiopaque, for example by adding a radio-opaque material such as adding barium sulfate and/or zirconium compounds to the powder and/or liquid component.

Optionally, the average molecular weight of PMMA in all beads is 80,000, optionally 100,000, optionally 120,000, optionally 140,000, optionally 160,000, optionally 180,000 Dalton or intermediate or lesser or greater values. In an exemplary embodiment of the invention, the average molecular weight PMMA in all beads is approximately 110,000 Dalton. Optionally, at least some of the beads include styrene. In an exemplary embodiment of the invention, styrene is added to PMMA beads in a volumetric ratio of 5-25%.

According to the invention, at least some beads contain PMMA with a higher molecular weight. Optionally, the higher molecular weight is 600,000, optionally 900,000, optionally 1,100,000 Dalton or intermediate or greater values. Optionally, the beads with the higher molecular weight comprise 0.25%, 0.5%, 1%, 2%, 3%, 4%, 5% or intermediate or lesser or higher of the total bead population. This type of formulation provides a cement characterized by a short mixing time and/or a cement which achieves a viscosity of 500 to 900 Pascal-second in 2 to 3 minutes from the beginning of mixing and/or which remains sufficiently flowable for injection for at least 6 to 10 minutes.

In an exemplary embodiment of the invention, higher molecular weight PMMA in the polymer beads cause the mixture to achieve a flowable plastic phase earlier than previously available cements and/or to remain in the flowable plastic phase longer than previously available alternatives. Optionally, altering the percentage of higher molecular weight PMMA in the polymer beads alters a viscosity profile of the resultant mixture.

Optionally, at least one bead of PMMA has molecular weight in the range of 700,000 Dalton to 1,000,000 Dalton. In an exemplary embodiment of the invention, approximately 3% of the beads have PMMA characterized by a molecular weight in this range.

In an exemplary embodiment of the invention, a setting material is formulated to have a high viscosity for a working window of significant duration, for example, 5, 8, 10 or intermediate or more minutes. In an exemplary embodiment of the invention, the following formulation is used: a set of beads formed of PMMA/Styrene of diameter 10-200 microns and an amount of 20 cc MMA per 9.2 grams beads. In an exemplary embodiment of the invention, MMA solvates and/or encapsulates the beads and the viscosity of the mixture remains high, at the beginning due to the solvation and friction between the beads and later, as the beads dissolve, due to the progressing of polymerization. The beads may also be provided in a mixture comprising a range of sizes. It should be noted that the properties of the materials may be selected to improve a viscosity working window, even if strength of the final cement is compromised.

In an exemplary embodiment of the invention, the working viscosity is set by selecting the bead size and/or material ratios and/or molecular weights of polymer provided in the beads.

In an exemplary embodiment of the invention, the working viscosity is influenced by the presence of particles of hardened acrylic polymer added to the mixture.

## Mechanical viscosity increasing agents

In an exemplary embodiment of the invention, the cement includes particles characterized by a large surface which do not participate in the polymerization reaction. Examples of materials suitable for use as particles characterized by a large surface which do not participate in the polymerization reaction include, but are not limited to Zirconium, hardened acrylic polymer and bone. Optionally, the particles characterized by a large surface which do not participate in the polymerization reaction are not X-ray transparent so that they aid in visualization of injected cement. In an exemplary embodiment of the invention, the large surface area particles impart added viscosity to the cement mixture independent of polymerization. Optionally, the added viscosity comes from friction of particles against one another in the cement.

## Polymerization Reaction Kinetics

In an exemplary embodiment of the invention, the mixture of polymer and monomer components produces a material with a viscosity in the range 500 to 900 Pascal-second within 120, optionally within 100, optionally within 60, optionally within 30, optionally within 15 seconds or lesser or greater or intermediate times. In an exemplary embodiment of the invention, once a high viscosity is achieved, the viscosity remains stable for 5 minutes, optionally 8 minutes, optionally 10 minutes or lesser or intermediate or greater times. In an exemplary embodiment of the invention, stable viscosity indicates a change of 10% or less in two minutes and a change of 20% or less in 8 minutes. The time during which viscosity is stable provides a window of opportunity for performance of a medical procedure.

## Material with a glass transition temperature

In an exemplary embodiment of the invention, a bone cement includes a material characterized by a glass transition temperature higher than 37 degrees Celsius. Heating such a material above its glass transition temperature, weakens the material. The weakening transforms the material to a dough-like or putty-like state. In an exemplary embodiment of the invention, the dough-like material is suitable for delivery using a delivery system as disclosed herein. After delivery, the dough cools to 37 degrees Celsius and hardens. Examples of materials with a glass transition temperature above 37 degrees include, but are not limited to, polycaprolactone (PCL) and/or Polylactic acid (PLA). Polymers with glass transition temperatures suitable for use in the context of the invention are commercially available, for example "Lactel absorbable polymers" (Curect Corp.; Pelham, AL, USA).

In an exemplary embodiment of the invention, a material with a glass transition temperature above 35, optionally 40, optionally 45, optionally 50, optionally 55, optionally 60 degrees Celsius is selected.

Optionally, the delivery system is heated to maintain the material above the glass transition temperature. In an exemplary embodiment of the invention, a heating element is provided in and/or adjacent to the cement reservoir and/or the cannula.

## Use of Bone in bone cement

In an exemplary embodiment of the invention, the bone cement or other dough-like material includes processed bone (from human or animals origin) and/or synthetic bone. Optionally, the cement has osteoconductive and/or osteoinductive feature. Optionally, the processing of the bone includes grinding. One of ordinary skill in the art will be capable of processing bone using known methods for use in the context of the present invention. In an exemplary embodiment of the invention, the bone cement comprises 50%, optionally 60 % optionally 70% or intermediate or greater percentages of bone powder and/or granules and/or chips.

## Additional implant devices

Optionally, an implant is also injected into the vertebra, for example, before, during or after injection of the material. Exemplary implants are metal or polymer cage or intra ventricular devices and enclosing mesh or solid bags or balloons. Optionally, bone graft is injected. Optionally, where an implant is provided, the material is extruded through the implant, for example from an axial section thereof in a radial direction. Optionally, devices such as, for example, those described in PCT/ILOO/00458; PCT/ILOO/00058; PCT/ILOO/00056; PCT/ILOO/00055; PCT/ILOO/00471;

PCT/IL02/00077; PCT/IL03/00052; and PCT/IL2004/000508, PCT/IL2004/000527 and PCT/IL2004/000923 are used.

Optionally, the material is extruded into a performed cavity, for example a cavity formed using an inflatable balloon. Optionally, the material is extruded into an inter-vertebral space, for example a disc-space.

Optionally, a material which sets to a hardened condition, for example, PMMA is co- extruded with or extruded before or after material which does not so set. Optionally, the setting material comprises less than 60% of the material, for example, less than 40%, less than 20% or intermediate values.

**Other tissue and general**

While the above application has focused on the spine, other tissue can be treated as well, for example, compacted tibia plate and other bones with compression fractures and for tightening implants, for example, hip implants or other bone implants that loosened, or during implantation. Optionally, for tightening an existing implant, a small hole is drilled to a location where there is a void in the bone and material is extruded into the void.

It should be noted that while the use in bones of the above methods and devices provide particular advantages for bone and vertebras in particular, optionally, non-bone tissue is treated, for example, cartilage or soft tissue in need of tightening. Optionally, the delivered material includes an encapsulated pharmaceutical and is used as a matrix to slowly release the pharmaceutical over time. Optionally, this is used as a means to provide anti-arthritis drugs to a joint, but forming a void and implanting an eluting material near the joint.

A bone cement according to the invention may be injected into a bone void as a preventive therapy and/or as a treatment for a fracture, deformity, deficiency or other abnormality. Optionally, the bone is a vertebral body and/or a long bone. The cement may be inserted into the medullary canal of a long bone. Optionally, the cement is molded into a rod. The rod may serve as an intra-medular nail.

It will be appreciated that the above described methods of implanting and treating may be varied in many ways, including, changing the order of steps, which steps are performed more often and which less often, the arrangement of elements, the type and magnitude of forces applied and/or the particular shapes used. In particular, various trade-offs may be desirable, for example, between applied forces, degree of resistance and forces that can be withstood.

Further, the location of various elements may be switched, for example, the location of the power source. In addition, a multiplicity of various features, both of method and of devices have been described. It should be appreciated that different features may be combined in different ways. In particular, not all the features shown above in a particular arrangement are necessary in every similar exemplary arrangement. In addition, some of the features described herein may be adapted for use with prior art devices. The particular geometric forms should not be considered limiting. For example, where a cylindrical tube is shown, a rectangular tube may be used. Although some limitations are described only as method or apparatus limitations, apparatus programmed and/or designed to carry out the methods are included.

When used in the following claims, the terms "comprises", "comprising", "includes", "including" or the like means "including but not limited to". It will be appreciated by a person skilled in the art that the present invention is not limited by what has thus far been described. Rather, the scope of the present invention is limited only by the following claims.

**Claims**

1. A bone cement comprising:

   a first powder component which includes poly(methyl methacrylate) (PMMA) with at least 80% of the PMMA having a bead size of between 10 and 200 $\mu$m, and
   a second liquid component which includes methyl methacrylate (MMA),
   in which contacting the first component and the second component produces a mixture which is suitable for *in vivo* use,
   **characterised in that** a portion of the PMMA has a molecular weight between about 600,000 Dalton and about 1,200,000 Dalton and **in that** the mixture attains a viscosity greater than 500 Pascal.seconds within an initial period, and the viscosity of the mixture remains between 500 and 2000 Pascal.seconds for a working time of at least 5 minutes after the initial period, the viscosity of the mixture being measured using a capillary extrusion rheometer for measuring an applied force acting on a piston to displace the mixture in a capillary tube at a constant displacement rate, the measurements being performed at an average temperature of 22.3°C and a constant displacement rate of 3mm/min.

2. The bone cement of claim 1, in which the initial period is less than 3 minutes and the mixture solidifies after the working time

**3.** The bone cement of claim 2, in which the first component includes PMMA and barium sulphate.

**4.** The bone cement of claim 2, in which the second component includes MMA and DMPT.

**5.** The bone cement of claim 2, in which the first component includes PMMA, barium sulphate, and benzoyl peroxide, and in which the second component includes MMA, DMPT and hydroquinone.

**6.** The bone cement of claim 1, in which the first component contains about 69.4% w/w PMMA, about 30.1% barium sulphate, and about 0.5% benzoyl peroxide, and in which the second component contains about 98.5% v/v MMA, about 1.5% DMPT, and about 20 ppm hydroquinone.

**7.** The bone cement of claim 1, further comprising processed bone and/or synthetic bone that are mixed together with the first component and the second component.

**Patentansprüche**

**1.** Knochenzement, der Folgendes umfasst:

eine erste pulverförmige Komponente, welche Polymethylmethacrylat (PMMA) aufweist, wobei wenigstens 80% des PMMA eine Korngröße zwischen 10 und 200 $\mu$m besitzt, und
eine zweite flüssige Komponente, welche Methylmethacrylat (MMA) aufweist,
wobei durch Kontakt der ersten Komponenten mit der zweiten Komponente eine Mischung erzeugt wird, welche für eine Verwendung *in vivo* geeignet ist,
**dadurch gekennzeichnet, dass** ein Teil des PMMA ein Molekulargewicht zwischen etwa 600.000 Dalton und etwa 1.200.000 Dalton besitzt und dass die Mischung eine Viskosität erreicht, die größer als 500 Pascal x Sekunde in einer Anfangszeit ist, und
die Viskosität der Mischung zwischen 500 und 2000 Pascal x Sekunde für eine Verarbeitszeit von etwa 5 Minuten nach der Anfangszeit verbleibt, wobei die Viskosität der Mischung gemessen wird unter Verwendung eines kapillaren Extrusionsrheometers zum Messen einer anliegenden Kraft, die auf einen Kolben wirkt, um die Mischung in einer kapillaren Röhren mit einer konstanten Verdrängungsrate zu verdrängen, wobei die Messungen bei einer mittleren Temperatur von 22,3°C und einer konstanten Verteilungsrate von 3 mm/Minute erfolgen.

**2.** Knochenzement nach Anspruch 1, wobei die Anfangszeit kleiner als 3 Minuten ist und die Mischung nach der Verarbeitungszeit aushärtet.

**3.** Knochenzement nach Anspruch 2, wobei die erste Komponente PMMA und Bariumsulfat aufweist.

**4.** Knochenzement nach Anspruch 2, wobei die zweite Komponente MMA und DMPT ausweist.

**5.** Knochenzement nach Anspruch 2, wobei die erste Komponente PMMA, Bariumsulfat und Benzoylperoxid aufweist und wobei die zweite Komponente MMA, DMPT und Hydrochinon aufweist.

**6.** Knochenzement nach Anspruch 1, wobei die erste Komponente etwa 69,4% w/w PMMA, etwa 30,1% Bariumsulfat und etwa 0,5 % Benzoylperoxid enthält und wobei die zweite Komponente etwa 98,5 % v/v MMA, etwa 1,5 % DMPT und etwa 20 ppm Hydrochinon enthält.

**7.** Knochenzement nach Anspruch 1, welches ferner bearbeiteten Knochen und/oder synthetischen Knochen umfasst, die mit der ersten Komponente und der zweiten Komponente gemischt werden.

**Revendications**

**1.** Ciment osseux comprenant :

un premier composant poudreux qui comprend du polyméthacrylate de méthyle (PMMA), avec au moins 80 % du PMMA ayant une taille de billes comprise entre 10 et 200 $\mu$m, et

un second composant liquide qui comprend du méthacrylate de méthyle (MMA),

dans lequel une mise en contact du premier composant et du second composant produit un mélange qui est approprié pour une utilisation in vivo,

**caractérisé en ce qu'**une partie du PMMA possède un poids moléculaire compris entre environ 600 000 Daltons et environ 1 200 000 Daltons et **en ce que** le mélange atteint une viscosité supérieure à 500 pascals-secondes au cours d'une période initiale, et la viscosité du mélange reste comprise entre 500 et 2 000 pascals-secondes pendant un temps de travail d'au moins 5 minutes après la période initiale, la viscosité du mélange étant mesurée en utilisant un rhéomètre capillaire à extrusion afin de mesurer une force appliquée agissant sur un piston pour déplacer le mélange dans un tube capillaire à une vitesse de déplacement constante, les mesures étant réalisées à une température moyenne de 22,3 °C et à une vitesse de déplacement constante de 3 mm/minute.

2. Ciment osseux selon la revendication 1, où la période initiale est inférieure à 3 minutes et le mélange se solidifie après le temps de travail.

3. Ciment osseux selon la revendication 2, dans lequel le premier composant comprend du PMMA et du sulfate de baryum.

4. Ciment osseux selon la revendication 2, dans lequel le second composant comprend du MMA et de la DMPT.

5. Ciment osseux selon la revendication 2, dans lequel le premier composant comprend du PMMA, du sulfate de baryum, et du peroxyde de benzoyle, et dans lequel le second composant comprend du MMA, de la DMPT et de l'hydroquinone.

6. Ciment osseux selon la revendication 1, dans lequel le premier composant contient environ 69,4 % p/p de PMMA, environ 30,1 % de sulfate de baryum, et environ 0,5 % de peroxyde de benzoyle, et dans lequel le second composant contient environ 98,5 % p/p de MMA, environ 1,5 % de DMPT, et environ 20 ppm d'hydroquinone.

7. Ciment osseux selon la revendication 1, comprenant en outre de l'os traité et/ou de l'os synthétique qui sont mélangés ensemble avec le premier composant et le second composant.

100

IDENTIFY BONE ~102

BONE ACCESS ~104

INJECT MATERIAL ~106

(OPTIONAL)
RESTORE HEIGHT ~108

END PROCEDURE ~110

FIG.1A

FIG.1B

*122* FORM PASSAGE TO BONE

*124* PENETRATE CORTEX WITH GUIDE WIRE

*126* RETRACT GUIDE WIRE (OPTIONAL)

*128* ADVANCE DRILL INTO BONE

*130* ADVANCE CANULA TO BONE

*132* REMOVE GUIDE WIRE, DRILL

*134* INSERT DELIVERY SYSTEM

*136* INJECT

*138* FEEDBACK

*140* REFILL (OPTIONAL)

*142* CHANGE DIRECTION

*144* TOP OFF (OPTIONAL)

*146* COMPLETE PROCEDURE

*120*

FIG.2

FIG.3A

FIG.3B

FIG.3C

FIG.3D

FIG.3E

FIG.3F

FIG.4A

EP 1 850 797 B1

FIG.4B

500

502

FIG.5A

510

512

FIG.5B

528

532

522

524

526

530

520

FIG.5C

408

602

402

604

FIG.6A

408'

606

608

402

604

FIG.6B

408''

610

402

FIG.6C

EP 1 850 797 B1

FIG.7A

EP 1 850 797 B1

744

742

714'

720'

740

FIG.7B

FIG.7C

FIG.7D

FIG.7E

FIG.7F

FIG.7G

FIG.7H

FIG.7I

FIG.7J

FIG.7K

FIG.7L

FIG.7M

FIG.7N

FIG.7O

FIG.7P

FIG.7Q

EP 1 850 797 B1

FIG.7R

FIG.7S

2509

2500

2510

2003

H

2003

FIG.7S1

FIG.7T

FIG.7U

FIG.7V

4100

4200

PRESSURE

PRESSURE RELEASE

4300

4210

4231

2200

4227

4220

4225

4230

EP 1 850 797 B1

4200

4100

4210

4300

4220

2215

2200    2115    4231

4227

FIG.7W

PRESSURE

4100

4300

4150

FIG.7X

FIG.7Y

EP 1 850 797 B1

FIG.8A

FIG.8B

FIG.9A

FIG.9B

FIG.10A

EP 1 850 797 B1

FIG.10B

EP 1 850 797 B1

FIG.10C

FIG.10D

EP 1 850 797 B1

FIG.10E

FIG.10F

FIG.11A

FIG.11B

FIG.11A

FIG.11B

—1206

—1200

—1204

—1202

FIG.12A

1204

1202

1204

1202

FIG.12B

—1234

—1238

—1230

1232

FIG.12C

—1236

FIG.13

4012

4030

4010

4008

4009

4000

4006A

4006C

4006B

4005

4003

4004

4025

4020

4025

4002

**FIG.14A**

4000

4012

4010

4002

**FIG.14B**

FIG.15

FIG.16

FIG.17

EP 1 850 797 B1

5025

2003

5020

FIG.18

5025

2003

5022

5021

5020

FIG.19

5032

5031

5030

5000

2003

5025

5020

5011

FIG.21

5030

2003

5000

FIG.20

4000

4012

4010

4012

4011

4021

4004

5011

FIG.22

5000

4021

4011

5011

FIG.23

FIG.24

FIG.25

4021

2003

5022

5032

5030

5031/4011

5030/4011

5021

5020

5011

FIG.26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4969888 A
- US 5108404 A
- US 6383188 B
- US 20030109883 A
- US 20020068974 A
- US 6348055 B
- US 6383190 B
- US 4494535 A
- US 4653489 A
- US 4653487 A
- US 6241734 B
- US 6613054 B
- US 20040260303 A
- US 4341691 A

- US 60721094 B
- WO 0044319 A
- WO 2004110300 A
- IL OO00458 W
- IL OO00058 W
- IL OO00056 W
- IL OO00055 W
- IL OO00471 W
- IL 0200077 W
- IL 0300052 W
- IL 2004000508 W
- IL 2004000527 W
- IL 2004000923 W

**Non-patent literature cited in the description**

- **NUSSBAUM DA et al.** The Chemistry of Acrylic Bone Cement and Implication for Clinical Use in Image-guided Therapy. *J Vase Interv Radiol,* 2004, vol. 15, 121-126
- **G BAROUD et al.** Injection biomechanics of bone cements used in vertebroplasty. *Bio-Medical Materials and Engineering,* 2004, vol. 00, 1-18
- The viscosity of acrylic bone cements. *Journal of Biomedical Materials Research,* 1992, vol. 16, 219-243

- **ISHIKAWA et al.** Non-decay fast setting Calcium phosphate cement: Hydroxyapatite putty containing an increased amount of sodium alginate. *J Biomed Mater Res,* 1997, vol. 36, 393-399
- **KUNIO ISHIKAWA ; YOUJI MIYAMOTO ; MASA-AKI TAKECHI ; YOSHIYA UEYAMA ; KAZUOMI SUZUKI ; MASARU NAGAYAMA ; TOMOHIRO MATSUMURA.** Effects of neutral sodium hydrogen phosphate on setting reaction and mechanical strength of hydroxyapatite putty. *J Biomed Mater Res,* 1999, vol. 44, 322-329